(19) **Europäisches Patentamt / European Patent Office / Office européen des brevets**

(11) **EP 2 064 375 B1**

(12) **EUROPEAN PATENT SPECIFICATION**

(45) Date of publication and mention of the grant of the patent:
**21.11.2012 Bulletin 2012/47**

(51) Int Cl.:
*C40B 40/04* (2006.01)  *C12N 11/00* (2006.01)
*C12Q 1/02* (2006.01)  *C40B 30/00* (2006.01)
*C40B 50/14* (2006.01)

(21) Application number: **07719904.0**

(22) Date of filing: **06.06.2007**

(86) International application number:
**PCT/CA2007/000988**

(87) International publication number:
**WO 2007/140595 (13.12.2007 Gazette 2007/50)**

(54) **ASSAY SUPPORTS COMPRISING A PEG SUPPORT, SAID SUPPORT ATTACHED FROM A PEG SOLUTION IN CLOUD POINT (THETA SOLVENT) CONDITIONS**

ASSAYTRÄGER, UMFASSEND EINEN PEG-TRÄGER, WOBEI DER TRÄGER AUS EINER PEG-LÖSUNG UNTER TRÜBUNGSPUNKTBEDINGUNGEN (THETA-LÖSUNGSMITTEL) GEBUNDEN WIRD

SUPPORTS D'ESSAI COMPORTANT UN SUPPORT DE POLYETHYLENE GLYCOL, LEDIT SUPPORT FIXE PROVENANT D'UNE SOLUTION DE POLYETHYLENE GLYCOL DANS DES CONDITIONS DE POINT DE TROUBLE (SOLVENT THETA)

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **06.06.2006 US 804021 P**

(43) Date of publication of application:
**03.06.2009 Bulletin 2009/23**

(73) Proprietor: **Société de Commercialisation des Produits de la Recherche Appliquée - SOCPRA-Sciences et Génie s.e.c. Sherbrooke, QC J1K 2R1 (CA)**

(72) Inventors:
• **VERMETTE, Patrick**
  **Sherbrooke, Quebec J1N 4H3 (CA)**
• **MARTIN, Yves**
  **Sherbrooke, Quebec J1R 0K3 (CA)**
• **MONCHAUX, Emmanuelle**
  **F-15150 Laroquebrou (FR)**

(74) Representative: **Srinivasan, Ravi Chandran J A Kemp 14 South Square Gray's Inn London WC1R 5JJ (GB)**

(56) References cited:
EP-A1- 1 428 871    WO-A2-2005/028619
US-A- 5 976 826    US-A1- 2005 042 455
US-A1- 2006 019 235

• OTSUKA HIDENORI ET AL: "Two-dimensional multiarray formation of hepatocyte spheroids on a microfabricated PEG-brush surface" CHEMBIOCHEM - A EUROPEAN JOURNAL OF CHEMICAL BIOLOGY, WILEY VCH, WEINHEIM, DE, vol. 5, no. 6, 7 June 2004 (2004-06-07), pages 850-855, XP002298016 ISSN: 1439-4227
• SOULTANI-VIGNERON: 'Immobilisation of oligo-peptidic probes for microarray implementation' J. OF CHROMATOGRAPHY B vol. 822, no. 1-2, 05 August 2005, pages 304 - 310, XP004983043
• JONES: 'Ryanodine receptor binding to FKBP12 is modulated by channel activation state' JOURNAL OF CELL SCIENCE vol. 118, no. 20, 15 October 2005, pages 4613 - 4619, XP008092783
• SEONG: 'Current status of protein chip development in terms of fabrication and application' PROTEOMICS vol. 3, no. 11, 2003, pages 2176 - 2189, XP008051364
• ITO: 'Preparation of a protein micro-array using a photo-reactive polymer for a cell-adhesion assay' BIOMATERIALS vol. 24, no. 18, August 2003, pages 3021 - 3026, XP004441782

- ITO: 'Photo-reactive polyvinylalcohol for photo-immobilized microarray' BIOMATERIALS vol. 26, no. 2, January 2005, pages 211 - 216, XP004519238
- 'Bioacore Life Sciences Sensor Chips', [Online] XP008095421 Retrieved from the Internet: <URL: http://www.biacore.com/lifesciences/pr oducts/sensor_chips/guide/index.html>

Remarks:
   The file contains technical information submitted after the application was filed and not included in this specification

**Description**

CROSS REFERENCE TO RELATED APPLICATIONS

This application claims the benefit, under 35 U.S.C. § 119(e), of U.S. provisional application Serial No. 60/804,021 filed on June 6, 2006.

FIELD OF THE INVENTION

The present invention relates to assay supports, such as microarrays and similar devices. More specifically, the present invention is concerned with assay supports, capable of assaying binding and/or activity of a bioactive entity, such as a bioactive molecule or a cell.

BACKGROUND OF THE INVENTION

Understanding and controlling the interactions between naturally-derived or synthetic materials and biomolecule-containing fluids or cells is of increasing importance in a variety of fields. For example, biomolecule interactions with a variety of substrate materials, i.e. surfaces, are central to numerous analytical systems, including immunodiagnostics, gene microarrays, protein microarrays and microfluidic "lab-on-a-chip" devices. Analytical techniques such as capillary electrophoresis (CE), surface plasmon resonance (SPR), and quartz crystal microbalance (QCM) also intimately depend on biomolecule-surface interactions. Performances of biomedical devices including cardiovascular replacements (e.g., catheters, valves, and stents), contact and intraocular lenses, shunts, filters, diaphragms, pumps, membranes, drug delivery devices and surgical components also depend on control of biomolecule-surface interactions. Maritime surfaces, including heat exchange units, ship-board components (e.g., hulls, superstructure), installations (pump derricks), docks, and environmentally exposed instruments also require control of biomolecule deposition (algae, fungus, microbial exudates) on their surfaces.

Of major common concern within these fields is the level of non-specific biomolecule (e.g., protein, organism, nucleic acid) binding to target and device substrates. In non-sensing or non-diagnostic applications, this concern generally focuses on surface-induced biofouling (deposition of biological material that compromises function). This is particularly problematic in biomedical devices and maritime applications. For example, non-specific adsorption can often lead to failure of a medical device or to that of a biosensor exposed to complex biological environments, altering their functions or inducing their rejection. In medical, food, and environmental sensing and diagnostics, specificity, signal-to-noise ratios, and detection limits of a target analyte (e.g., polysaccharides, nucleic acid, drug, peptide, protein), often at limited concentrations in a milieu of non-target biomolecules (e.g., serum proteins), are limited by non-specific non-target (e.g., protein) binding on the substrate. Reduction or elimination of this non-specific binding noise will improve device performance, and enhance signal-to-noise ratios, detection sensitivity and specificity of many analytical systems.

In particular, cell binding (in contrast with biomolecule binding) is of great importance in the field of cell microarrays (chips). A cell microarray is a device for assaying selective binding of a cell thereto. For these chips to be efficacious cells must bind to specific areas of the array and the rest of the array must show low cell binding. Cell microarrays are used to trigger local cellular behaviors which can, for example, be indicative of a cellular population identity, its reaction or modulation of its behavior to a special environment or stimulus, or many other factors.

Different approaches have been taken toward the development of cell microarrays. For example, many reviews report the fabrication of protein arrays (Seong et al., Proteomics 3, 2003 ; Snyder et al., Curr Opn in Chem Biol 7, 2003) and give insight as to the fabrication of cell microarrays. Kataoka et al. report the use of hydrophilic dots to immobilize hepatocyte-based biosensors and list geometrical impact on cell behavior (Kataoka et al., ChemBioChem 5, 2004). Zhu et al. presents a soft lithography technique to pattern proteins on low cell binding surfaces for general cell applications (Zhu et al., Biomaterials 25, 2005). Published US patent application 20040147045 (Nelson, July 29, 2004) describes a protein chip fabrication method based on wet chemistry and mentions the compatibility of cells with the chip for fundamental investigations. Published US patent application 20040253607 (Nock et al., December 16, 2004) discloses the screening of phage displayed peptides without clearing of the cell culture using protein arrays described in other patent documents and patent application. WO02052045 describes a fabrication method for channeled protein chips through which cells may pass and enrich rare cells (such as fetal cord cells, patent application WO2005047529).

Also, compositions and methods of preparing functional thin films or surface coatings with low cell binding have been described in US patent 6,844,028. Individually addressable micro-electromagnetic unit array chips have been reported in US patent 6,806,050. Matrix coatings comprising hydrogel for sensing surfaces capable of selective biomolecular interactions have been described in US patent 5,436,161. Coatings for various types of substrate whereby a hydrophilic polymer layer is bound by means of a polymer adhesion promoter layer to a substrate have been disclosed in US patent application 200504455. Compositions and methods for molecular interaction assays performed in solution and on solid surfaces have been reported in US patent 6,878,523. US patent 6,951,761 describes CryoArrays, which permit the analysis of samples (such as protein, nucleic acid, virus, or cell samples) in arrays that are prepared at low temperatures. Methods and devices for making new and inexpensive mini-arrays suitable for gene expression analysis have been described in US patent 7,033,761.

## SUMMARY OF THE INVENTION

The invention relates to assay supports, such as microarrays and similar devices, and methods of use and manufacture thereof. The assay support is capable of assaying binding and/or activity of a bioactive entity, such as a bioactive molecule or a cell.

The present invention provides an assay support for assaying selective binding or response of a cell, said support comprising:

(a) a substrate having a surface, preferably a substrate selected from the group consisting of tissue culture plates, membranes, microscope slides, glass, mica, silicon wafers, plastic materials, polytetrafluoroethylene, FEP materials, polyurethanes, polypropylene, polycarbonate, polyethylene, metallic substrate, gold-based substrate, silver-based substrate, stainless steel-based substrate, titanium-based substrate, palladium-based substrate and copper-based substrate;

(b) one or more first layers covalently attached to one or more areas of said surface, said first layers exhibiting low-cell binding properties; and

(c) bioactive molecules which are covalently attached to said one or more first layer and which are selected from the group consisting of natural proteins, recombinant proteins, growth factors, antibodies, enzymes, peptides, drugs, amino acid sequences, lipids, polysaccharides, proteoglycans, vitamins and mixtures thereof, comprised in one or more discrete regions of said first layer, said bioactive molecules being capable of cell binding,

wherein said support is obtainable by covalently attaching said one or more first layers to said one or more areas of said surface, and disposing said bioactive molecules onto one or more discrete regions of the first layer.

The invention further provides a method of manufacturing a support for assaying selective binding or response of a cell. This method comprises the steps of (a) covalently attaching one or more first layers to one or more areas of a surface of a substrate, the one or more first layers exhibiting low-cell binding properties; and (b) disposing bioactive molecules onto one or more discrete regions of the first layer, the bioactive molecules being capable of cell binding.

The method of the invention may further comprise, prior to covalently attaching the first layer, the step of modifying the surface, for example using traditional wet chemistry techniques or by plasma enhanced chemical vapour deposition.

The invention further provides a support produced by the method of the invention.

In embodiments the supports and methods of the invention, low-cell binding properties lead to a statistically significant lower number of cells binding to the first layer per area unit relative to the number of cells binding to the discrete region, wherein the statistically significant lower number of cell has a p-value of 0.05 or less.

Also, in embodiments of the supports and methods of the invention, the substrate may be selected from the group consisting of tissue culture plates, membranes, microscope slides, glass, mica, silicon wafers, plastic materials, polytetrafluoroethylene, FEP materials, polyurethanes, polypropylene, polycarbonate, polyethylene, metallic substrate, gold-based substrate, silver-based substrate, stainless steel-based substrate, titanium-based substrate, palladium-based substrate and copper-based substrate.

In embodiments, the surface of the substrate may be modified using traditional wet chemistry techniques or by plasma enhanced chemical vapor deposition.

In embodiments, the supports and methods of the invention, the first layer may comprise a poly(alkylene glycol), e.g., a poly(ethylene glycol) (PEG)-based polymer. The PEG-based polymer may be linear PEG or star PEG. In an embodiment, the PEG-based polymer may linear PEG. The linear PEG may be attached to the substrate from a PEG solution in cloud point conditions.

"Cloud point conditions as used herein, refer to conditions under which a polymer agglomerates or aggregates based on the principle that some polymers undergo agglomeration or aggregation under certain conditions (e.g., at certain salt [e.g., high salt] concentrations and/or at certain temperature conditions. The type of e.g., salt, the salt concentration, and/or the temperature conditions needed for the polymer to agglomerates/aggregates differs from polymer to polymer. Such a solution can be obtained for example by first dissolving the polymer in an adequate solvent and then e.g., adding one or more salts to the solution and/or changing its temperature until a cloudy appearance, which is indicative of the presence of polymer aggregates, is obtained.

Also, in embodiments, the first layer may comprise a polysaccharide. The polysaccharide may be carboxy methyl dextran (CMD). The CMD may be attached to the substrate from a solution having a minimum EDC:COOH ratio.

In embodiments, the first layer may comprise a polyelectrolyte. In an embodiment, the polyelectrolyte may be poly(acrylic acid).

The bioactive molecules are selected from the group consisting of natural proteins, recombinant proteins, growth factors, enzymes, peptides, drugs, amino acid sequences, lipids, polysaccharides, proteoglycans, vitamins and mixture thereof. Also, the bioactive molecules may be covalently attached to the first layer. In an embodiment, the bioactive molecules may be covalently attached to the first layer under dew-point conditions.

In embodiments, the support of the invention may comprise two or more discrete regions. The discrete regions may be geometrically arranged on the substrate. In embodiments, the discrete regions may comprise different bioactive molecules or different mixture of bioactive molecules. In more specific embodiments, the bioactive molecules may be deposited onto the first layer using a robotic arrayer. More specifically, the support may be a microarray.

The invention further provides a method of determining cell activity comprising contacting the surface of the support of the invention with the cell.

The invention further provides a method of studying the activity of a compound of interest. This method comprises the step of (a) contacting the surface of the support of invention with a sample comprising cells; and (b) contacting the cells with the compound. More specifically the compound may be a drug.

The invention further provides a method of selectively enriching a first cell from a population of cells, comprising contacting the support of the invention with the population, wherein the bioactive molecules are capable of selectively binding the first cell. In embodiments, the first cell may a hematopoietic cell.

The invention further provides a method of immobilizing cells comprising contacting the support of the invention with the cells. In embodiments, the cells may be hematopoietic cells, myoblasts, endothelial cells, fibroblasts or platelets.

The invention further provides a method of promoting attachment, proliferation and differentiation of cells comprising contacting the support of the invention with the cells. In more specific embodiments, the cells may be myoblasts.

The invention further provides a method of promoting cells behaviors comprising contacting the support of the invention with the cells. In embodiments, the cells may be endothelial cells or fibroblasts.

The invention further provides a method of detecting activated cells comprising contacting the support of the invention with the cells. In an embodiment, the cells may be platelets.

Therefore as described above, assay supports, such as microarrays and similar devices, and their methods of use and manufacture are provided. More specifically, the assay support is capable of assaying binding and/or activity of a bioactive entity, such as a bioactive molecule or a cell.

The assay support of the present invention comprises a layer exhibiting low cell binding covalently attached to a substrate. Non limiting examples of substrates that may be used are: tissue culture plates, membranes, microscope slides, glass, mica, silicon wafers, any plastic materials such as Teflon™ (polytetrafluoroethylene) and perfluorinated poly(ethylene-co-propylene) (FEP) materials, polyurethanes, polypropylene, polycarbonate, polyethylene, etc., and any metallic surfaces and/or substrates such as gold-based, silver-based, stainless steel-based, titanium-based, palladium-based, copper-based. Such a substrate may be selected on the basis of the end-use applications. For example, in the case of cell culture, the solid substrate is selected based on the characterization methods later used to evaluate cell behavior.

Advantageously, substrates may be cleaned using appropriate techniques. For example, FEP (Dupont) and mica are prepared first by taking a sample of approximately 4 $cm^2$ and cleaning it in a solution 5:1 of surfactant and HPLC-grade methanol. Then, using tweezers cleaned with the same solution, the sample is rinsed with deionized water (Millipore) and blow dried with an air gun equipped with a filtration membrane of 0.2 $\mu$m diameter (Millipore).

Plasma enhanced chemical vapor deposition (PECVD) may for example be used to create stable reactive layers. This method of surface modification has been applied extensively in the biomaterials field because the films produced are very smooth and defect-free and can be deposited onto many different materials and complex geometries. Typically, the substrates to be modified are introduced into a chamber made of Teflon and glass. A gaseous monomer is introduced in the reactor chamber until a target pressure is reached. The PECVD process is initiated by applying a radio-frequency current between two electrodes between which the substrate is placed. The operating conditions depend on the choice of monomer, which in turn depends on the desired chemical end-result wanted.

Depending on the nature of the substrate and the end-result to be achieved, other surface modification techniques may also be used, such as traditional wet chemistry techniques and more specifically, the use of standard wet chemistry to modify glass substrates.

To create the assay supports of the invention, for example, a layer of a low-cell binding polymer is covalently attached to the substrate. A low-cell binding layer is a layer that limits cell attachment and responses to a level at which such layer can be used to distinguish bio-specific cell responses on regions bearing bioactive molecules or niches (i.e. signals) to those bearing no bioactive signals (i.e. those bearing low-cell binding surfaces only), thus providing an improved signal to noise ratio under assay conditions. Such a layer can be defined as a surface on which adhesion to the regions with the bioactive molecules rather than to the low cell binding areas is expressed for investigated cells, as a statistically significant lower number (p-value of 0.05 or less) of cells on the low cell binding area (per unit area) than on the bioactive spots. The low-cell binding potential of this layer is achieved by the presence of synthetic or natural polymers covalently attached to the substrate supporting the low cell binding layer.

To produce surfaces with varying low-cell binding properties, polymers and/or biopolymers that have different structural and/or interfacial properties may be deposited on various substrates. Non limiting examples of such polymers and/or biopolymers are poly(acrylic acid) (PAAC), linear and star poly(ethylene glycol) (PEG), and carboxy methyl dextran (CMD). More generally, PEG-based polymers, such as poloxamers (e.g., Pluronics), polysaccharides (such as CMD) and polyelectrolytes (such as PAAC) may be used. Other materials, such as phospholipids, and other polymers, such as chitosan and pullulan, may also be used. Actually, any polymer, biopolymer, polysaccharide or polyelectrolytes exhibiting low-cell binding may be used.

The characteristics of the underlying low-cell binding polymers can be modulated to achieve different end results by varying the polymer conditions of immobilization. Non limiting examples of parameters that can be systematically varied to produce underlying layers with different characteristics are the type of polymer used, the molecular weight of the polymer, the ratio of different polymer in a mixture of polymers, the conditions of the polymer immobilization onto the substrate (for example, the amount of catalyst used), the addition of salts during the immobilization (particularly for polyelectrolytes), the degree of cross-linking of the polymer and the functional groups of the polymer.

In an embodiment, linear PEG may be covalently attached to the substrate from a PEG solution in could point conditions. The PEG concentration of this solution may be adjusted to the type of salt and its associated concentration necessary to produce cloud point conditions as can easily be determined by the person of ordinary skill in the art. For example, in some conditions, this solution may have a PEG concentration higher than about 1 mg/mL.

In other embodiments, CMD may be attached to the substrate from a solution having a minimum EDC:COOH ratio adapted to the degree of carboxylation and the concentration of CMD as can easily be determined by a person of ordinary skill in the art. For example, in some conditions, a EDC:COOH ratio greater or equal to 5:1 may be used. In addition, a PAAC layer may be attached from a PACC solution.

Bioactive molecules are covalently attached, to A the previously deposited low-cell binding layer. A variety of chemical reactions may be used depending on the molecules present and on the end-objective of the surface. For example, the use of poly(ethylene glycol) low-cell binding layers may imply chemical de-protection of the layer to expose functional chemical groups. Also, for covalent attachment, the evaporation of the bioactive molecules solutions during the immobilization reaction may be advantageously diminished or prevented by placing the substrates in chambers keeping the surfaces close to the dew point conditions.

Examples of bioactive molecules are proteins, growth factors, enzymes, peptides, and/or drugs. Examples of bioactive molecules which may be deposited following the deposition of a low-cell binding underlayer are recombinant or natural proteins (including, but not limited to, growth factors, antibodies, and enzymes), peptides, amino acid sequences, lipids, polysaccharides, roteoglycans, and vitamins. In embodiments, a mixture of such molecules may be deposited.

The bioactive molecules solutions can be, for example, composed of proteins in a sterile buffer solution (e.g., PBS buffer at about neutral pH (e.g., pH 7.4)). The concentrations can be, for example, varied from less than about 0.1 ng/ml to more than about 100 mg/ml. These bioactive molecules may form one or more discrete regions on the support.

It is possible to use various coupling agents to link the bioactive molecules to the low cell binding layer. It is also possible to add molecular spacers.

Chemical gradients and varied bioactive signals can be achieved on the surfaces by modifying the chemical nature of the bioactive molecules deposited on the low cell binding substrate.

The physicochemical characteristics of the discrete regions may be controlled by modifying, for example, the surface molecular composition, the integrity of the attached molecules, the thickness of the low-cell binding layer, and/or the forces applied by the polymer chains. The biological characteristics of the discrete regions may be controlled by modifying, for example, the bio-activity of the bioactive molecules used.

The bioactive molecules may be precisely arrayed in the form of small droplets and covalently attached in the form of discrete regions on the previously deposited polymers/biopolymers at precise geometrical positions on the surfaces, thereby forming a microarray. Solutions containing the bioactive molecule(s) to be immobilized may be advantageously deposited on the low-cell binding layers using a robotic arrayer (e.g., BioChip Arrayer™, Perkin Elmer) with arraying capabilities such as those used to manufacture DNA and protein chips. This robot may, for example, deposit a minimum of 300 pl liquid material at precise locations on the surfaces. This facilitates the fabrication of precisely delimited areas on the surface presenting different or identical droplets of the bioactive molecule(s) encircled by an immobilized low-cell binding polymer.

The microarrays may expose at well known coordinates on the X-Y axis various covalently attached bioactive molecules with, for example, a gradient of composition and/or with various low-cell binding areas with specific properties. Such a chemical gradient and varied bioactive signals may be achieved on the surface by modifying the chemical nature of the droplets deposited on the low-cell binding layer. The result, for example, would look like a 200-, 500-, 1,000-, or 10,000-spot domino, with each spot (discrete region) consisting of a different bioactive molecule or mixture of different bioactive molecules, which can then be tested for various purposes using living cells (e.g., cell growth, medical diagnostic). The number of spots may depend on the surface area of the solid substrate and the surface area that is needed for the application (for living cells to attach, for example). These arrays of bioactive molecules have a flexible design, wherein, among others the size and layout of the spots may be varied at will.

The assay supports of the invention may be used by placing their surface in contact with cells (e.g., eukaryotic cells, prokaryotic cells, bacteria, yeasts, plant cells, animal cells) bathed in a liquid environment. For long-term applications, the supports and the cells may be maintained in a sterile environment. Depending on the application, exposure of the supports to the cells can be followed by the use of a variety of techniques, such as fluorescent labeling of the cells or the substrate, microscopy techniques or biochemical assays. It is also possible to monitor specific cells events on the surfaces (for example using microscopy and other biological assays).

Non-limiting examples of different characterization techniques that may be used to probe the supports and microarrays of the invention are X-ray Photoelectron Spectroscopy (XPS), Atomic Force Microscopy (AFM), Surface Plasmon Resonance (SPR), Matrix Assisted Laser Desorption Ionization - Mass Spectrometry (MALDI-MS), fluorescence labeling, etc. Many techniques can be utilized to monitor the quality and characteristics of the arrays, for example:

- X-ray photoelectron spectroscopy can be used to quantify the atomic O, C, N atomic concentrations of the different spots of a given array, and the ratios of O/C and N/C can be deduced for the different spots;

- atomic force microscopy in imaging and force modes can be used to measure the roughness of the spots, the forces applied by the bioactive molecules on approaching objects (steric, repulsive, attractive) and to know the effects of different saline solution on the extension of the chains;

- surface plasmon resonance allows the approximate measure of the thickness of the spots and to determine the presence of the spots;

- antibody staining can be used to assess the presence and activity of specific bioactive molecules immobilized on the surfaces.

Final testing also implicates cells and tests involving, for example, the adhesion of the cells in different areas of the substrates can be undertaken. The link between cellular behavior and exact composition and dimensions of the spots may be routinely determined for each particular system used and can be exploited for different applications.

The assay supports of the invention can find applications in several areas. One such area is cell research. Thus, supports of the invention can be, for example, custom-made to study the behavior of special cell lines under different localized stimuli.

In embodiments, various cell types may be used in the context of the invention, including, for example, eukaryotic cells, prokaryotic cells, animal cells, plant cells, mammalian cells, fibroblasts, stem cells, hematopoietic cells, myoblasts, and entdothelial cells.

Another area of application is medical and biological diagnostics. Many diseases are still difficult and/or costly to identify and assay supports of the invention could represent a high-throughput solution to the identification of cells presenting clinical significance. For example, cells from blood can be placed in contact with specifically designed "virgin" chips (without cells) and allowed to sediment on the surface. The surface can then be rinsed and observed under a standard microscope to locate cells having adhered on the surface. The presence of specific patterns of cells immobilized on the surface could then be directly and rapidly correlated with the presence of defective or unusual cells only present in certain diseases (e.g., cancer cells). Other diagnostic applications could be, for example, the detection of pathogens in water treatment. The assay supports of the invention could also be useful as medical products based on cell behavior and recognition through supports of the invention for prenatal testing, general disease identification and tissue regeneration.

Another area is the testing of new drugs. Out of the many molecules having a possible therapeutic potential, only a very few will prove to be efficient to act adequately on diseased cells after lengthy and costly animal testing. Assay supports of the invention could provide preliminary screening to quickly determine molecules that have a chance of succeeding the animal testing. Many different molecules at different concentrations, or mixtures of molecules, could be placed on individual cells immobilized on chips and their response to the drugs monitored rapidly.

Yet another area is stem cell applications and tissue engineering. Stem cells have the potential to revolutionize medicine. However, they have proved to be very sensitive to their culture environment and this is the main limiting factor for their widespread use in therapies. Assay supports of the invention can be used to study and modulate stem cell behavior and differentiation patterns by presenting to the cells specific biological signals. Stem cells could then be "tamed" and used for tissue reconstruction.

Still, another area is rare cell enrichment. Currently, rare cells (mainly different types of stem cells or cancer cells) have to be enriched by costly means to be useful to medicine and research (either for diagnostic or future use). The two major techniques currently used are the Fluorescence Activated Cell Sorting (FACS) and magnetic beads techniques. Assay supports of the invention could provide a much cheaper alternative to the previous two techniques by stimulating the adhesion of the rare cells on the chip surface, thereby eliminating non-useful cells.

Other examples of applications are:

- Local immobilization or repulsion of endothelial cells from umbilical blood for the *in vitro* study and modulation of angiogenesis.

- Local proliferation and immobilization of adult muscle stem cells for the *in vitro* study of cellular differentiation.

- Local immobilization of non-adherent hematopoietic cells for the *in vitro* study of hematopoiesis.

- Local immobilization of human fibroblast for high-throughput pharmaceutical testing.

- Selective immobilization of activated platelets from whole blood for cardiovascular medical diagnostic.

- Selective immobilization of specific white blood cells (T cells) activated in Alzheimer's disease for early diagnostic of the pathology.

- Membrane surface treatment for the selective filtration of cells of interest.

The assay supports of the invention have been demonstrated to be useful for a wide array of adherent and non-adherent cells and are therefore useful to trigger specific cell responses over a wide range of cells. More specifically, the bioactive signals to detect platelet activation, to enrich hematopoietic cells, to promote myoblast attachment, proliferation, and differentiation and/or to promote endothelial cells and fibroblasts behaviors, which may be covalently attached, physisorbed and/or specifically attached on low-cell binding surfaces, have been covalently attached while retaining their bioactivity. Platelet activation has been detected using the microarray based on low-cell binding surfaces and bioactive niches described herein. Also, hematopoietic cell enrichment and attachment has been carried out, myoblast attachment, proliferation, and differentiation has been promoted and endothelial cell and fibroblast attachment, proliferation, and migration has been promoted using assay supports of the invention. The results of all these tests were statistically significant revealing a statistically significant lower number of cells per unit area on the low-cell binding area (p-value of 0.05) than on the bioactive spots.

Moreover, the assay support of the invention will also be useful for medical diagnostic and the study of cell-material interactions.

Other objects, advantages and features of the present invention will become more apparent upon reading of the following non-restrictive description of specific embodiments thereof, given by way of example only with reference to the accompanying drawings.

## BRIEF DESCRIPTION OF THE DRAWINGS

In the appended drawings:

Figure 1 is a XPS C 1s high-resolution spectra of different PEG-covered samples;

Figure 2 shows QCM hydration data of four PEG-covered surfaces with water injection at 3 minutes;

Figure 3 shows QCM dynamic data of protein adsorption on different PEG-covered surfaces with injection of the FBS solution at 5 minutes;

Figure 4 shows compression AFM force curves using the colloidal probe method for different PEG samples in water (A) and in RPMI buffer (B);

Figure 5 is the size distribution of PEG agglomerates in theta solvent in number % [The size distributions shown were stable over time (i.e. from injection to end of experiment) for all PEG concentrations.];

Figure 6 is the mean PEG aggregate size in theta solvent in volume % as a function of time;

Figure 7 is the fluorescence intensity measured from covalently-bound CF fluorescence intensity and adsorbed CF for different PEG layers. On this figure, * indicates non-homogenous fluorescence and ** indicates non-cloud point sample and non-homogenous fluorescence [Insert shows a typical CF geometrical design immobilized on a 1 mg/ml PEG sample produced under cloud point.];

Figure 8 is a schematic of the platelet activation measurement technique;

Figure 9 is a schematic of the disposition of bioactive molecules on the PEG surfaces for the platelet activation study [Image is not to scale. Dots are separated from each other by a distance of 300 microns.];

Figure 10 is a typical ELISA result showing local NBT-NCIB precipitation upon reaction with surface-immobilized secondary antibody [The area presented here corresponds to the 50 µg/ml CD62 droplet deposition region.];

Figure 11 is the flow chamber designed to (1) circulate blood between parallel plates at a selected surface shear rate, (2) limit blood exposure to air, and (3) allow continuous observation of samples during blood circulation [The silicone gasket can be fabricated into different thicknesses to modify the slit width.];

Figure 12 presents the simulation results showing shear rate distribution in the flow chamber for a 6ml/min flow rate [The average surface shear rates were $1300s^{-1}$ for the 0.2-mm slit and $63s^{-1}$ for the 1-mm slit.];

Figure 13 shows the fluorescently-labeled surface-adhered platelets on anti-CD62 50µg/ml spots with artificial activation of the platelets using collagen (A) and without artificial activation (B) and on anti-CD61 50 µg/ml spots with artificial activation of the platelets using collagen (C) and without artificial activation (D) [The circles represent the protein array as defined by the mask. The statistical test is positive for all the spots in A, C and D, but negative for those in B (p-value < 0.05). White particles represent aggregated platelets.];

Figure 14 is a schematic of the disposition of bioactive molecules on the PEG surfaces [image is not to scale; dots are separated from each other by a distance of 300 microns.];

Figure 15 shows 48-hour cell adhesion to stem cell factor signal on 1 mg/ml PEG sample;

Figure 16 shows a chip design exhibiting 100 µg/ml fibronectin spots deposited on 1 mg/ml PEG surfaces;

Figure 17 shows the results of a 60-hour myoblast exposition to fibronectin-based chips;

Figure 18 shows the guiding system to localize individual CMD spots for XPS and AFM analyses;

Figure 19 is (a) a high-resolution XPS C 1s spectra of a freshly deposited HApp layer and of CMD spots made under conditions #1 and #3, and (b) a high-resolution XPS C 1s spectra of CMD layers produced under conditions #1 on a fully covered surface and on an array;

Figure 20 shows representative colloidal probe force measurements between a silica colloidal probe and HApp layers on a polymer array in two PBS solutions (pH 7.4);

Figure 21 shows representative colloidal probe force measurements between a silica colloidal probe and spots of CMD grafted on HApp surface using high EDC+NHS/COOH ratios in two PBS solutions, (a) is silica-CMD layers#1 and 6 and silica-CMD layers#3 and (b) is silica-CMD layers#8;

Figure 22 shows representative colloidal probe force measurements between a silica colloidal probe and spots of CMD grafted on HApp surface using low EDC+NHS/COOH ratios in two PBS solutions, (a) is silica-CMD layers#4 and 7, and (b) is silica-CMD layers#2 and 5;

Figure 23 shows protein adsorption on spots of CMD graft layers evaluated by SPR microscopy, (a) is an image of CMD layers produced in conditions #2 and #5 obtained at plasmon angle $\theta_{SPR}$ (scale bars: 500µm), (b) shows the SPR angle shifts (°) resulting from FBS protein adsorption on CMD arrays [Conditions #9 involve the use of 70kDa CMD, 25% carboxylation degree, high EDC+NHS/COOH ratio, and a 2mg/ml CMD solution concentration.];

Figure 24 is a schematic picture (not to scale) of immobilized CMD molecules [Final layer conformation can be controlled by electrolyte concentration and coupling agent ratio (EDC+NHS/COOH) during immobilization.];

Figure 25 shows optical microscope images of fibroblasts seeded on CMD arrays following 4-hour (a,b) and 12-hour (c,d) cell seeding on CMD spots made using conditions #8 (a,c) and conditions #3 (b,d);

Figure 26 shows the initial cell behavior on CMD arrays of cells on the HApp layer (a,b,c) and on a CMD spot (d,e,f: conditions #1 and g,h,i: conditions #7) [Cells were fixed 4 hours following cell seeding.];

Figure 27 shows the initial cell behavior on CMD arrays of cells on the HApp layer (a-c), on cell-resistant CMD spots (d-f, conditions #9), and on CMD layers made using conditions #4 (g-i) [Cells were fixed 12 hours following cell seeding.];

Figure 28 shows the CMD spots surface coverage following 3-day exposition to confluent cells [Spot size was measured each day and compared to initial spot size. (*) Some spots were completely covered on Day 3, therefore the average initial size was not 1, resulting in larger standard deviations.];

Figure 29 shows the CMD spots exposed to confluent fibroblasts for a period of 3 days for CMD layers made using conditions # 5 (a-c), #2 (d-f), and #9 (g-i).

Figure 30 shows fibroblast actin cytoskeleton (a,d,g,j), focal adhesion formation (b,e,h,k) and human fibronectin deposition and reorganization (c,f,i,l) after 3 days of confluence on CMD spots for cells found on HApp surfaces (a,b), at the edge of CMD spots made using conditions #1, #3, and #9 (c-f), on CMD spot made using conditions #6 (g,h,i,k), and on CMD spot made using conditions #5 (j,l);

Figure 31 shows the determination of an optimal cell-resistant CMD surface [CMD surfaces immobilized using optimized conditions and various carboxylation degrees (referred to as CMD-5, -25 and -50) and PEG surfaces made under cloud point conditions (PEG-CP) were exposed to confluent fibroblasts for a period of 3 days.] (A) spot size evolution, and (B): optical microscope images of spots over the 3 days;

Figure 32 shows endothelial cells on a RGD spot after 3 days of culture with 10% serum [Biomolecules array was made on a cell-resistant CMD surface.];

Figure 33 shows endothelial cells (A) and fibroblasts (B) density on biomolecules spots 6 hours after seeding with no serum;

Figure 34 shows endothelial cells shape factor 6 hours after seeding on biomolecules arrays;

Figure 35 is the expression of endothelial cell specific markers after 3 days of culture on biomolecules arrays - labeling of VE-cadherin (A) and von Willebrand factor (B);

Figure 36 is a schematic of a microarray according to an illustrative embodiment of the invention; and

Figure 37 is a schematic showing, in an embodiment, fabrication steps of a support according to an illustrative embodiment of the invention.

## DESCRIPTION OF ILLUSTRATIVE EMBODIMENTS

The present invention is illustrated in further details by the following non-limiting examples.

## EXAMPLE 1

### PEG-Based Surfaces

Low-cell binding amine-terminated poly(ethylene glycol) thin layers were prepared. The effect of the PEG-like molecules immobilization conditions on their mechanical and physicochemical properties were studied.

The physicochemical and mechanical properties of amine-terminated covalently-bound poly(ethylene glycol) (PEG) layers are dependent on fabrication methods. For example, the use of a theta solvent yields dense polymer layers with properties not well described by traditional models. Light diffraction techniques were used to understand the influence of polymer aggregation kinetics in a theta solvent on the final properties of the fabricated PEG layers. The polymer layer properties were characterized using X-ray photoelectron spectroscopy (XPS), atomic force microscopy (AFM) in force mode, quartz crystal microbalance (QCM) and fluorescence microscopy. Results show that polymer concentration in solution is an indicator of final layer properties, and that the use of a theta solvent induces complex aggregation phenomena in solution yielding layers with unique properties. The PEG layers fabricated through the process described herein are also shown to be chemically reactive, and thus conducive for the immobilization of bioactive molecules.

Poly(ethylene glycol)-modified (PEG-modified) surfaces have good anti-biofouling (fouling by biomolecules) properties. PEG layers are generally thought to provide a repulsive interaction barrier through steric repulsion and hydration/water structuring of greater magnitude and longer range than either attractive electrostatic or van der Waals interactions [Vermette, P.; Meagher, L. Colloids and Surfaces B-Biointerfaces 2003, 28, 153-198].

An optimized and characterized PEG-based system allowing the localized covalent binding of a variety of bioactive molecules onto a wide range of substrates has been developed. Efforts were placed to understand the effect of reaction parameters on physicochemical and mechanical hydrated PEG layer properties. Quartz crystal microbalance (QCM), atomic force microscopy (AFM) using the colloidal probe force measurement and X-ray photoelectron spectroscopy (XPS) were used in this regard. Studies were carried out to assess the possible effect of polymer aggregate kinetics in solution at different polymer concentrations on PEG layer properties. The measure of aggregate sizes was achieved using light diffraction. Moreover, patterns of covalently-bound molecules were created onto the PEG layers in a chip-like fashion. The possibility to produce defined patterns was assessed by fluorescence techniques.

### Plasma polymer surfaces

Plasma enhanced chemical vapor deposition (PECVD) was used to create stable reactive amine-bearing layers [Griesser et al. Journal of Colloid and Interface Science 2003, 259, 13-26.]. Substrates to be modified were introduced into a home-made cylindrical plasma reactor. This reactor was composed of several sub-units. The reactor chamber was made of a glass cylinder (30-cm outside diameter, 1-cm glass thickness, 50-cm height). Two disk-shaped horizontal copper electrodes (14-cm diameter, 5-mm thickness) were maintained in the chamber by vertical copper rods (top rod: 7-mm diameter with an adjustable vertical position; bottom rod: 2.5-cm diameter, 17-cm chamber length). The rods were screwed into the electrodes, and were fixed to Teflon plates (35-cm diameter, 5-cm thickness) at the top and bottom of the chamber. The top rod could be moved vertically to modify the distance between the electrodes. The glass chamber was fitted to the Teflon™ plates through circular slits encasing Viton gaskets. The monomer was stored in a round-bottom flask and its entrance in the reactor chamber was controlled using a manual diaphragm valve. The monomer entered the chamber from the top Teflon™; plate through an FEP tubing (6.4-mm internal diameter). FEP is a polymer made of a combination tetrafluoroethylene and hexafluoropropylene. All tubings between the pump and the reactor chamber were made of stainless steel. The monomer exiting the reactor chamber was trapped by a nitrogen cold trap from MDC Inc. (Hayward, CA, model 434011) before reaching the vacuum rotary pump (Trivac® D 25 BCS from Leybold, Hanau, Germany). The pressure in the reactor chamber was measured by a Baratron® capacitance manometer (model #627801TQC1B, MKS Instruments Inc., Anover, MA) and was electronically regulated at the required level by a butterfly control valve (model #000890260, MKS Instruments Inc., Anover, MA) linked to an exhaust valve controller type 252 (MKS Instruments Inc., Anover, MA). The electrical field within the reactor chamber was generated by an AC RF power generator LF-5 from RFPP Inc. (Voorhees, NJ) with a working power range of 10 to 200 W and a frequency range of 50 to 400 kHz.

N-heptylamine monomer (Sigma, Ontario (CAN), cat. 126802) was introduced in the reactor chamber until a pressure of 40 mTorr was reached. The PECVD process was initiated by applying a radio-frequency current between two electrodes. The samples were deposited onto the bottom electrode. The operating conditions were set at a frequency of 50 kHz, a glow discharge power of 80 watts, a distance between the electrodes of 10 cm, and a deposition time of 45 seconds.

PEG layer fabrication

Heptylamine plasma polymer (HApp) substrates were made to react at room temperature in a solution of Boc-$NH_2$-poly(ethylene glycol)-NHS of 3,400-Da molecular weight from Nektar Therapeutics (San Carlos (CA), cat. 4M530F02). The PEG concentration was varied from 0.1 mg/ml to 5 mg/ml in either de-ionized water or in a theta solvent, i.e. in this case a $Na_2SO_4$ solution near saturation (Sigma, Ontario (CAN), cat. 239313). Samples were allowed to react overnight under agitation and rinsed repeatedly in saline solutions and de-ionized water to remove salts and unbound PEG molecules. Varied substrates were used to immobilize the PEG layers depending on end-use. Perfluorinated poly (ethylene-co-propylene) tape from Dupont (Teflon FEP Type A, Mississauga (CAN)) was used for XPS analyses and fluorescence studies. This substrate was chosen mainly for its ease of use and the possibility to detect a fluorine signal in XPS analyses. Quartz substrates coated with gold electrodes for QCM analyses were obtained from Maxtek (CA, 5 MHz blanks). Cleaved mica substrates were used for AFM force measurements. All substrates were cleaned in RBS 35 surfactant (Pierce, Rockford (IL), cat. 27952), rinsed in water and in ethanol, and blown-dried using filtered air. Immobilized Boc-NH2-PEG-plasma layers were Boc-deprotected following manufacturer's recommendations by immersing the substrates in a 50% v/v solution of trifluoroacetic acid (Sigma, Ontario (CAN), cat. T62200) and dichloromethane (Sigma, Ontario (CAN), cat. D65100) for one hour. The substrates were afterwards repeatedly rinsed in a 5% v/v solution of n,n-diisopropylethylamine (Sigma, Ontario (CAN), cat. D3887) in dichloromethane.

Analysis of surface chemical composition by X-ray photoelectron spectroscopy (XPS) measurements

XPS analyses were performed using an AXIS HSi spectrometer from Kratos Analytical Ltd. (Manchester, UK). XPS measurements were conducted with a monochromated aluminum K alpha source at a power of 180 Watts. Pressure of the XPS chamber during measurements was lower than $5 \times 10^{-8}$ mbar. FEP samples were fixed on XPS holder by a silver glue to prevent charge accumulation during measurements. High-resolution C 1s and N 1s spectra, along with survey spectra, were taken for each sample. Moreover, at least three different areas on each substrate were analyzed. Each condition was represented by at least three samples.

Analysis of viscoelastic properties of PEG layers and protein adsorption by quartz crystal microbalance (QCM) measurements

Quartz crystal microbalance measurements were performed using an apparatus from Resonant Probes GmbH (Hochgrevestr, Germany). The gold-coated quartz resonators were placed into a commercial holder (Maxtek (CA), CHT100). Data from the network analyzer (Agilent, Palo Alto (CA), HP4396A) was analyzed using the software from Resonant Probes. For protein adsorption assays, PEG-coated substrates were placed in the QCM holder immersed in a 37°C bath to maintain the temperature constant. RPMI 1640 medium (Sigma, Ontario (CAN), cat. R8758) at the same temperature was automatically injected at a rate of 10 ml/min into the QCM chamber. Upon stability of the signal (at least one hour), a solution of 10% v/v de-complemented fetal bovine serum (Sigma, Ontario (CAN), cat. F2442) in RPMI 1640 was injected into the chamber at 10 ml/min. This injection was normalized at time 5 minutes to compare different layers. The choice of the protein medium used for the adsorption tests was dictated by the wide range of proteins contained in FBS, so that the versatility of protein resistance of the layers could be assessed. The frequency and half-band-half-width shift (i.e. the dissipation times the frequency of vibration divided by two) were recorded by the software to monitor changes in layer mass (e.g., adsorption of proteins) and in layer viscoelastic properties.

For PEG grafting monitoring (i.e. so-called PEGylation), plasma-modified QCM substrates were rapidly placed into the holder in contact with water. The frequencies of harmonic vibrations were located and a Boc-PEG-NHS solution was automatically introduced into the holder at a rate of 10 ml/min. The frequency and half-band-half-width signals were recorded as for the adsorption assays. In this case, the whole system was maintained at room temperature (i.e. 22°C) for the PEGylation tests. For hydration assays, PEG layers covalently attached onto the quartz resonators were blown dried and placed into a desiccator for at least 48 hours. The crystals were placed rapidly into the dry holder and harmonics were taken in air. Water was injected at a rate of 10 ml/min for thirty seconds and variations in frequency and half-band-half-width were measured until stabilization of the signals (at least one hour). Other water injections were made to ensure a stable signal. All QCM experiments were carried out in at least two different samples for each condition. For all QCM measurements, data were taken at least for harmonics located at 15, 25, 35 and 45 MHz (the fundamental frequency being 5 MHz). Harmonic number 5 (i.e. 25 MHz) is the harmonic reported in the graphics presented herein.

Analysts of structure of PEG layers by atomic force microscopy (AFM) force measurements

A Bioscope (Digital Instruments, Santa Barbara, CA) coupled to a Zeiss inverted microscope and linked to a Nanoscope IIIa controller was used to conduct force measurements on the hydrated PEG layers based on a colloidal probe method. Briefly, the AFM was operated at a frequency of 1 Hz and ramp sizes of 100, 250 and 500 nm, recording both approaching and extending data. Silica microspheres of 4.12 $\mu$m of diameter (Bangs Laboratories, Fisher (IN), cat. SS05N) were attached using medical-grade epoxy-type glue (Master Bond, Hackensack (NJ), cat. EP30MED) to $Si_3N_4$ cantilevers with a measured spring constant of 0.137 N/m (Veeco, Woodbury (NY), DNP-S) using the resonance method proposed by Cleveland et al. [Cleveland et al. Review of Scientific Instruments 1993, 64, 403-405]. Cantilever deflection and z-piezo position was transformed into force-versus-distance curves. Force measures were done at room temperature in 100, 10 and 1 mOsm NaCl solutions buffered in 10 mM Hepes, in RPMI 1640 media and in deionized water, allowing for at least 1 hour of stabilization before taking measures. At least three different spots were studied on each sample, and at least 20 force curves were recorded for each spot.

PEG aggregation measurements during cloud point conditions

While in a theta solvent, the Boc-PEG-NHS polymer forms aggregates that are visible to the naked eye through the formation of cloudiness in the solution (thus the term cloud point"). A Malvern Mastersizer 2000 (Malvern Instruments, Malvern, UK) was used to measure the aggregate size distribution based on light diffraction (i.e. aggregates yield different diffraction patterns relatively to their size). The particle size was determined as a function of time using a measured refractive index for the solvent of 1.35787 (using a refractometer from Schmidt+Haensch, model DUR-W2, GER) and a refractive index of 1.47 for the PEG aggregates. The approximate shear rate during the measures was of 34 s$^{-1}$ corresponding to a stirring rate of 150 rpm. The instrument was thoroughly cleaned before each sample. The solvent was first introduced into the circulation loop of the apparatus. A concentrated homogenized PEG solution was fabricated by hand agitation and rapidly added at time 0 min into the circulation loop of the apparatus to achieve the desired final PEG concentration. This procedure contrasts with other procedures reported to monitor PEG agglomeration in theta solvent where the PEG is completely dissolved in a good solvent such as water before incorporation in the apparatus filled with the theta solvent [Pang, P.; Englezos, P. Colloids and Surfaces A-Physicochemical and Engineering Aspects 2002, 204, 23-30]. The procedure was chosen to mimic the actual use of the PEG molecules to form layers as speed is preferred to avoid hydrolysis of the active ester end-group before exposure to the amine substrate.

Chemical bonding of carboxyfluorescein to the amine-bearing PEG surfaces

A BioChip Arrayer (PerkinElmer, Wellesley (MA)) was used to dispense picoliters of solution to precise locations on PEG surfaces. Carboxyfluorescein (CF, Molecular Probes, Eugene (OR), cat. C-194) was made to react with the de-protected amine groups on the PEG layers through the use of N-ethyl-N'-(3-dimethylaminopropyl)carbodiimide (EDC) (Sigma, Ontario (CAN), cat. E1769) and N-hydroxysuccinimide (NHS) (Sigma, Ontario (CAN), cat. H7377) to form a stable amide bond between the PEG layer and the fluorescent molecules. Solutions with ratios of 0.5:0.5:1 between EDC:NHS:CF using different CF concentrations were placed in multi-well plates. The Biochip Arrayer sampled the solutions to deposit droplets of ca. 300 pl at different locations on the substrates, in this case to form the layer "Y" with dots varyingly spaced. One or both of the activated carboxylic groups on the CF molecule could then react with the amine groups available on the PEG-coated substrates and form stable amide bonds. Samples were then rinsed thoroughly in saline buffer and in de-ionized water while avoiding exposure to light. Fluorescence was preserved by using Fluoro-Guard (Bio-Rad Laboratories, Hercules (CA)) and fixing the FEP samples beneath a glass cover slip with transparent nail polish. Fluorescence on the samples was observed using a Leica binocular microscope with appropriate filters. Photos were taken at an exposure of 1 second and green intensity was measured using SigmaScan Pro software (Systat Software, Richmond (CA)). Calibration curves were constructed to insure the use of proper concentrations of CF to deposit on the surfaces to be in a linear regime and avoid quenching. CF solutions of concentrations ranging from 0.017 to 1.7 mM were thus made to react with three different samples. For each sample, it was observed that the use of a 0.17 mM CF solution was in a linear and non-quenching regime and this concentration was then deposited on all samples to be tested. At least twenty droplets were deposited on each sample and at least two samples were studied for each immobilization conditions.

Chemical properties of the PEG layers

Covalent immobilization of protected Boc-PEG-NHS onto radio-frequency generated plasma reactive amine layer was performed at PEG solution concentrations varying from 0.1 mg/ml to 5 mg/ml in either water or an aqueous $Na_2SO_4$ theta solvent (yielding the so-called cloud point or CP condition). Successful covalent immobilization of the polymer layers can be monitored by XPS measurements. Table 1 presents the atomic composition of different PEG layers obtained from XPS survey spectra.

Table 1: XPS survey data for different PEG-treated samples.

| Sample | %C | %O | %N | %F | O/C | N/C | Exposure to oxygen |
|---|---|---|---|---|---|---|---|
| FEP | 35.8 $\pm$ 0.6 | 0.9 $\pm$ 0.2 | 0.0 | 63 $\pm$ 1 | 0.03 | 0.0 | NA |
| Heptylamine plasma | 87 $\pm$ 1 | 2.5 $\pm$ 0.5 | 10.1 $\pm$ 0.5 | 0.0 | 0.03 | 0.12 | < 30 min |
| Heptylamine plasma | 83.4 $\pm$ 0.8 | 9.3 $\pm$ 0.5 | 7.3 $\pm$ 0.5 | 0.0 | 0.11 | 0.09 | > 3 days |
| PEG 0.1 mg/ml | 81 $\pm$ 1 | 13.0 $\pm$ 0.4 | 5.4 $\pm$ 0.2 | 0.0 | 0.16 | 0.07 | > 3 days |
| PEG 1 mg/ml | 81 $\pm$ 1 | 13 $\pm$ 2 | 5.3 $\pm$ 0.4 | 0.0 | 0.16 | 0.07 | > 3 days |
| PEG 5 mg/ml | 81 $\pm$ 1 | 12.6 $\pm$ 0.6 | 6.1 $\pm$ 0.4 | 0.0 | 0.16 | 0.08 | > 3 days |
| PEG 0.1 mm/ml CP | 81.4 $\pm$ 0.5 | 14.0 $\pm$ 0.5 | 4.6 $\pm$ 0.2 | 0.0 | 0.17 | 0.06 | > 3 days |
| PEG 1 mg/ml CP | 77 $\pm$ 1 | 18.6 $\pm$ 0.8 | 4.5 $\pm$ 0.3 | 0.0 | 0.24 | 0.06 | > 3 days |
| PEG 5 mg/ml CP | 79 $\pm$ 1 | 15.7 $\pm$ 0.9 | 5.3 $\pm$ 0.5 | 0.0 | 0.20 | 0.07 | > 3 days |
| CP denotes samples fabricated from a PEG solution in a cloud point state. | | | | | | | |

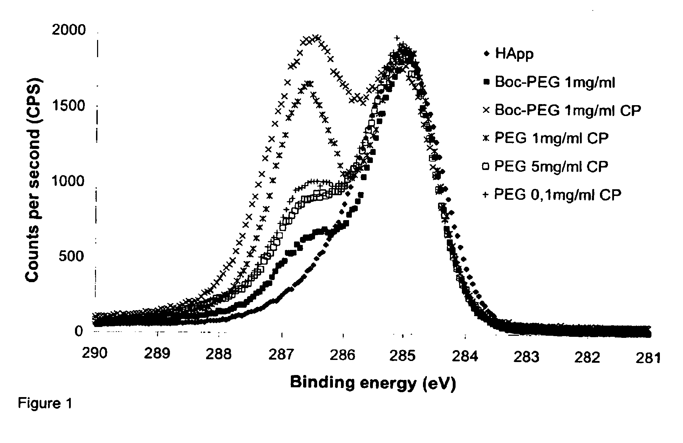

As shown in Table 1 and Figure 1, the use of the theta solvent increases the amount of PEG molecules immobilized on the surface. The observed oxygen present on all PEG-treated surfaces increases markedly compared to the oxidized bare plasma layer. For surfaces where the polymer was immobilized under cloud point conditions, a maximum O/C ratio of 0.24 observed for the 1 mg/ml case. Considering the molecular formula, a pure PEG layer would present an O/C ratio of close to 0.33. Such a high ratio would be very surprising in a non-hydrated environment (even for a brush-like structure that can be adopted by the PEG layers), such as in the XPS chamber, where the PEG chains known to be surrounded by water molecules in an aqueous environment probably collapse when dried and yield layers thinner than 10 nm (i.e. the depth of analysis of the XPS instrument). It is worthy to note that the highest O/C ratio does not come from the highest PEG solution concentration, 5 mg/ml, but from the intermediate concentration of 1 mg/ml.

Table 1 also indicates that the surface chemical composition of PEG layers deposited under good solvent conditions (i.e. non-cloud point) is not affected by the PEG concentration, i.e. changing the solution concentration of the polymer did not significantly modify the O/C ratio for these layers.

Although a high O/C ratio can indicate the presence of the characteristic repeating ether chemical bonds of the PEG molecule, it is impossible to distinguish contributions from other oxygen sources such as the oxidation results of the base plasma layer. High-resolution carbon spectra can then be used to identify the nature of carbon bonds on the surface. Such spectra of the different samples are presented in Figure 1.

By comparing the oxidized heptylamine plasma layer (HApp) of Figure 1 to the immobilized PEG layers, it is clear that the ether carbon (located at 286.5 eV) plays a greater role for layers with PEG and that the proportions of Table 1 are respected. High-resolution nitrogen spectra were also recorded to ensure that C-N bonds (also located at 286.5 eV) are not responsible for the different intensities.

XPS data was also used to confirm the stability of the PEG layers both after autoclaving and after acidic Boc deprotection using trifluoroacetic acid. The absence of contaminants such as salts and silicone was also verified. As observed in Table 1, no samples present Fluor atoms except for the bare FEP tape. This indicates that the plasma layer is thicker than the depth of analysis of the instrument estimated at ca. 10 nm and no "holes" were detected (by AFM imaging) in the substrates.

PEG layer resistance to protein adsorption

The main reason to produce substrates covered by thin PEG layers is to lower or eliminate non-specific cell-material interactions. A rough estimation of this property is to monitor dynamic protein adsorption on the polymer layers, as this adsorption will dictate the interaction level of cells with the material. The quartz crystal microbalance apparatus (QCM) was used to measure the adsorption of the large spectra of proteins found in fetal bovine serum (FBS) on the different PEG samples. This can be accomplished with the QCM through the analysis of the frequency and half-band-half-width (HBHW) signals, as the QCM apparatus directly measures minute changes in the distribution of oscillating frequencies of a gold-coated quartz crystal occurring as the load present on the crystal changes. Among the advantages of the technique is the possibility to work in different aqueous media and the extreme sensitivity of the technique. The results for protein adsorption are shown in Table 2.

The lowering of the resonance frequency of the vibrating quartz crystal in QCM experiments is physically correlated with the mass of material vibrating with the crystal. For systems where no energy loss is associated with the vibration, the variation in frequency is proportional to the mass on the crystal through the well-known Sauerbrey equation.

Table 2: QCM results presenting protein adsorption on different PEG-coated quartz crystals at steady state.

| Surface | Average frequency shift (Hz) | Sayerbrey protein mass (ng/mm$^2$) | Average half-band-width shift | Time to steady state (min) |
|---|---|---|---|---|
| Gold | -270 $\pm$ 6 | 0.95 $\pm$ 0.02 | 75 $\pm$ 5 | 2 $\pm$ 1 |
| Heptylamine plasma | -291 $\pm$ 8 | 1.03 $\pm$ 0.03 | 80 $\pm$ 10 | 18 $\pm$ 1 |
| PEG 0.1 mg/ml CP | -560 $\pm$ 30 | 2.0 $\pm$ 0.1 | 200 $\pm$ 30 | 30 $\pm$ 1 |
| PEG 0.5 mg/ml CP | -215 $\pm$ 6 | 0.8 $\pm$ 0.2 | 75 $\pm$ 6 | 10 $\pm$ 1 |
| PEG 1 mg/ml CP | -11 $\pm$ 3 | 0.1 $\pm$ 0.1 | 0 | 13 $\pm$ 1 |
| PEG 1 mg/ml CP (de-protected) | -48 $\pm$5 | 0.2 $\pm$ 0.2 | 52 $\pm$ 8 | 20 $\pm$ 1 |
| PEG 5 mg/ml CP | 0 | 0 | 0 | 5 $\pm$ 1 |
| PEG 5 mg/ml | -150 $\pm$ 10 | 0.53 $\pm$ 0.04 | 25 $\pm$ 4 | 3 $\pm$ 1 |
| PEG 0.1 mg/ml | -290 $\pm$ 10 | 1.00 $\pm$ 0.04 | 88 $\pm$ 6 | 3 $\pm$ 1 |
| CP denotes samples fabricated from a PEG solution in a cloud point state. | | | | |

From Table 2, while gold and plasma layers adsorb equivalent quantities of protein from FBS, large differences are observed for PEG-treated samples. PEG surfaces obtained from non-cloud point conditions were all shown to adsorb significant amount of proteins; the best and worst surfaces are listed in Table 2. While no significant variations in chemistry was apparent by XPS measurements, higher PEG concentrations are shown to achieve superior protein repellency properties. In this case, a solution of 5 mg/ml PEG lowers approximately by half the amount of adsorbed proteins by bare gold, plasma or 0.1 mg/ml PEG surfaces assuming similar energy losses in all systems.

Once again, the effect of using a theta solvent for immobilization is seen as PEG samples made using 1 mg/ml to 5 mg/ml of PEG in theta solvent conditions almost completely repel protein adsorption. Samples produced using 5 mg/ml PEG solutions under cloud point conditions lower protein adsorption below the threshold of the sensitivity of the technique. For the PEG surfaces under study (i.e. hydrated viscoelastic layers), the sensitivity of the QCM apparatus is in the 2 Hz range, as determined by the standard deviations obtained from different steady state measurements.

One final aspect to be noted is that Boc de-protection increases slightly the amount of adsorbed protein at steady state. Without being bound to any particular theory, it appears that de-protected sample presents a positively charged terminal amine group, which can create electrostatic forces with negatively charged protein domains. However, this effect is marginal for most surfaces (the worst case being presented in Table 2) and is proportional to the amount (by XPS) of PEG present on the surface (i.e. sample 1 mg/ml presents the larger number of PEG molecules and thus would present the larger electrostatic effect).

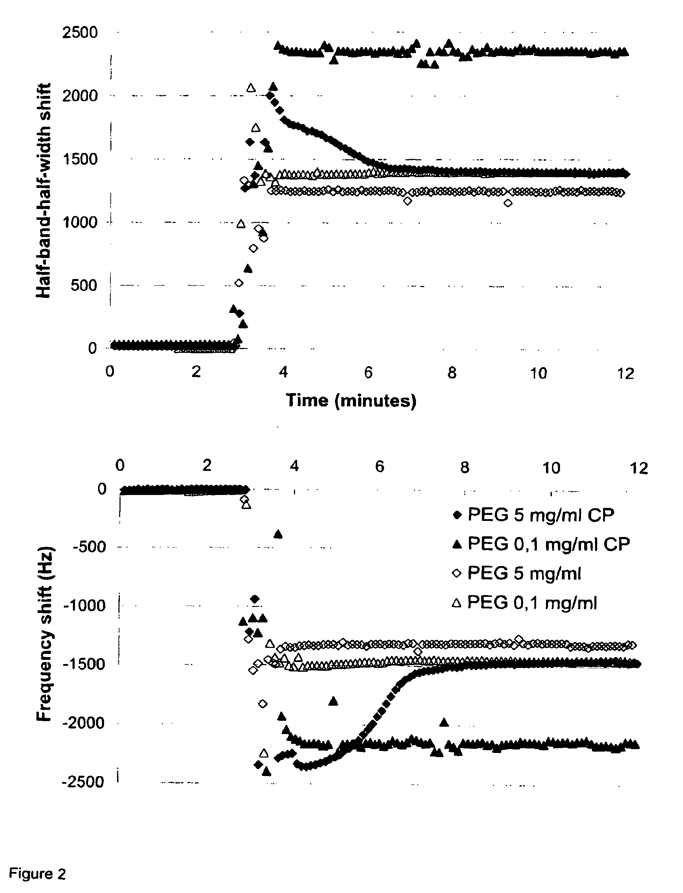

To get a better insight on the water structuring properties of the different samples, QCM hydration measurements were made where water was rapidly injected on dried PEG layers and frequency and half-band-half-width signals were recorded. These results are presented in Figure 2.

Figure 2 presents the hydration data of four representative PEG surfaces with water injection at 3 minutes. The samples that were not constructed using cloud point conditions present similar dynamic behaviors, rapidly (i.e. less than one minute) reaching a steady-state and show comparable frequency and half-band-half-width shifts. This indicates similar water uptake and water structuring properties. However, surfaces constructed with the use of a theta solvent present noteworthy differences between samples. First, surfaces covered with 0.1 mg/ml PEG (under cloud point) were shown to entrap more water molecules than all other samples illustrated by a 33% decreased frequency shift and a 70% increased half-band-half-width (compared to the other three PEG surfaces tested). Moreover, this water is rapidly arranged around the PEG molecules as the steady state of the system is also reached in less than one minute. Second, the sample constructed with 5 mg/ml PEG (under cloud point) reach similar frequency and half-band-half-width shifts than non-cloud point samples, but shows a different dynamic behavior.

In control theory, physical systems dynamic responses to stimuli can be expressed either as first, second or higher order differential equations, depending on the physical phenomena. From this observation, variables can be used to completely define dynamic responses; for second order systems, three variables are sufficient. They are the steady state gain (i.e. Kp in the standard nomenclature of second order systems), which basically gives the steady state variation between unperturbed and perturbed states, the natural period of oscillation (i.e. $\tau$) and the damping factor (i.e. $\zeta$) which together express the dynamic "shape" of the response. In Figure 2, PEG surfaces made with 5 mg/ml (under cloud point) present a second-order dynamic behavior relatively to the frequency shift as illustrated by the "S" shape of the system frequency response to hydration (i.e. there is an initial lag in the frequency response). A similar dynamic behavior was observed for samples made with 1 mg/ml (under cloud point) (data not shown). While the steady state gains (i.e. the Kp) of the 1 mg/ml and 5 mg/ml PEG surfaces (under cloud point) are comparable at values of 750 approximately, the natural period of oscillation (i.e. $\tau$) is different for both systems (if both systems are considered critically damped, $\zeta = 1$, which is reasonable considering the shape of the frequency response) at approximately 1 minute for the 5 mg/ml system and at approximately 4 minutes for the 1 mg/ml system. Interestingly, these time parameters correspond to the times to steady state for protein adsorption of Table 2, indicating a very probable relation between water structuring effects and protein adsorption.

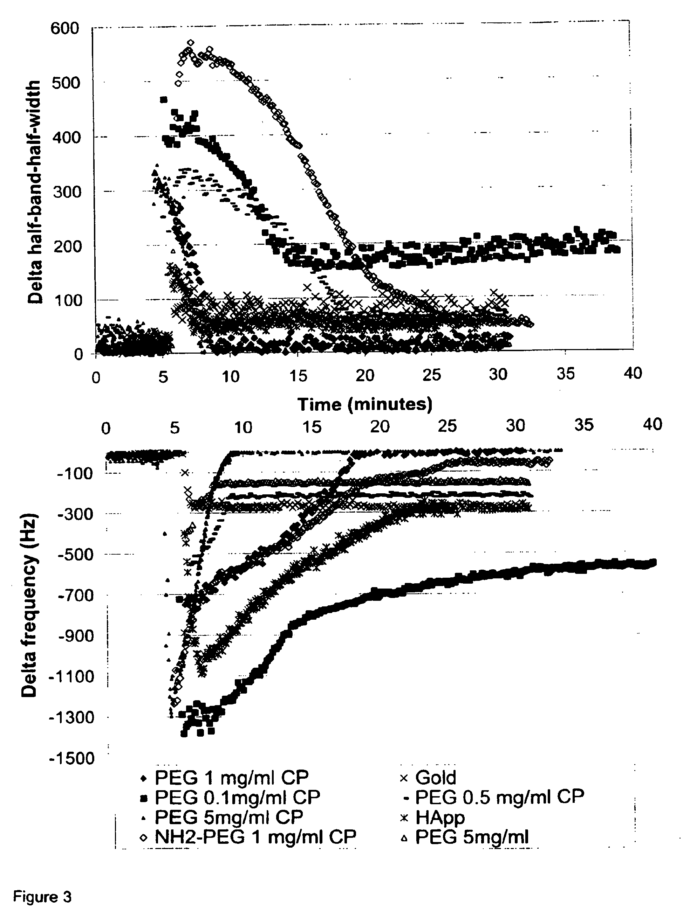

The dynamic response to protein adsorption of the different samples presented in Table 2 was determined. Figure 3 presents the dynamic recordings of the protein adsorption measurements.

The dynamic responses presented in Figure 3 are in good agreement with the hydration data of Figure 2. The response of PEG surfaces produced under non-cloud point conditions (represented in Figure 3 by the 5 mg/ml sample) is very rapid as the steady state is rapidly reached. The bare gold sample presents a similar dynamic behavior. However, all dynamic responses of the cloud point-made PEG layers present a second order-like dynamic response. Moreover, the steady state gains relatively to frequency for all these surfaces is comparable at a value of approximately 1100, and the time constants (the natural periods of oscillation) follow a similar pattern than for the hydration results of Figure 2 as the 1 mg/ml PEG surfaces (produced under cloud point) present a value of approximately 4 minutes while the 5 mg/ml PEG surfaces (produced under cloud point) present a value of 1 minute. The 0.1 mg/ml PEG surfaces (produced under cloud point) present a 10 minutes time constant, but their gain is lower due to the large amount of protein molecules adsorbed on the layer. Once again, this is coherent with the large quantity of protein molecules that was found to be vibrating with the surface in Figure 2. Without being bound to any particular theory, it appears that while PEG layers made with non-cloud point solvents repel, to some extent, protein adsorption mainly by steric forces, PEG layers made in theta solvent achieve their anti-biofouling potential through a combination of steric forces and hydration/water structuring forces. Architecture of the PEG layers thus plays a role in the resistance to protein adsorption.

Two PEG surfaces in Figure 3 present charges and create electrostatic interactions with the charged protein molecules. The heptylamine plasma layer is probably positively charged to some extent in the buffer media and this can explain the second-order dynamic behavior to protein adsorption as negatively charged proteins can rapidly adsorb on the surface followed by the adsorption of other proteins with higher affinity to the surface (through the well-known Vroman effect) until a steady state is reached. The de-protected 1 mg/ml cloud point sample presents a similar behavior as positive charges on the amine PEG end-group may also be present. In this case, the electrostatic effect is probably responsible for the increased lag of the dynamic response (i.e. time constant of approximately 6 minutes compared to 4 minutes for protected PEG) and for the large half-band-half-width variations compared to the protected sample.

Polymer layer mechanical properties by QCM and AFM measurements

The mechanical properties of the immobilized PEG molecules were characterized to get a better insight on the physical properties affecting protein adsorption and, ultimately, interactions with living cells. The QCM apparatus has been used to determine elastic compliance modulus of thin layers in viscoelastic systems (i.e. exhibiting energy losses linked to vibration). The elastic modulus can be isolated from QCM PEG grafting data (i.e. PEGylation), as the final frequency and half-band-half-width shifts measured at different harmonics were used in mathematical models previously validated with experimental data. The model used to calculate the elastic compliance of the different samples was derived by Du and Johannsmann and, for thin films in water, can be expressed as:

$$\frac{\delta\Gamma}{-\delta f} \approx 2\pi n f_0 \eta J'_f$$

where $\Gamma$ is the half-band-half-width, f the frequency (in Hz), n is the overtone order, $f_0$ is the reference frequency (5 MHz in our system), $\eta$ is the viscosity of water (in Pa•s), and $J'_f$ is the elastic compliance (in Pa-1). By plotting the negative of the half-band-half-width shift divided by the frequency shift in function of the overtone order, one may determine from the slope of the graph the elastic compliance of the thin film. The PEGylation results are reported in Table 3.

The reported frequencies and half-band-half-widths are those obtained following repeated rinsing in water. It is to be noted that the surface density reported in Table 3 is simply calculated by converting the mass determined by the Sauerbrey equation using the molecular weight of the PEG polymer.

Based on the results of Table 3, an order of magnitude can be observed, which is coherent with the XPS data of Table 1. Again, PEG surfaces fabricated under cloud point show a larger number of PEG molecules on their surface compared to non-cloud point samples. Maximal PEG density is achieved at cloud point sample 1 mg/ml. The elastic compliance of the thin films indicates a rather rigid behavior except for, interestingly, the 0.1 mg/ml PEG CP sample, which elastic compliance is 9 times that of the other samples. It should be recalled that the elastic compliance is the inverse of the real part of the shear modulus, so that a high elastic compliance indicates a "softer" material. The architecture of this sample is definitively very different to that of the other samples.

Table 3: QCM results (delta f and delta $\Gamma$) of PEG layers immobilized on HApp-activated quartz crystals for different experimental conditions. Calculated Sauerbrey mass, surface density from Sauerbrey mass and elastic compliance modulus are also presented.

| Surface | Average frequency shift (Hz) | Sauerbrey PEF mass (ng/mm$^2$) | PEG surface density (chains/nm$^2$) | Average half-band-width shift | Elastic compliance (GPa$^{-1}$) |
|---|---|---|---|---|---|
| PEG 0.1 mg/ml CP | -270 $\pm$ 20 | 1.0 $\pm$ 0.2 | 0.2 | 940 $\pm$ 60 | 19 $\pm$ 3 |
| PEG 1 mg/ml CP | -1400 $\pm$ 60 | 4.9 $\pm$ 0.3 | 0.9 | 480 $\pm$ 30 | 1.9 $\pm$ 0.3 |
| PEG 5 mg/ml CP | -1150 $\pm$ 80 | 4.0 $\pm$ 0.2 | 0.7 | 500 $\pm$ 40 | 2.1 $\pm$ 0.3 |
| PEG 0.1 mg/ml | -250 $\pm$ 40 | 0.9 $\pm$ 0.3 | 0.15 | 200 $\pm$ 40 | 2.4 $\pm$ 0.4 |
| PEG 1 mg/ml | -310 $\pm$ 20 | 1.1$\pm$ 0.2 | 0.2 | 230 $\pm$ 30 | 2.2 $\pm$ 0.3 |
| PEG 5 mg/ml | -340 $\pm$ 30 | 1.2 $\pm$ 0.2 | 0.2 | 230 $\pm$ 20 | 2.1 $\pm$ 0.2 |

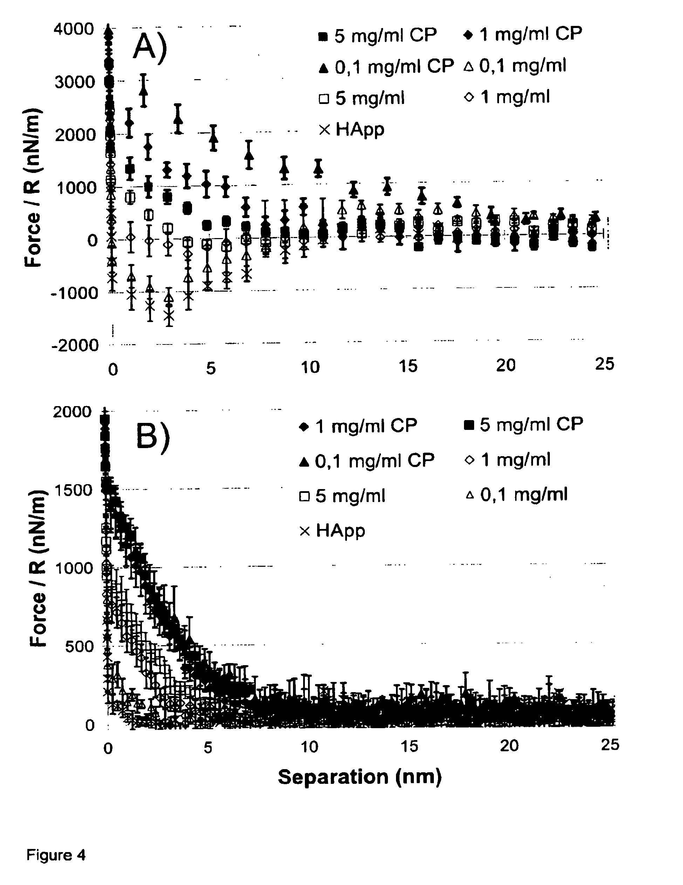

The AFM can also be used to get information on the mechanical properties of the polymer layers, specifically by using a colloidal probe force measurement method. In this technique, a relatively large sphere (i.e. diameter of 4.1 microns) glued to a cantilever is approached to the surface and is used to fully compress the polymer layer before retracting. The cantilever deflection is recorded and translated into force values relatively to the diameter of the sphere. The results of such measurements in water and in a 300-mOsm RPMI 1640 buffer are reported in Figure 4 for different PEG samples.

The AFM compression force curves of Figure 4 show that the hydrated PEG layers have a "thickness" of 4 to 8 nm in RPMI buffer, the thickest films exhibited by the cloud point-fabricated samples while the 0.1 mg/ml non-cloud point was the thinnest. By thickness, here, we mean the separation distance at which the force between the colloidal probe and the PEG surfaces became non-nil when approaching the cantilever towards the surfaces. All PEG samples produced under cloud point have similar compression profiles while the non-cloud point samples have decreased compression resistance and thickness from highest to lowest concentrations. The AFM compression data in water in the same figure shows the electrostatic effects of the negatively charged AFM silica sphere and the positively charged plasma layer for the samples with small PEG densities i.e. for non-cloud point PEG samples 1 mg/ml and 0.1 mg/ml, as a "jump to contact" was recorded upon approach of the surface. This probably translates into a poor coverage of the entire surface for these two conditions, which is coherent with XPS data of Table 1 and protein adsorption experiments. 5 mg/ml PEG surfaces produced under cloud point show similar behavior to compression in water and in buffer, but the 1 mg/ml and, principally, the 0.1 mg/ml PEG surfaces produced under cloud point both present a significant increase of their thickness, while their resistance to compression (i.e. the slope of the compression curve) is slightly reduced. Without being bound to any particular theory, the use of a better solvent and the high degree of freedom of their architecture probably enable the extension of the polymer chains in the solvent, a result that was not expected and not previously reported.

Agglomeration kinetics of PEG molecules in theta solvent

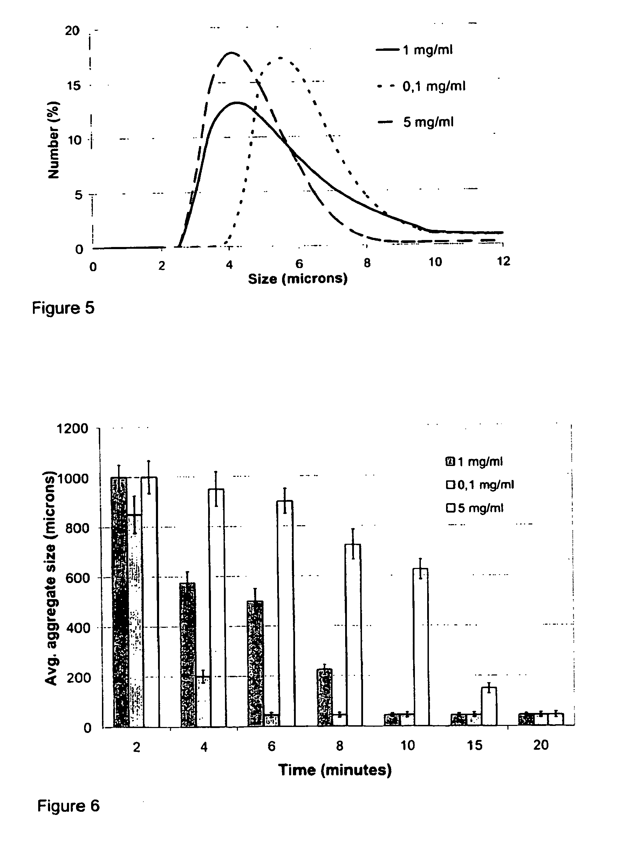

The very wide range of characteristics (i.e. number of bound PEG molecules, resistance to protein adsorption and mechanical properties) exhibited by the PEG surfaces produced under cloud point at different PEG concentrations is not coherent with the traditional Alexander and de Gennes models, contrarily to the PEG surfaces fabricated in water. In a theta solvent, PEG molecules form large aggregates of different size distributions depending on a variety of factors such as concentration, shear stress and temperature. Using a light diffraction-based instrument, the Malvem Mastersizer, the size of the polymer aggregates was measured over time to assess possible effects of PEG agglomerate size in solution on final PEG layer chemistry and properties. For the light diffraction measurements, PEG was dissolved for two minutes by hand agitation and added directly to the theta solvent circulating in the apparatus to mimic as closely as possible the actual procedure for PEG immobilization. The agitation in the apparatus was also set to be as close as possible to the agitation present in typical immobilization reactions. The results in number % are presented in Figure 5 and the size of the agglomerates in volume % over time is presented in Figure 6.

Figure 5 indicates that, in terms of number %, the PEG aggregate size is constant throughout the surface immobilization reaction. Lowering the PEG concentration tends to yield larger polymer aggregates in solution, with a 50% difference between the 0.1 mg/ml and 5 mg/ml cases. PEG aggregate sizes in terms of volume % were shown to vary with time for all PEG concentrations. Indeed, Figure 6 clearly shows that very large PEG aggregates are present in the system and that the kinetics of large aggregates formation are principally controlled by a homogenization/dissolution process. When placed in the theta solvent, solid PEG initially forms very large aggregates (which can be at some times multi-modal in their size distribution) which are then homogenized by the shear stress provided by the impeller in the apparatus and the gradual dissolution of the PEG molecules.

Chemical bonding Properties of the de-protected PEG layers

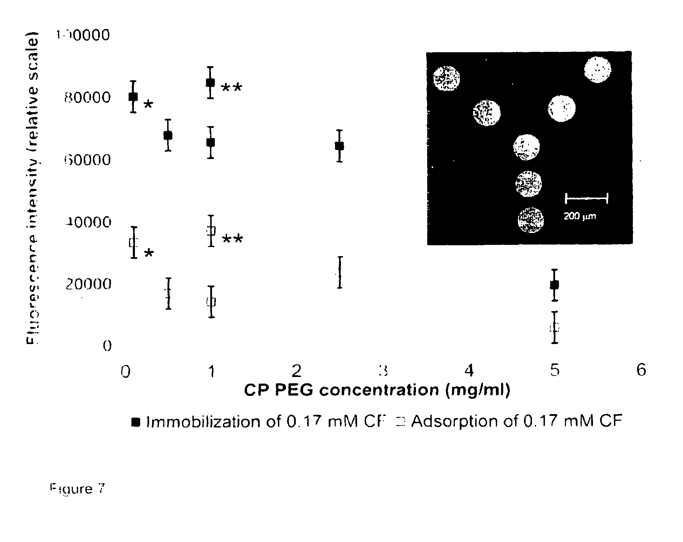

The immobilized PEG layer system studied herein allows the covalent immobilization of molecules after Boc de-protection of the PEG molecules exposing primary amino groups. Moreover, the molecules can be immobilized in a spatially defined manner by using a dispensing robot. A fluorescent molecule, carboxyfluorescein (CF) was deposited by a dispensing robot on the different samples and immobilized by a covalent amide bound. The fluorescence intensity, after rinsing, is indicative of the quantity of CF molecule that successfully bound to the PEG-end amines if the concentrations of CF used are located in the linear non-quenching regime. This was ensured by a calibration curve done on different samples (data not shown). The results of the immobilization of CF are shown in Figure 7.

Figure 7 shows that all samples covalently bind CF molecules as the signals of the covalently immobilized CF were always stronger than those of the adsorbed CF for a given PEG sample. The samples made from 2.5 mg/ml PEG under cloud point and lower PEG concentrations all show similar chemical availability of the amine end-groups for CF immobilization, except for the 0.1 mg/ml sample, which exhibits non-homogenous fluorescence that could be due to CF molecules trapped in the PEG layer architecture. As observed from QCM data, the 0.1 mg/ml PEG sample was also the sample which was the most prone to protein adsorption. The behavior of the samples fabricated under non-cloud point conditions is for all concentrations similar to that of the 0.1 mg/ml cloud point sample. Non-homogenous fluorescence is also observed with an increase in CF adsorption. Thus, all the layers could be used to graft covalently and spatially molecules with varying yields, which depend on the underlying PEG surfaces.

A system to immobilize PEG molecules on various solid substrates has been described herein, and the use of two reaction parameters i.e. (1) the use of a theta solvent and (2) different polymer concentrations in solution, were studied to assess their impact on PEG layer properties. Results show that PEG layers deposited using cloud point conditions present unusual reaction PEG concentration-dependent architectures as observed, noticeably, by protein adsorption assays and AFM force measurements. While high polymer concentrations yield structurally stiff layers, low PEG concentrations exhibit higher structural flexibility which enhances chemical bonding capabilities, but, on the other hand, can enhance non-specific protein adsorption.

**EXAMPLE 2**

**Selective Detection of Activated Platelets from Whole Blood for Cardiovascular Medical Diagnostic Using PEG-Based Supports**

In hemostasis, platelets rapidly adhere at sites of vessel wall injuries, aggregate and form a hemostatic plug that is strengthened by a fibrin network. At the onset of this phenomenon, platelets adhere on von Willebrand factor (VWF), collagen and fibronectin located in exposed extracellular matrix after vessel injury. The exposition of platelets to these molecules induces important changes in their morphology and physiology, which are needed to stimulate platelet aggregation and fibrin formation. This process is termed platelet activation. In contrast to non-activated platelets, activated ones, for example, express the trans-membrane protein P-selectin, present an alternate conformation of the GPIIb/IIIa integrin and even extend pseudopods to enhance aggregation.

The clinical significance of platelet activation is very important. An increased platelet activation level in circulating blood has been demonstrated to be an important stroke risk factor and is thought to precede strokes. Moreover, the efficiency of oral antiplatelet activation drugs (e.g., aspirin, clopidogrel, ticlopidine) can be monitored through the measurement of platelet activation levels. Finally, the quality of some blood products can be determined through the measurement of platelet activation.

Unfortunately, no definitive measurement technique currently exists to measure effectively, accurately and repeatedly platelet activation in the clinic. Noticeably, the techniques currently used all require important precautions for blood manipulations to avoid ex *vivo* activation and depend largely on the skills of technicians. Aggregation assays (including clotting time assays) all depend on patient platelet count and blood lipid content (when whole blood is used) and are considered too imprecise to monitor slight changes in platelet activity. The current gold standard for the measurement of platelet activation is flow cytometry, as it is more precise than other techniques and is not dependent on platelet counts. However, it is not readily available for routine testing due to its high cost and the need to employ a dedicated and skilled operator.

One of the key aspects when working with platelets is their elevated sensitivity to shear stresses and foreign materials. Very high levels of shear can damage and activate platelets. As such, detection techniques must minimize shear stress peaks imposed on whole blood. For example, needles of gauge lower than 20 should be used to minimize activation. Moreover, platelet adhesion on surfaces has been demonstrated to be strongly influenced by the shear rate at the surface, as well as protein deposition rate. For example, *in vivo* platelets are thought to first temporarily attach to von Willebrand factor at high surface shear rates through glycoprotein Ibα which in turns allows the strong and permanent binding of the platelets through the integrin $\alpha_{IIb}\beta_3$. As such, controlling effectively the shear rate imposed on platelets at every stage of their manipulation is an issue to consider. Parallel plate systems that allow the estimation of shear rates have previously been used to monitor platelet adhesion.

When exposed to foreign materials such as glass or plastics, platelet activation and adhesion rapidly occurs (i.e. 2-3 minutes under static conditions). Low-fouling materials such as immobilized poly(ethylene glycol) (PEG) surfaces have been shown to delay platelet activation. This property has been largely attributed to the capacity of these materials to lower protein adsorption. The nature of the low-fouling material and the immobilization technique used play a role in this effect.

The flow chamber instrument developed by Hubbell and McIntire was modified to allow the visualization of platelet adhesion *in vitro* while controlling shear stresses imposed on platelets throughout the circulation by using computer-assisted mathematical modeling. Platelet activation in whole blood was detected by using thin PEG layers that lower protein adsorption and platelet adhesion while presenting local bio-signaling to selectively attach activated platelets. Blood was circulated in the flow chamber over these surfaces in an attempt to create an effective, rapid and low-cost instrument to evaluate platelet activation in whole blood.

Experimental method

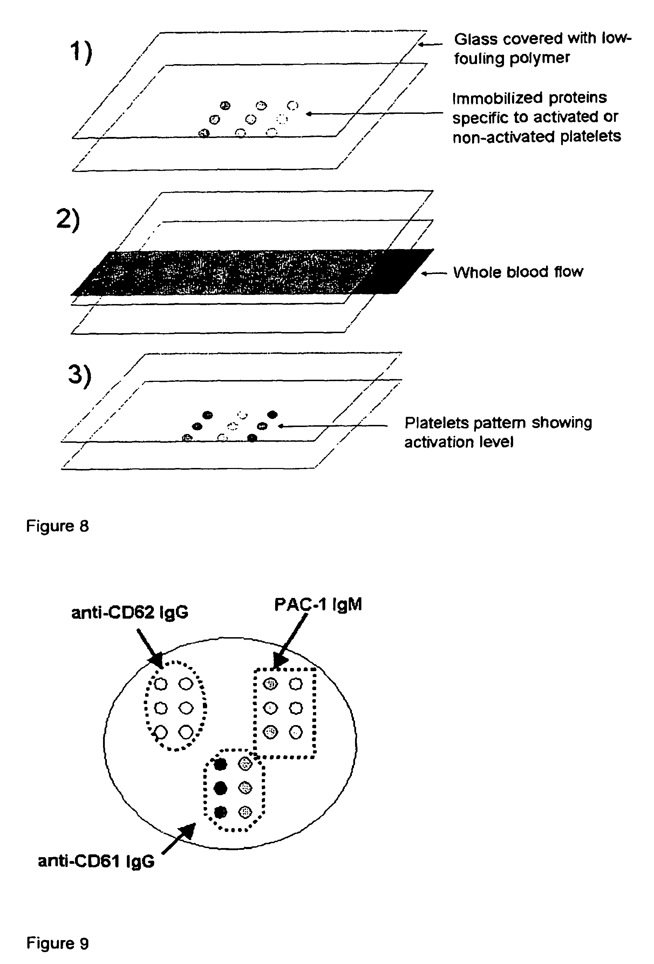

A schematic of the technique developed to measure platelet activation levels is shown in Figure 8. The main components of the instrument are: 1) low-fouling polymer-coated glass slides to limit platelet activation and adhesion, 2) covalently immobilized protein arrays, and 3) a flow chamber in which the shear environment is well defined. Aside from this property, the flow chamber is also used to limit the volume of blood necessary for the testing, to avoid blood exposure to air, and to hold the glass slides while allowing observation by optical microscopy.

Reactive poly(ethylene glycol) (PEG)-based low-fouling surfaces were fabricated. Briefly, n-heptylamine plasma polymerization was used to generate a primary amine reactive layer on glass slides (50mm x 24mm borosilicate, Carolina (NC), USA) or perfluorinated poly(ethylene-co-propylene) tape from Dupont (Teflon FEP Type A, Mississauga, Canada). Boc-NH2-poly(ethylene glycol)-NHS of 3,400-Da molecular weight from Nektar Therapeutics (cat. 4M530F02, San Carlos (CA), USA) was then made to react with the amine groups through its NHS end-groups to create a stable amide bond.

The layer final properties can be modulated by varying reaction conditions such as polymer concentration or the use of a theta solvent. PEG layers were fabricated using a PEG concentration of 1 mg/ml in a theta solvent at room temperature. The theta solvent used was a 1.4M $Na_2SO_4$ solution. Modified surfaces can be stored in buffered saline at 4°C for extended periods of time without change in properties (data not shown). Immobilized Boc-NH2-PEG-plasma layers were Boc-deprotected following manufacturer's recommendations. Briefly, the substrates were immersed in a 50% v/v solution of trifluoroacetic acid (cat. T62200, Sigma-Aldrich, Ontario, Canada) and dichloromethane (cat. D65100, Sigma-Aldrich, Ontario, Canada) for one hour. The substrates were afterwards repeatedly rinsed in a 5% v/v solution of N,N-diisopropylethylamine (cat. D3887, Sigma-Aldrich, Ontario, Canada) in dichloromethane.

Anti-CD62 (anti-p-selectin) mouse IgG (cat. 30402, BioLegend, San Diego (CA), USA) and anti-CD61 mouse IgG (cat. 555752, BD Biosciences, Boston (MA), USA) were arrayed on the low-fouling surfaces using a BioChip Arrayer from PerkinElmer (Wellesley (MA), USA). The surfaces were previously activated through the use of the cross-linking agent, disuccinimidyl carbonate (DSC, cat. 225827, Sigma-Aldrich, Ontario, Canada) to incorporate active ester groups on the amine surfaces. Twenty drops (ca. 300 pl per drop) of dilute protein solutions were deposited on the activated surfaces and placed overnight in a controlled humidity chamber to allow amide bond formation between the available primary amine groups of the proteins (i.e. N-terminal and lysine residues) and the NHS-activated PEG surfaces. The droplet design used to measure platelet activation is presented in Figure 9. Following reaction, surfaces were thoroughly rinsed in 300mOsm PBS buffer at pH 7.4.

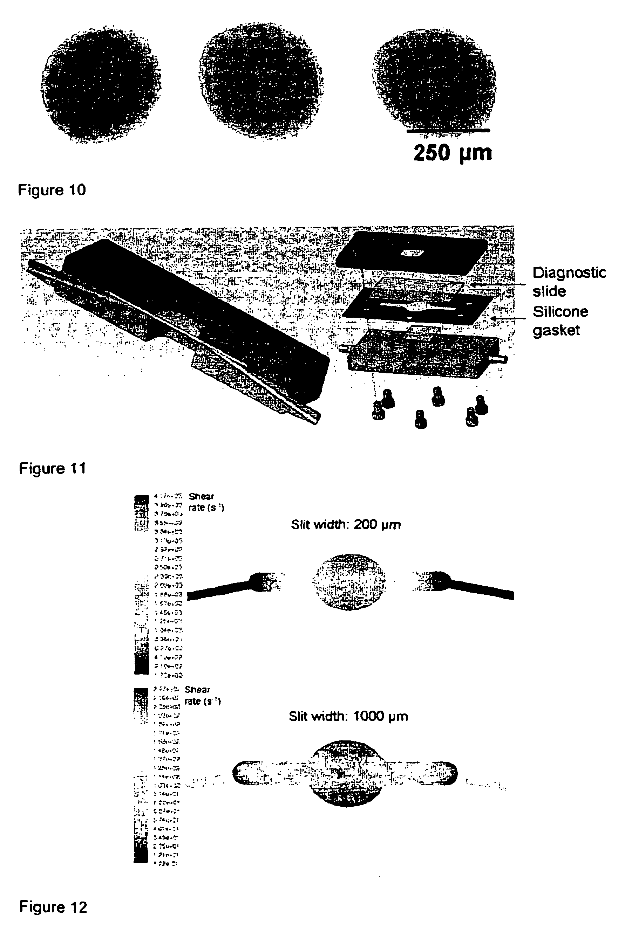

The presence and bio-availability of the immobilized antibodies was characterized by ELISA. Alkaline phosphatase-conjugated anti-mouse IgG (cat. S372B, Promega, Madison (WI), USA) was diluted 1:3000 and made to react with the surfaces for one hour under agitation. The surfaces were then rinsed with PBS buffer for one hour under agitation. Finally, BCIP/NBT (cat. B5655, Sigma-Aldrich, Ontario, Canada), used according to manufacturer instructions, yielded a purple precipitate upon reaction with the enzyme alkaline phosphatase. Thus, purple regions are indicative of the presence of mouse-derived antibodies (such as CD62 and CD61). Typical observation using a standard Leica binocular microscope connected to a camera is shown in Figure 10. Although variations in coloration intensity were observed between different antibodies and concentrations, the ELISA assay was only used for a qualitative assessment of the presence of the immobilized antibodies and, as such, these differences were not considered. Finally, it should be noted that surfaces that were incubated for up to two months at 4°C in 300mOsm buffered medium were tested through ELISA for stability and showed no significant difference compared to fresh samples and that three different batches yielded similar qualitative results with slight variations in droplet shapes.

To ensure that spatial location of the protein niches was reproducible and to allow the localization of the antibodies without staining, a mask was fabricated using commercial soluble cake icing color. Spots of different color depending on antibody and concentration were deposited on a blank glass substrate using the design of Figure 9. A second glass surface was bound using epoxy glue on top of the mask for protection. By placing the mask underneath the treated samples, it is possible to determine the precise location of the deposited transparent protein arrays using standard microscopy.

A flow chamber was fabricated to fit the 50mm x 24mm glass slides, to prevent blood exposure to air and to subject platelets to a define shear rate throughout its flow outside the circulation, noticeably at the interface between the blood flow and the diagnostic surface. The design of the chamber was adapted from the previous work of Hubbell and McIntire. The final design of the flow chamber is shown in Figure 11. The chamber was fabricated to circulate blood between parallel plates distanced by the thickness of a custom-fabricated thin silicone sheet gasket. The gaskets used were of approximately 200 microns and were fabricated between parallel Teflon plates and cut using a custom-made punch. All parts were machined in polysulfone material, which can readily be sterilized and cleaned. The entrance and exit were made to receive male Luer lock connections. The fabrication allows a leak-free circulation and the possibility to continuously monitor the platelet deposition through apertures made in the chamber. The dimensions of the chamber when assembled are 80mm in length, 38mm in width and 10mm in thickness. It should be noted that a standard 18mm square microscope cover slip is used in conjunction with the glass sample to allow continuous observation. The cover slip was bonded to the polysulfone using epoxy glue. The design and machining of the chamber was made using the SolidWorks software (Solidworks, Concord (MA), USA).

Simple momentum balances, assuming laminar flow between flat parallel plates, yield for the chamber a simple mathematical equation relating the flow rate to the surface shear rate:

$$Q = \frac{2B^2W}{3} \times \frac{\tau_{xz}}{\mu}$$

(1)

where Q is the flow rate (ml/s), B is half the slit width (cm), W is the total flow chamber width (cm), $\tau_{xz}$ is the shear stress

(dyne/cm$^2$) in the z direction (vertical) caused by the flow rate in the x direction (direction of the blood flow), and $\mu$ is the blood viscosity (poise). The blood viscosity was estimated at 4 cp and assimilated to a Newtonian fluid. The shear rate (s$^{-1}$) is given by the velocity gradient ($\delta v_x/\delta z$) and is reflected as the ratio of the shear stress to viscosity ($\tau_{xz}/\mu$). The shear rate at the flow chamber surface is commonly used in the platelet-related literature. In the present case, for a slit width of 200 $\mu$m, using the total chamber width of 0.8 cm, we obtain the relation:

$$Q = 0.00005 * \frac{\tau_{xz}}{\mu} \qquad (2)$$

Moreover, for a slit width of 1 mm, a similar relation can be calculated:

$$Q = 0.001 * \frac{\tau_{xz}}{\mu} \qquad (3)$$

Using Equations 2 and 3, it is possible to estimate the required flow rate to achieve a specific surface shear rate between the platelets and the diagnostic surface. For example, to obtain a shear rate of 100s$^{-1}$, a flow rate of 0.3 ml/min is required in the 0.2-mm width, while a flow rate of 8 ml/min is needed in the 1-mm slit. This flow rate was set in the chamber by using a positive displacement precision injection system (Model 200, KDScientific, Holliston (ME), USA) consisting in a syringe mechanically pushed by a computer-controlled motor.

To ensure the validity of Equations 2 and 3, to assess their precision and to verify that platelets are not subjected to large stresses that could artificially modify their physiological state throughout their circulation in the chamber, computational fluid dynamics (CFD) modeling was used to evaluate the flow environment in the chamber, more specifically the surface shear rate imposed on platelets as they approach the diagnostic slide, the maximal shear imposed on blood cells throughout the injection and the pressure drop necessary to circulate blood at the desired shear rate. Briefly, the chamber was discretized by an unstructured finite volume method to convert the continuity and momentum equations to algebraic equations that can be solved numerically. The flow model used here was based on the Navier-Stokes equations for segregated laminar isothermal flow in conjunction with a k-$\varepsilon$ turbulent model. The walls were treated as non-slip boundaries and blood was treated as a single phase and Newtonian fluid with a viscosity of 4 cp. For the simulation, the circulation was provided by mass flow rate and pressure outlet. The software used for the simulation was FLUENT 6.2 (Fluent, Lebanon (NH), USA).

Typical experiments were carried out by setting up the sterilized chamber in a laminar flow hood. Blood was sampled from donors following a protocol approved by an independent ethics committee. Briefly, citrate tubes (cat. 8003894, BD Vacutainer, Boston (MA), USA) and needles of gauge 20 or lower were used to collect blood samples. The samples were used within two hours to ensure platelet function. Platelets were artificially activated by adding collagen (cat. 5368, Helena Laboratories, Beaumont (TX), USA) at a 2 $\mu$g/ml concentration in whole blood five minutes prior to injection. Blood was then injected in the circulation chamber at a constant rate for one minute. The flow rate used here was adjusted to obtain a surface shear rate of 100s$^{-1}$ (e.g., 0.3 ml/min for a 0.2-mm slit). Following blood injection, the chamber was rinsed with 7.4 iso-osmolar PBS buffer for two minutes using the same flow rate as for blood. Microscope observations are carried out throughout this process using a camera-conjugated Zeiss stereomicroscope (Carl Zeiss Canada, Toronto (ON), Lumar V12) and appropriate filters. When needed, surface-bound platelets were then be fixed with 10% formaldehyde for further experiments, such as SEM observation. To enable microscope observations during blood circulation in the chamber and to eliminate the need for rinsing, quinacrine dihydrochloride (cat. Q3251, Sigma-Aldrich, Ontario, Canada) was added at a concentration of 10$\mu$M to the whole blood samples five minutes prior to injection in order to fluorescently label platelets. Quinacrine dihydrochloride is reported to label platelets and leucocytes when used in whole blood and have no effect on platelet function. Blood samples were destroyed after use.

Still images, on which fluorescently-labeled platelet aggregates yielded high luminosity areas, were treated manually to quantify the number of surface-adhered platelets. Using the mask previously described, spot locations were delimited on the images. Using Photoshop software (Adobe, San Jose (CA), USA), 50 $\mu$m squares were created to evaluate the average pixel luminosity inside their boundaries. The average pixel luminosity in the spots was compared to the luminosity obtained in twenty random locations outside the spots. As the luminosity distribution was exponentially distributed, the natural logarithm of the luminosity was taken to perform the statistical analysis. Areas of null average luminosity were assimilated to areas of 0.01 average luminosity to allow the transformation of the data. Moreover, the logarithmic transformation generates negative values for surfaces with luminosity from 0.01 to 0.99 which, although counterintuitive, allow sounder statistical comparisons as the normality criteria are better respected. Student t-tests were performed to determine if the average natural logarithm of luminosity from a spot was statistically different from that of its surroundings (indicating a positive test for this spot or group of spots).

Results

CFD modeling was carried out to verify the validity of the laminar parallel plate model to calculate the surface shear rate imposed on platelets as they approach the diagnostic slide in the flow chamber. Two different chamber dimensions were studied to get an insight on the effect of plate separation on the shear environments.

Images of the surface shear rate distributions in the 0.2-mm and 1-mm slit width chambers are presented in Figure 12 for a 6 ml/min flow rate. The shear rates in the diagnostic area agree within 25% to the estimations calculated from Equations 2 and 3. At larger slit widths, the shear rates calculated from the simpler equations are in good agreement with those obtained from computer-assisted simulation, while at smaller slit widths, the shear rates calculated from the simulation can be slightly lower in the diagnostic area. Equations 2 and 3 can then be preferred to computer-assisted simulations to routinely evaluate surface shear levels in the parallel-plate blood circulation device owing to their adequate accuracy and ease of use.

The shear rates calculated from the simulation are uniform throughout the central area of the chambers, which corresponds to the localization of the protein arrays. Moreover, shear peaks more than three times higher than shears calculated in the parallel plate area of the apparatus are present in the entrance and exit areas of the chamber, for both chamber dimensions. This can be important when working at high flow rates as large shear stresses are known to activate platelets. However, the exact shear levels at which platelets activation occurs are not well characterized. Most flow systems that use standard tubing and diagnostic slides present shear peaks at the junction between the tubing and the flat surfaces. In this system, initial simulations on a variety of chamber designs showed shear peaks considerably larger than those calculated for the presented chamber. Iterations were made to minimize shear peaks, mainly by modifying the tubing-slide junction configuration.

The chamber fabricated to circulate blood samples was able to provide a controlled surface shear rate between the platelets and the protein arrays. However, some limitations should be kept in mind. First, the peak levels in shear stresses observed at the entrance and exit of the chamber should be considered when setting a flow rate to avoid artificial platelet activation (i.e. high flow levels would result in significantly higher shear levels imposed on the platelets than those calculated in the diagnostic area). Secondly, blood hematocrit and fibrinogen content are known to alter its viscosity, altering to some extent the surface shear stress. Moreover, whole blood acts as a non-Newtonian fluid at shear rates lower than $100s^{-1}$. As such, the fluid dynamics calculations developed should be taken with care when working at low flow rates. Finally, other shear stresses imposed on blood cells during venous sampling, transportation and circulation should be minimized so that shear-related ex *vivo* platelet activation is minimized.

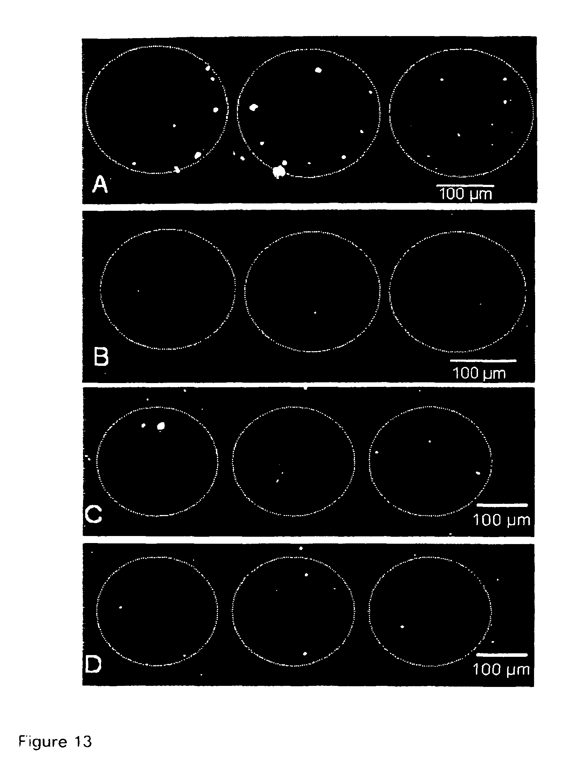

To ensure the capacity of the instrument to locally capture activated platelets from whole blood, collagen-activated platelets with fluorescent labeling were injected in the flow chamber as described above. Following rinsing, the diagnostic slides were observed under the microscope and yielded images such as those presented in Figure 13 for spots of anti-CD62 and anti-CD61 exposed to artificially activated or non-activated platelets in whole blood. While evident differences appear between the various conditions in Figure 13, the number of adhered platelet aggregates was assessed by the evaluation of the intensity of the fluorescent signal on the surfaces. As shown in Table 4, statistical analysis of the luminosity signals revealed that collagen activation yielded significant platelet immobilization on anti-CD62 and anti-CD61 spots (p-value < 0.03). Moreover, non-activated platelets did not significantly adhere on anti-CD62 spots while they showed significant anti-CD61 adhesion on two thirds of the tested samples (platelets are known to present CD61 independently of their activation levels).

Platelets were activated exclusively by exposition to a collagen concentration of 2 $\mu$g/ml (representing a positive test) and compared to platelets that were not activated artificially (representing a negative test). This example was intended to demonstrate the capacity of the instrument to detect activated platelets.

Table 4: Summary of platelet detection results and corresponding statistical analysis.

| | Activated platelets | Non-activated platelets |
| --- | --- | --- |
| Average In (luminosity) anti-CD62 spots | 0.74[1] (1.47) | -3.78[1] (1.44) |
| | 0.93[2] (0.84) | -2.90[2] (2.25) |
| | 0.19[3] (0.92) | -3.21[3] (1.58) |

(continued)

| | Activated platelets | Non-activated platelets |
|---|---|---|
| Average In (luminosity) anti-CD61 spots | 0.39[1] (1.50) | -0.64[1] (1.38) |
| | 0.06[2] (1.20) | -1.35[2] (2.40) |
| | -0.84[3] (1.90) | 0.01[3] (1.24) |
| Average In (luminosity) | -3.38[1] (1.83) | -3.78[1] (1.46) |
| area outside the spots | -1.66[2] (0.72) | -3.51[2] (1.89) |
| | -3.99[3] (1.62) | -3.91[3] (1.23) |
| Statistical test (Student t test with $\alpha$=0.05) anti-CD62 spots | Positive[1] (p-value < 0.01) | Negative[1] |
| | Positive[2] (p-value < 0.01) | Negative[2] |
| | Positive[3] (p-value < 0.01) | Negative[3] |
| Statistical test (Student t test with $\alpha$=0.05) anti-CD61 spots | Positive[1] (p-value < 0.01) | Positive[1] (p-value 0.05) |
| | Positive[2] (p-value 0.02) | Negative[2] (p-value 0.08) |
| | Positive[3] (p-value 0.03) | Positive[3] (p-value 0.01) |
| Superscripts refer to the blood sample used. Values in parentheses correspond to the standard deviation (n=6). The natural logarithm of the luminosity is used to respect the normality criteria necessary for statistical testing. This generates negative values for samples with luminosity from 0.01 to 0.99. | | |

The instrument presented enables an evaluation of platelet activation from whole blood.

## EXAMPLE 3

**Selective Immobilization of Non-adherent FDC-P1 Hematopoietic Cells on Hematopoietic Stem Cell Niche Bearing Stem Cell Factor Using PEG-Based Supports.**

Supports of the invention were used to mimic aspects of the hematopoietic stem cell (HSC) niche. HSC and hematopoietic cells (HC) such as FDC-P1 are non-adherent cells that can adhere to specific molecular signals. This phenomenon is important in hematopoiesis, the formation of blood, and is of significance in various diseases and proposed treatments.

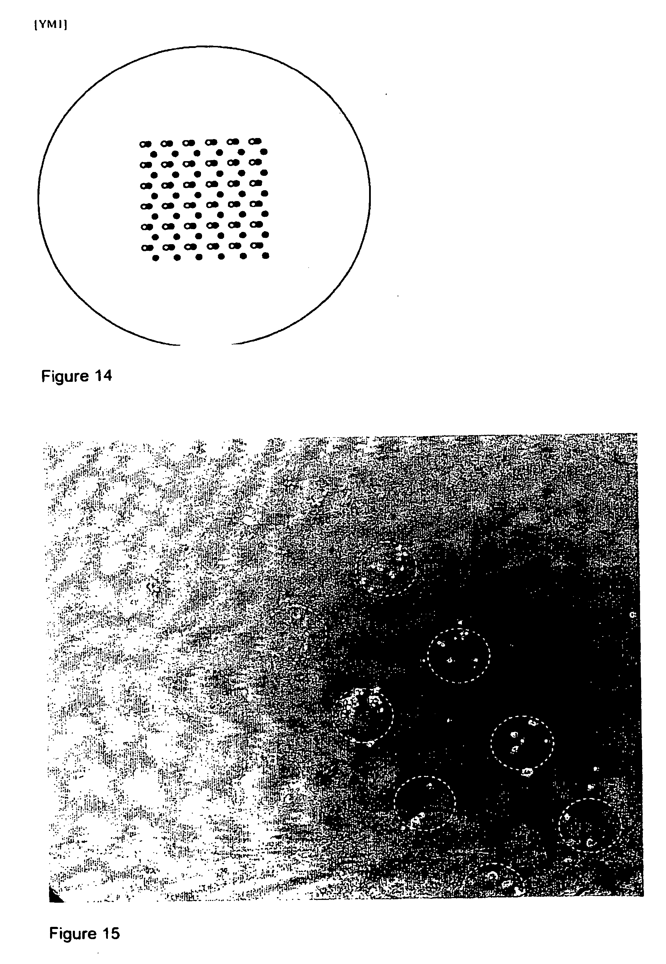

Surfaces, shown in Figure 14, were made from PEG low cell binding layers made at different conditions (i.e. cloud point, concentrations). The reaction to immobilize the stem cell factor (SCF) is similar to the one described in the platelet section, as well as the ELISA test used to verify its effectiveness. In Figure 14, pale dots represent immobilized stem cell factor and dark dots represent fibronectin. The deposited protein concentration is of 50 ug/ml. The distance between fibronectin and SCF spots was varied, and in some cases fibronectin was not deposited.

Results of a 48-hour exposition of FDC-P1 cells to SCF arrays are shown in Figure 15. As seen, islets of cells were formed on the surfaces, corresponding to the deposited SCF spots. Again, the statistically lesser number of cells (p-value of 0.05) in the low cell binding area than on the bioactive spots is used to confirm the effectiveness of the surface. A much lower number of cells can be found on the low cell binding area. The results also shown that the use different PEG under layers yields different cell attachment behavior.

## EXAMPLE 4

**Selective Immobilization of Myoblasts (Muscular Adult Stem Cells) Using PEG-Based Supports**

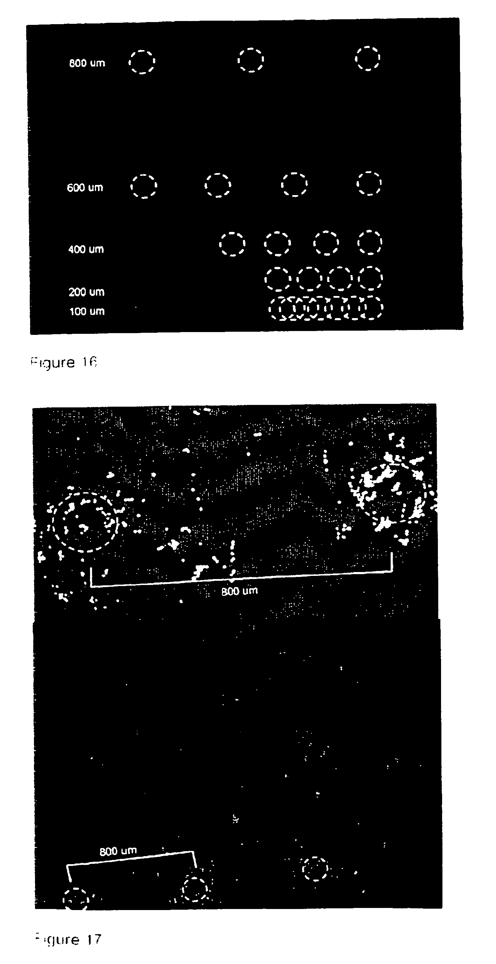

Adult muscular stem cells were cultivated for three days on 1 mg/ml PEG surfaces onto which the fibronectin design presented in Figure 16 was covalently immobilized.

This was accomplished to show the feasibility of using the support of the invention to control adult stem cell fate locally in *vitro.* The deposition technique, immobilization chemistry, preparation, identification of the spots, and ELISA testing is similar to those described above.

Two result samples are shown in Figure 17, showing a high signal-to-noise ratio. Again, a statistical difference (p-value 0.05) between the cell numbers in low-cell binding areas versus the cell number in spots (per surface area) is used to assess the chip performance.

**EXAMPLE 5**

**Development of dextran-derivative arrays to identify physico-chemical properties involved in biofouling from serum.**

The aim of example is to investigate the effect of immobilization conditions on the physicochemical properties of carboxymethyl dextran (CMD) graft layers and on the biofouling level. The relationship between CMD graft layer structures and the level of biofouling from serum was investigated.

The polysaccharide studied was an anionic derivative of dextran i.e. CMD, with various carboxyl density on the chain. CMD is a highly flexible polysaccharide, due to its $\alpha$(1-6) linkages, present in an extended coil conformation in pure water due to intra-molecular charge repulsion and present in a random coil form in electrolyte solutions owing to the salt screening effect. High-molecular weight dextrans present very few long branches, whose content increases with molecular weight. Hence, CMD conformation depends on its molecular weight, its charge density and on solution salt concentration.

CMDs were immobilized on plasma-modified surfaces bearing amine groups, via their carboxylic groups, using carbodiimide chemistry. To simultaneously study parameters affecting CMD graft layer properties, polymer arrays were developed. These polymer arrays can also be referred to as polymer chips and are based on DNA and protein micro-array technology. Polymer arrays were used to allow the high-throughput study of a large numbers of CMD coatings on a same solid substrate offering diversity in term of CMD layer physico-chemistry and limiting batch-to-batch variations. This method was used to relate immobilization conditions with the resulting CMD layer physico-chemical properties and with the level of protein adsorption onto these different polymer coatings. X-ray photoelectron spectroscopy (XPS) was used to screen the effect of CMD immobilization conditions on the surface chemical composition directly on the CMD arrays. These XPS results were compared to those of surfaces fully covered (i.e. by using no arrays) by CMD graft layers made using the same immobilization conditions. Atomic force microscopy (AFM) colloidal probe force measurements were used to study the impact of the immobilization conditions on the force profiles obtained between a silica sphere and the CMD graft layers. Finally, surface plasmon resonance (SPR) microscopy was used to investigate the homogeneity of the CMD spots across the arrays and also to investigate the relative protein adsorption onto these different CMD spots directly on the arrays.

Experimental section

Materials

Borosilicate glass substrates (Assistent, Sondheim, Germany) were washed in Sparkleen solution (Fisher Scientific Canada, Ottawa, ON, Canada), thoroughly rinsed with Milli-Q water (Millipore Canada, Nepean, ON, Canada) with a resistivity of not less than 18.2 M$\Omega$.cm and dried with 0.2-$\mu$m filtered air. For SPR analysis, LaSFN9 substrates were purchased from ResTec (Framersheim, Germany). Before metal vaporization, the SPR substrates were washed in Sparkleen solution and rinsed in Milli-Q water, in HPLC-grade ethanol, and finally in Milli-Q water before being air-dried with 0.2-$\mu$m filtered air. Vaporization of a 5-nm chromium layer and of a 48-nm gold layer was done by electron beam evaporation in vacuum. Before utilization, substrates were cleaned under UV, immersed in HPLC-grade ethanol, rinsed in Milli-Q water and dried with 0.2-$\mu$m filtered air. Metallized samples could be reused for few experiments, then, an additional cleaning step was done in piranha solution (3:1 ratio $H_2SO_4$ / $H_2O_2$) to remove any organic materials.

Carboxymethyl dextrans (CMDs) with different ratios of carboxyl groups to sugar residues were prepared as follows. Five (5) g of dextran (70 and 500 kDa MW, cat. #17-0280 and #17-0320, Amersham Biosciences, Uppsala Sweden) were dissolved in 25 ml of a 1M NaOH solution, then bromoacetic acid (cat. #3016, Lancaster Synthesis Inc., Pelham, NH, USA) was added to yield a final concentration of either 0.125, 0.5 or 1 M. The solution was stirred overnight and dialyzed against Milli-Q water, 1 M HCl, and finally Milli-Q water for 24 hours each. Solutions were then lyophilized and stored at -20°C. The carboxylation degree was determined by $^1$H NMR: carboxylation degrees obtained were 5%, 25%, and 50% for 70-kDa dextran treated with 0.125M, 0.5M and 1 M bromoacetic acid, respectively, and 5% and 40% of carboxylated residues for 500-kDa dextran treated with 0.125M and 1 M bromoacetic acid, respectively.

Phosphate buffered saline at pH 7.4 (PBS) used in these experiments was prepared in Milli-Q water using NaCl (0.137M), KCl (0.003M), $Na_2HPO_4$ (0.008M), and $KH_2PO_4$ (0.002M). This 150mM solution was diluted in Milli-Q-water to make a 10mM solution.

<u>Fabrication of CMD arrays</u>

For polymer chips, the precisely delimited region of low-fouling polymers were made using a non-contact dispensing robot (BioChip Arrayer, Perkin Elmer Life and Analytical Sciences, Boston, MA) with arraying capabilities such as those used to manufacture DNA and protein chips. Each piezo-tip is able to deliver approximately 350 pL per pulse at a maximum rate of 500 pulses per second. The number of spots depends on the surface area of the solid substrate and the surface area that is needed for the application.

To investigate structure diversity of carbohydrate macromolecules, CMD preparations of different compositions (MW, %COOH, solution concentration) and immobilization conditions (NaCl/monomer constituting the dextran and EDC+NHS/COOH ratios) were printed on solid surfaces previously coated with an amine-bearing surface. As many parameters were tested and as CMD arrays were characterized by various techniques, to reduce analysis time, eight (8) CMD conditions were randomly chosen by Design-Expert 6.0 software (StatEase Inc, Minneapolis, MN, USA) based on a quarter $2^k$ factorial design model. Conditions studied are presented in Table 5.

Table 5: CMD immobilization conditions used to produce spots of CMD graft layers.

| Conditions | #1 | #2 | #3 | #4 | #5 | #6 | #7 | #8 |
|---|---|---|---|---|---|---|---|---|
| MW (kD) | 70 | 500 | 70 | 500 | 70 | 500 | 70 | 500 |
| %COOH | 5 | 5 | 50 | 40 | 5 | 5 | 50 | 40 |
| C (mg/mL) | 1 | 1 | 1 | 1 | 3 | 3 | 3 | 3 |
| NaCl:monomer | 10 | 1 | 1 | 10 | 10 | 1 | 1 | 10 |
| EDC:COOH | 10 | 0.1 | 10 | 0.1 | 0.1 | 10 | 0.1 | 10 |
| NaOH 10N | | | + | + | | | + | + |
| $\mu$l (mM) | 60 | 6 | 70 | 150 | 185 | 20 | 80 | 365 |

+ indicates when 10M NaOH was added to enhance the dissolution of CMD.

Clean borosilicate and SPR substrates were first surface-modified by plasma polymerization of n-heptylamine (99.5% purity, cat. #126802, Sigma-Aldrich, Oakville, Canada) in a custom-built plasma reactor. Briefly, the reactor chamber is made of a glass cylinder (28-cm inside diameter, 50-cm height). Two disk-shaped horizontal copper electrodes (14-cm diameter, 5-mm thickness) are maintained in the chamber by vertical copper rods (top rod: 7-mm diameter with an adjustable vertical position; bottom rod: 2.5-cm diameter, 17-cm length). Samples were placed onto the bottom electrode. Conditions of operation used were an initial monomer pressure of 0.040 Torr, an ignition power of 80 W, an excitation frequency of 50 Hz, a deposition time of 45 sec, and a distance between the electrodes of 10cm. The resulting n-heptylamine plasma polymer (HApp) layer exposes functional amine groups that were used to graft CMD layers using carbodiimide chemistry.

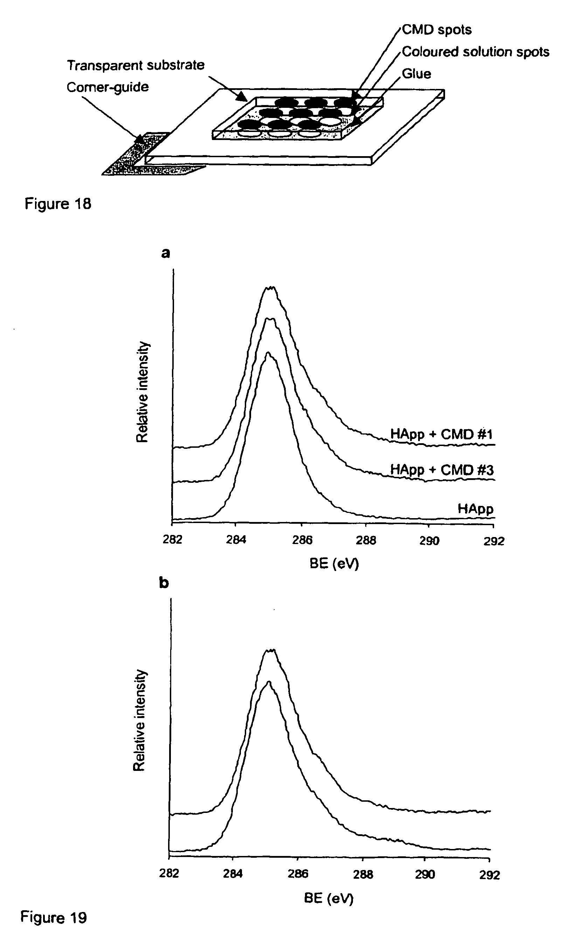

NaCl was added to CMD solutions at varying ratios (Table 5); 10M NaOH was added to enhance CMD dissolution in some systems (as indicated in Table 5). CMD solutions were filtered (0.45-$\mu$m pore size filters) to eliminate CMD aggregates. Then, solutions were activated with (1-ethyl-3-(3-dimethylaminopropyl)carbodiimide (EDC, cat. #E1769, Sigma-Aldrich) and *N*-hydroxysuccinimide (NHS, cat. #H7377, Sigma-Aldrich) for 10 minutes. EDC/NHS ratio was fixed to 1 for all CMD conditions. Solutions were then dispensed with the BioChip Arrayer on HApp-coated surfaces. The piezo-tips were set to dispense 50, 1000 or 1500 drops of solution per spot for AFM, XPS and SPR analyses, respectively, as determined by the areas of analysis required by these techniques. As the CMD grafting reaction is a wet-based chemistry, the reaction was allowed to proceed overnight in a humidity saturated air chamber, preventing any evaporation or condensation. CMD arrays were rinsed with 1M NaCl solution (2x12 hours), then with Milli-Q water (2x12 hours), dried and stored under argon atmosphere until required. Because it is difficult to see where the CMD spots have been deposited on the solid substrates, for XPS and AFM analyses CMD arrays were made with exactly the same design as a pattern-food colorant array (Figure 18). Briefly, a transparent borosilicate substrate was glued to a previously made food colorant array with epoxy glue, the assembly was coated by HApp and CMD spots were deposited just above colorant spots.

In parallel, to compare the surface composition of the CMD spots with fully CMD-covered surfaces, some substrates freshly covered by HApp layers were immersed in activated CMD solutions for 2 hours, under agitation, to produce surfaces uniformly covered (i.e. no arrays) by CMD graft layers. These surfaces were rinsed and preserved like the polymer arrays.

<u>Elemental composition of CMD spots by X-ray photoelectron spectroscopy (XPS)</u>

XPS analysis of arrays was performed using an AXIS HS spectrometer (Kratos Analytical Ltd., Manchester, GB) equipped with a monochromatic Al K$\alpha$ source at a power of 120 W. The pressure in the main vacuum chamber during analysis was typically $2 \times 10^{-8}$ torr. The elemental composition of the analyzed surface areas was obtained from survey spectra collected at a pass energy of 120 eV. High-resolution C 1s spectra were collected at 40 eV. Atomic concentrations of each element were calculated using CasaXPS (Casa Software Ltd) by determining the relevant integral peak areas, and applying the sensitivity factors supplied by the instrument manufacturer; a Shirley background was used. To compare the high-resolution C 1 s peak positions, the spectra were shifted to ensure that the leading edges of the fitted aliphatic CHx component were coincident. All spectral intensities were normalized to a maximal intensity corresponding to the full height of the fitted aliphatic CHx (285.0 eV) component peak. Survey scans and C 1s high-resolution spectra were collected on each spot (at least three (3) per condition).

<u>CMD spot layer structure by AFM force measurements</u>

Atomic Force Microscopy experiments were performed using a Digital Nanoscope IIIa Bioscope (Veeco Instruments, Santa Barbara, CA, USA). The interaction forces between a silica particle and immobilized hydrated CMD layers forming the arrays were measured using the colloidal probe method developed by Ducker et al. Silica colloidal spheres (mean diameter of 4.12 $\mu$m, Bangs Laboratories, Inc. Fishers, IN, USA) were attached to AFM $Si_3N_4$ cantilevers with a spring constant of 0.12 N/m via an epoxy adhesive (EP30MED, Master Bond, Hackensack, NJ, USA). The spring constant of the modified cantilevers was determined to be 0.1399 N/m using the resonance method proposed by Cleveland et al. Cantilever deflection versus travel distance curves were converted to plots of force-versus-separation distance using a home-made software. The linear region of the deflection curve was used to define zero separation distance.

Force measurements between silica colloidal probes and the CMD graft layers on the arrays were collected at room temperature in 10mM or 150mM PBS, allowing for equilibration for at least 1 hour before measurement. Three (3) different positions were studied per spot, for each buffer, two (2) spots per condition were analyzed, and at least ten (10) force curves were obtained for each position.

<u>CMD spot homogeneity and CMD fouling from serum measured by surface plasmon resonance (SPR) microscopy</u>

The SPR instrument from Resonant Probes GmbH (Goslar, Germany) uses a monochromatic laser light source at a wavelength of 633 nm. The beam was directed through a glass prism to the gold-coated glass substrate optically coupled to the prism using immersion oil (cat. #1812X, Cargille Laboratories Inc., NJ, USA). The reflected laser light intensity (including the SPR signal) was collected by a two-dimensional CCD camera coupled to a zoom lens as a function of the angle of incidence. The resulting data was processed by a computer to obtain experimental plasmon angles. For each polymer spot, angular scan curves were recorded from at least three sampling areas located centrally in each spot and at least two areas from surrounding HApp layers.

For serum adsorption assays, SPR data were recorded in air before and after serum adsorption. Following a first SPR analysis of the CMD arrays prior to serum incubation, CMD arrays were rinsed in 150mM PBS and incubated in a 50% (v/v) fetal bovine serum (FBS, cat. #F1051, Sigma-Aldrich) solution made in 150mM PBS for 1 hour at room temperature under agitation. Serum is a biological fluid composed of wide range of proteins and molecules and was chosen to test general protein resistance of CMD layers. Substrates were then rinsed with 150mM PBS, Milli-Q water, and dried with 0.2-$\mu$m filtered air before the second SPR analysis. The shift in plasmon angle, $\theta_{SPR}$, was measured following serum adsorption. The experiment was repeated twice.

<u>Results and Discussion</u>

<u>Elemental composition of CMD spots by XPS</u>

A high-precision robot designed to produce DNA micro-arrays was successfully utilized to spot CMD on HApp-coated borosilicate surfaces. CMD arrays were rinsed, dried and analyzed by XPS. Survey and high-resolution C 1s spectra were taken on CMD spots and on HApp background surfaces between the CMD spots.

XPS analysis of freshly deposited HApp layers on borosilicate substrates indicated a polymer rich in hydrocarbon- and nitrogen-containing species (Table 6). The elemental composition obtained on CMD spots revealed a marked increase in oxygen content relative to that observed on freshly deposited HApp layers, confirming the successful attachment of the polysaccharide to the HApp surface. Figure 19a shows representative examples of high-resolution C 1s spectra indicating the introduction of oxygen containing carbon species C-O (286,5 eV), C=O (287,9 eV) and O-C=O (289,2 eV), also confirming the grafting of CMD for all the immobilization conditions tested.

Table 6: Elemental composition of CMD arrays and fully covered control CMD surfaces on borosilicate glass determined by XPS analyses.

| | Atomic concentration % | | | Atomic ratio | |
|---|---|---|---|---|---|
| | C1s | O1s | N1s | O/C | N/C |
| Fresh HApp | 86.7 | 4.7 | 8.5 | 0.05 | 0.10 |
| Array HApp | 81.8 | 10.5 | 7.75 | 0.13 | 0.10 |
| #1 | 78.4 | 14.7 | 7.0 | 0.19 | 0.09 |
| #2 | 77.9 | 15.8 | 6.4 | 0.20 | 0.08 |
| #3 | 76.7 | 14.4 | 8.9 | 0.19 | 0.12 |
| #4 | 82.0 | 10.6 | 7.5 | 0.13 | 0.09 |
| #5 | 82.4 | 10.9 | 6.8 | 0.13 | 0.08 |
| #6 | 75.3 | 18.4 | 6.3 | 0.24 | 0.08 |
| #7 | 81.2 | 10.5 | 8.3 | 0.13 | 0.10 |
| #8 | 78.3 | 13.8 | 8.0 | 0.18 | 0.10 |
| #1 surface | 79.2 | 15.4 | 5.4 | 0.19 | 0.07 |
| #7 surface | 83.1 | 10.2 | 6.7 | 0.12 | 0.08 |

XPS analyses revealed that HApp layer on the array has a higher oxygen concentration than that of freshly deposited HApp layer bearing no CMD spot. This is due to spontaneous oxidation of the HApp layers via residual radicals while exposed to air between the plasma deposition, the making of the CMD arrays, and XPS analyses. Hence, CMD solutions were immediately deposited on HApp layers after removal from the plasma reactor, because post-plasma surface oxidation is a rapid process.

Finally, it was verified whether or not the surface compositions of the CMD spots were similar to those of CMD graft layers immobilized on fully covered surfaces and produced using the same immobilization conditions than those of CMD spots. Fully covered control CMD surfaces made according to the immobilization #1 and 7 were analyzed by XPS. XPS analyses revealed higher oxygen concentration on the uniform CMD surfaces made using conditions #1 (i.e. using no array) than on the corresponding CMD spots of the arrays. But, the O/C ratios were similar when comparing spots and fully covered surfaces for the 2 conditions tested. High-resolution C 1s spectra of CMD layers immobilized using conditions #1 on arrays and on fully covered surfaces are presented in Figure 19b and show similar profiles, with a lower O-C=O signal (at 289,2 eV) for the CMD spots. The difference may lie in the absence of agitation during the immobilization reaction on arrays that could result in a slightly less efficient immobilization.

Following the development of high-throughput technologies that include nucleic acid- and protein-based micro-arrays, researchers have immobilized carbohydrates in an array fashion for specific oligosaccharide-protein and polysaccharide-antibody interactions. The latter study showed that dextrans preserve their immunological properties, and hence chain conformation when adsorbed on solid substrates to create arrays. Here, O/C ratios obtained suggest that polysaccharides can be immobilized on arrays and may be used as acceptable models of the corresponding uniform surfaces.

Of particular interest is the effect of the EDC+NHS/COOH ratios on the O/C ratio as measured by XPS analyses of the CMD spots. Comparison of the O/C ratios of CMD layers immobilized using high EDC+NHS/COOH ratios (conditions #1, #3, #6, and #8) to those of CMD layers grafted using small EDC+NHS/COOH ratios (conditions #4, #5, and #7) indicates a lower grafting density for the latter, as less carboxyl groups were activated in CMD solutions and available to be involved in covalent linkage. Accordingly, CMD molecules immobilized on a plasma surface via a polyamine spacer layer were bound in higher amount than on HApp, owing to a higher grafting density. CMD coatings produced under conditions #2 and #6 presented higher oxygen amount than their counterparts. These conditions involved the use of very low ionic strength (Table 5) and therefore the quasi-absence of a screening effect, possibly resulting in an electrostatic attraction between the negatively charged CMD molecules and the positively charged freshly deposited HApp layers exposing partially protonated amine groups. The electrostatic attraction should lead to an improved diffusion towards the surface and a reorientation of the polymer chains so that maximum carboxyl groups are in contact with the surface.

AFM colloidal probe interaction forces with HApp layers

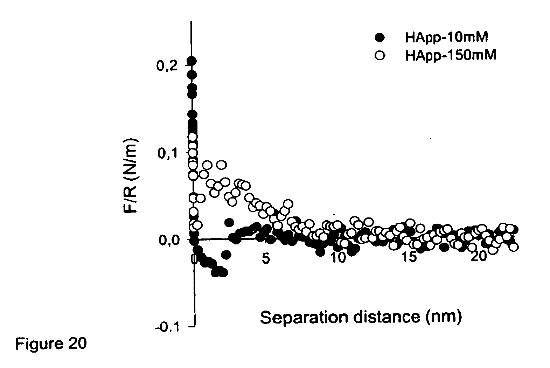

Figure 20 shows AFM surface force measurements performed between a silica colloidal probe and HApp layers on the array, at two different electrolyte concentrations. At 10mM PBS, the force-versus-separation curves show a jump-to-contact probably due to van der Waals forces acting between the silica colloidal probe and the HApp layers. A strong attraction was seen on retraction. The oxidized plasma surface bears some amide groups, which are very weak bases, probably not protonated at pH 7.4. Hence, the surface-oxidized HApp layer is almost neutral, explaining why there is no electrostatic attraction between the negatively charged silica sphere and the oxidized HApp surface on approach. However, at 150mM, interaction forces experienced between the silica colloidal probe and the HApp surfaces were repulsive over a small range, with a jump-to-contact before the hard-wall repulsion. Ionic species present in the buffer may have adsorbed on the surface during the equilibration step before measurements.

AFM colloidal probe interaction forces with CMD spots grafted using high EDC+NHS/COOH ratios

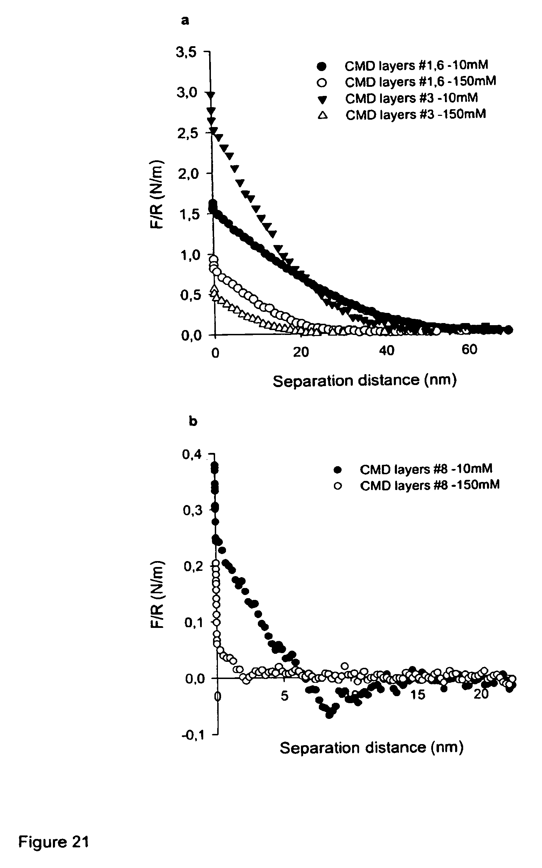

Surface force measurements recorded during the approach between silica colloidal probes and spots of CMD grafted on HApp layers using high EDC+NHS/COOH ratios are shown in Figure 21. An exponential repulsion between the colloidal probe and the CMD layer was observed at all electrolyte concentrations for conditions #1, #3, and #6 (21a). No adhesion was observed on retraction of the probe from the surface. The exponential repulsion exhibited a dependence on electrolyte concentration, reducing in range and distance with increasing ionic strength. However, the decay lengths of the force profiles presented do not match the theoretically calculated value for purely electrostatic interactions between surfaces immersed in salt solutions of the concentration used here, with higher discrepancy for CMD layers made with low carboxylated CMDs. For instance, the interaction forces in 10mM PBS displayed a decay length of approximately 13.2 nm for conditions #1 and #6 compared to a value of 2.5 nm for purely electrostatic forces. Thus, the interactions measured for the CMD surfaces were not purely electrostatic in nature. While repulsion has an electrostatic component, it can be concluded that the interaction force profiles presented here are largely a result of compression of the covalently attached CMD layers by the silica sphere. Polysaccharides are highly hydrated polymers with extended flexible chains, thus compressible. Moreover, CMD molecules are weak polyelectrolytes bearing negative charges repelling each other, intra- and inter-molecularly, which causes chain extension in solution. When electrolyte concentration increases, the diffuse ionic atmosphere screens charges, and the polymer chains collapse in a more random coil conformation.

CMD layers made of highly carboxylated CMD produced under conditions #3 appear to have smaller chain extension than those made of low-carboxylated CMDs (i.e. conditions #1 and #6) at all electrolyte concentrations tested here. Carboxyl density seems to regulate the number of pinning points on the polymer chain, thus the size of loops and tails protruding in solution.

Force-versus-separation curves of CMD spots made using conditions #8 differ from the ones previously presented: no exponential repulsion was observed between the colloidal probe and the surface, but rather a small jump-to-contact possibly resulting from van der Waals attraction was noted, followed by a relatively small compression dependent on electrolyte concentration (Figure 21 b). Also, an adhesion was observed on retraction in both buffers. This behavior suggests an absence of electrostatic repulsion between the silica sphere and the CMD layer, despite a high carboxyl group density, and a reduced extension of polysaccharide chains in solution. In Table 5, it can be found that the immobilization conditions #8 also differ from other conditions in that the ionic strength during the grafting reaction was really high. Reduction of intra- and inter-molecular electrostatic repulsion may have lead to the adsorption of random coiled shrink chains, packed close to the surface. The presence of a high-density of carboxyl groups and the use of a high EDC+NHS/COOH ratio induced the formation of many covalent bonds between the surface and CMD chains, forming many contacts with the surface (trains) and few short extensions in solution (loops and tails). High density of train segments came with a high surface area occupied per polymer chain i.e. fewer molecules were immobilized per unit area, resulting in a relatively low O/C ratio obtained by XPS. Moreover, as most carboxyl groups may have been engaged in covalent linkage with the surface, exposed charged groups and electrostatic interactions with the silica sphere were reduced. The layer was densely packed close to the surface, but had loops which extension and conformation freedom were reduced even at 10mM, resulting in a high polymer free energy. To lower the polymer free energy, there may have been displacement of Na+ ions towards the layer or a reduced dissociation of carboxyl groups to decrease electrostatic repulsion between polymer segments, and the need for chain extension. Both carboxyl groups engagement in trains and charges neutralization in loops would prevent electrostatic interactions with the silica sphere.

Hence, CMD graft layers that exerted repulsion towards silica spheres seemed able to do so via electrostatic interactions through charge exposition, and via steric-entropic repulsion owing to some chain extension and compressibility in solution.

AFM colloidal probe interaction forces with CMD spots grafted using low EDC+NHS/COOH ratios

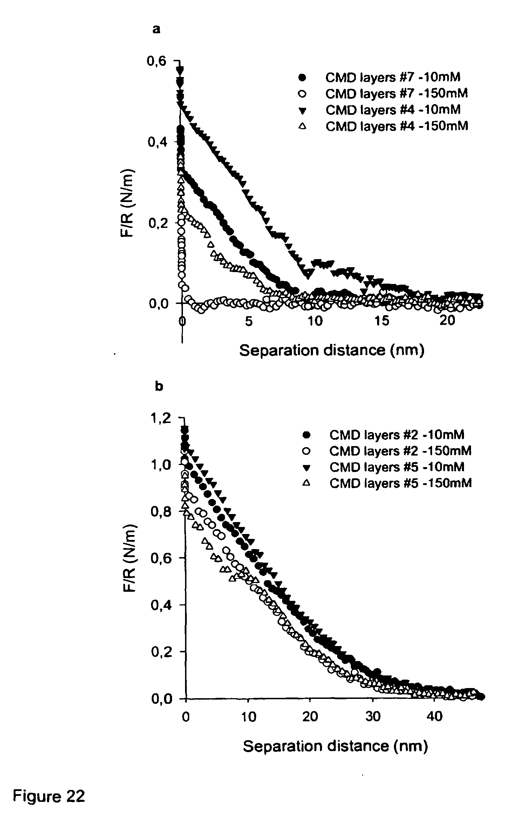

Force measurements recorded between a silica colloidal probe and spots of CMD made using conditions #7 showed a small attractive jump, resulting from van der Waals attraction in both electrolyte concentrations (Figure 22a). Following the jump between the two surfaces, hard wall repulsion was observed with a minimal compressibility that was dependent on electrolyte concentration, being larger for the lower electrolyte concentration. Strong attraction was observed on retraction. The lack of polymer extension into solution, shown by surface force measurements along with the XPS data, suggests a minimal amount of surface-immobilized CMD on the surface. Only few quite extended molecules seem randomly attached on the surface.

On the contrary, interaction forces between CMD layers made using conditions #4 with silica colloidal probes showed an exponential repulsion between the probe and the spots, with small discontinuities indicative of attractive jumps. Adhesion on retraction was observed. Decay length measurements suggest that the exponential repulsion is partly electrostatic in nature, but also due to the presence of a compressible layer, despite immobilization of few molecules on the surface (measured decay lengths of 5.2 nm compared to a theoretical value of 2.5 nm). Immobilization conditions #4 involved a high electrolyte concentration. As previously mentioned for conditions #8, in such conditions, polysaccharide molecules adsorb on the surface as shrink molecules close to the surface, whereas for CMD layers produced using conditions #7, molecules adsorbed on the surface in a quite extended conformation. As EDC+NHS/COOH ratio was low, only few carboxyl groups in contact with the surface were engaged in covalent linkage, probably leading to the formation of no trains but of short loops and tails. The resulting layers would seem not as diffuse as those made with conditions #7. They were more regular.

Figure 22b shows interaction forces experienced between a silica colloidal probe and layers made of low-carboxylated CMD molecules (conditions #2 and #5) immobilized with a small EDC+NHS/COOH ratio. As for layers made of low-carboxylated CMD molecules (conditions #1 and #6), they exhibit exponential and long-range repulsion, but almost not dependent on electrolyte concentration. Also, chains do not collapse at high electrolyte concentration. They behave like neutral polysaccharides, which extension and repulsion are not due to electrostatic interactions but rather to chains hydration. The low EDC+NHS/COOH ratio combined with low carboxylation degree suggest a low grafting density following the immobilization, leading to an uncharged diffuse layer, but extended enough to shield the plasma surface. However, at high electrolyte concentration, analysis of the interaction forces between a silica sphere and CMD layers made under conditions #5 showed that there was adhesion upon retraction and small attraction was sometimes observed on approach. This was not observed with layers made under conditions #2. As previously mentioned, immobilization of CMD layers made using conditions #2 involved the use of a very low salt concentration, allowing the adsorption of oriented and extended molecules, while those made using conditions #5 involved the use of quite high ionic strength, probably forming a randomly packed layer, with a high surface area occupied per molecule, hence, a reduced amount of adsorbed polysaccharide despite the high CMD solution concentration. Both final layers presented extended chains but in a different fashion: layers produced using conditions #2 were quite dense and uniform in term of chain extension and spatial distribution (at the molecular scale) while those produced using conditions #5 were not uniformly covering the surface, with random distribution of long loops and tails, and possibly some pinholes.

Low pinning density (resulting from a low EDC+NHS/COOH ratio) leads to an incomplete coverage of the surface with formation of mostly irregular, inhomogeneous layers, similar to adsorbed layers. Yet, immobilization in absence of salt induced adsorption of extended and oriented molecules on surface, improving the efficiency of immobilization; the homogeneous and extended formed layers provide steric hindrance from the surface.

CMD spot homogeneity and CMD fouling from serum measured by SPR microscopy

Surface plasmon resonance (SPR) spectroscopy is a widely used method for the determination of optical thickness of thin layers. When p-polarized light is shined through the glass slide and onto the gold surface at angles near the so-called "surface plasmon resonance" condition, the optical reflectivity of the gold changes very sensitively with the presence of a thin coating on the gold. SPR measures a certain combination of thickness and refractive index and the shift of the plasmon angle is used to determine the optical thickness.

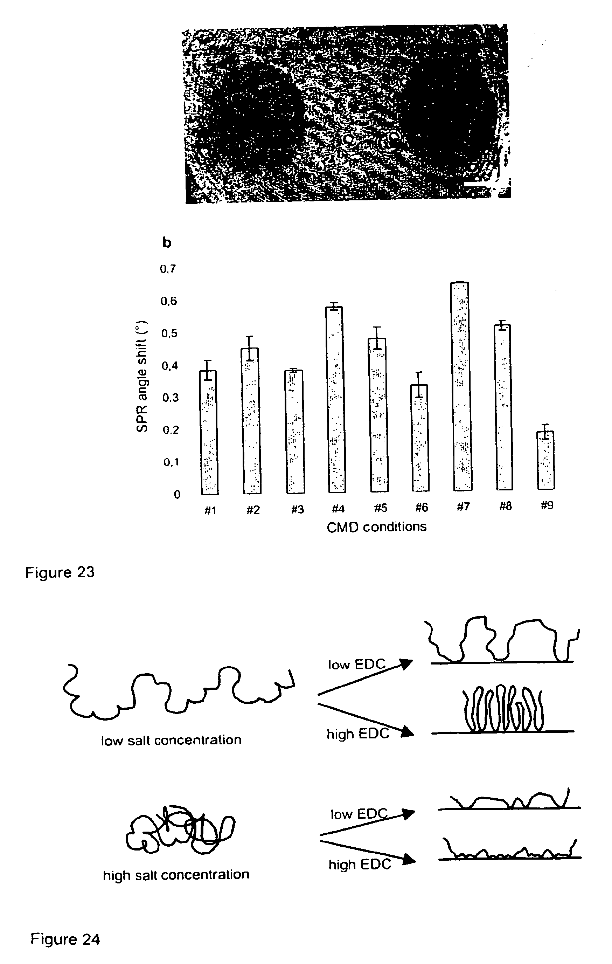

Reflected intensity is collected by a CCD camera through a zoom lens, and at plasmon angle, spots appeared as uniform dark surfaces distinct from HApp background (Figure 23a). CMD spots seemed homogeneous in optical density suggesting that CMD immobilization occurred uniformly across the whole spot area. As round spots were observed from an oblique angle, they appeared as ellipses.

CMD arrays were then exposed to a serum solution and analyzed by SPR microscopy. Serum is a biological fluid composed of many proteins (of various sizes, charge densities and shapes), and was then chosen to test general layer resistance to biofouling (including protein adsorption). CMD arrays were exposed to harsh conditions (incubation with 50% FBS, for 1 hour under agitation) first to enhance differences between CMD layers fouling levels, and second, to be more selective, to screen for the best fouling resistant surface(s). Figure 23b presents a summary of the serum adsorption on the different CMD spots tested here.

CMD layers made using conditions #4 and #7 were subject to non-specific adsorption from the FBS solution. XPS and AFM data suggest that those layers did not completely cover the surface and were not extended in solution. Plasma could shine through the CMD graft layers, therefore allowing for more serum component adsorption. CMD layers produced using conditions #2 and #5 presented a thicker, more extended layer, hindering the underlying plasma surface from the silica colloidal probe. Yet, serum components could largely adsorb: the absence of charge in the layers indicates the immobilization of a diffuse layer with an open structure, allowing proteins to penetrate [Piehler et al. Biosens. Bioelectron. 1997, 12, 531-538 and McArthur et al. Colloids and Surfaces B-Biointerfaces 2000, 17, 37-48]. CMD layers made using conditions #2 and #5 have mobile protruding chains, but too separated, therefore, proteins could penetrate without disturbing the chain conformation and the hydration layer.

On the contrary, when proteins approach dense layers with protruding loops such as CMD layers made using conditions #1, #3, and #6, flexible polymer chains are compressed, causing an entropic loss. Moreover, water molecules tightly bound to the polysaccharide units would be expulsed, causing an increase in polymer free energy; both phenomena would lead to better protein repulsion. No difference are noted between layers made using conditions #1 and #3, suggesting that the extension range and charge density have no major effect on protein rejecting ability. However, layers made under conditions #6 seem to be more protein resistant. XPS data revealed that a higher amount of polysaccharide was immobilized, most likely creating a denser and more repulsive layer. As previously mentioned, this may be due to better molecule reorganization at the surface or to higher CMD solution concentration during immobilization (Table 5). Besides, the quite rigid conformation of layers made using conditions #8, along with a high train conformation density and reduced loop extension and mobility, would prevent chain compressibility under protein contact and steric repulsion. Hence, density and thickness are important parameters on protein repulsion: proteins could penetrate into loose layers like those made under conditions #2 and #5, while the absence of compressibility (thin layers) observed in CMD layers produced under conditions #4, #7 and #8 prevented steric-entropic repulsion. As CMD are highly hydrated polymers, their ability to reject serum components (including proteins) may also result from the strength of chains interactions with surrounding water molecules.

CMD layers showing some resistance to serum adsorption were extended enough and dense and were characterized by a high EDC+NHS/COOH ratio, a low salt concentration and seemed more repellent when produced using a higher CMD solution concentration. Hence, an optimized immobilization condition (called #9) was defined from the results presented above, corresponding to 70kDa CMD MW, 25% carboxylic residues with a high EDC+NHS/COOH ratio and a 2mg/ml CMD solution concentration. This condition was used to produce CMD arrays to be tested towards serum adsorption (Figure 23b) and showed a low level of serum adsorption, well below CMD layers produced using conditions #1, #3, and #6. The combination of a higher CMD solution concentration with a high pinning density must have led to the immobilization of a denser and extended layer. This experiment allowed to determine immobilization parameters involved in layers ability to adsorb or reject proteins.

Conclusions

Carboxymethyl dextran (CMD) array technology was successfully developed and validated to simultaneously study covalent immobilization modes of CMD layers and their resulting CMD layer structures to relate them to the resistance towards serum adsorption.

XPS analysis was used to characterize the surface elemental composition, while AFM force measurements were used to study surface roughness and layer structure and density. XPS analyses revealed that polymer layers immobilized on arrays presented the same characteristics as layers grafted on uniform surfaces. Hence, polymer chips may be useful in surface sciences to study polymer structural diversity and relate their properties to their low-fouling behavior, in a high-throughput manner.

Screening of the CMD immobilization conditions revealed the importance of grafting reaction conditions on layer properties. Electrolyte concentration during immobilization influences polymer conformation upon initial adsorption, while EDC+NHS/COOH ratio and carboxylation degree regulate final layer density, segments distribution in trains, loops and tails and hence the layer thickness (Figure 24 shows a summary). Our results suggest that a lower ionic strength induces adsorption of an extended and ordered layer, resulting in diffuse or dense mobile layer depending on pinning density. The use of higher salt concentrations during immobilization likely initially leads to shrink, randomly adsorbed molecules on surface, forming erratic layers, with random distribution of loop sizes at low pinning densities, or the presence of many trains and reduced flexible loops in case of a high pinning density. The present XPS, AFM, and SPR analyses revealed no significant effect of the CMD molecular weight on the layer properties and on the level of fouling by serum.

Finally, serum adsorption experiments showed that diffuse, irregular and/or constrained layers were not resistant to serum adsorption. Dense, uniformly extended and highly hydrated layers have better protein repellence.

## EXAMPLE 6

**Study of cell adhesion resistance mechanisms using arrays of dextran-derivative layers.**

In the previous example, arrays of thin graft layers made of a dextran derivative i.e. carboxy-methyl-dextran (CMD), were developed to examine how immobilization conditions affect CMD layers structure and protein repellence. Solution ionic strength during immobilization influenced polymer conformation upon initial adsorption, while coupling agent concentration and carboxylation degree regulated final layer density and thickness. Moreover, protein adsorption experiments showed that minimum surface density and layer thickness were associated with a better protein rejection.

To further investigate how immobilization conditions used to produce CMD graft layers influence the cell repulsion ability of these layers, and how such repulsion can be optimized, CMD arrays were exposed to cells. Low-fouling polymer arrays were tested for their ability to reject cell adhesion. Short-term and long-term cell culture assays were conducted with human fibroblasts i.e. arrays were exposed to non-confluent and confluent cells. CMD graft layers cell resistance and cell behavior on these coatings were studied and related to immobilization conditions and layer structure. Finally, the cell resistance of an optimized CMD layer and that of a PEG layer were compared.

As explained above, control of cell-material interactions is a consideration in biomaterials science and tissue engineering. To improve biomaterial implant performance, one needs to control cellular interactions at the tissue-biomaterial interface using well defined surfaces. In this way, the purpose of bioactive surfaces is to promote specific cell and tissue responses, including selective cell adhesion. Controlled cell interactions was achieved firstly, by minimizing non-specific protein adsorption and more importantly cell adhesion and secondly, by chemically grafting specific cell ligands on cell-resistant biomaterial surfaces.

Polymer layers ability to resist protein adsorption and subsequent cell colonization in an organism results from interactive forces between the surface and molecules from biological fluids. These interactions may arise from various origins, such as electrostatic, van der Waals or hydration forces and steric-entropic effects.

Materials and Methods

Carboxy-methyl-dextran (CMD) synthesis

Carboxy-methyl-dextrans (CMDs) with different ratios of carboxyl groups to sugar residues were prepared as follows. Five (5) g of dextran (70 and 500 kDa MW, Amersham Biosciences, Uppsala, Sweden, cat. # 17-0280-01 and 17-0320-01, respectively) were dissolved in 25ml of a 1M NaOH solution. Then, bromoacetic acid (Lancaster Synthesis Inc., Pelham NH, cat. # 3016) was added to a final concentration of either 0.125, 0.5 or 1M. The solution was stirred overnight and dialyzed against Milli-Q water, 1 M HCl, and finally Milli-Q water for 24 hours each. Solutions were then lyophilized and stored at -20°C. The carboxylation degree was determined by $^1$H NMR: carboxylation degrees were 5%, 25%, and 50% for 70-kDa dextran treated with 0.125M, 0.5M and 1M bromoacetic acid, respectively, and 5% and 40% of carboxylated residues for 500-kDa dextran treated with 0.125M and 1M bromoacetic acid, respectively.

Fabrication of arrays of CMD graft layers

To investigate cell resistance of CMD graft layers, CMD preparations of different compositions (MW, %COOH, solution concentration) with the use of different immobilization conditions (NaCl/sugar unit and EDC+NHS/COOH ratios) were printed on surfaces previously covered with an amine-bearing surface. Conditions studied are presented in Table 7.

Table 7: CMD immobilization conditions used to produce arrays of CMD graft layers.

| Conditions | #1 | #2 | #3 | #4 | #5 | #6 | #7 | #8 | #9 |
|---|---|---|---|---|---|---|---|---|---|
| MW (kDa) | 70 | 500 | 70 | 500 | 70 | 500 | 70 | 500 | 70 |
| %COOH | 5 | 5 | 50 | 40 | 5 | 5 | 50 | 40 | 25 |
| Concentration (mg/ml) | 1 | 1 | 1 | 1 | 3 | 3 | 3 | 3 | 2 |
| NaCl:sugar unit | 10 | 1 | 1 | 10 | 10 | 1 | 1 | 10 | 5 |
| EDC+NHS:COOH | 10 | 0.1 | 10 | 0.1 | 0.1 | 10 | 0.1 | 10 | 5 |
| 10M NaOH | | | * | * | | | * | * | |

(continued)

| Conditions | #1 | #2 | #3 | #4 | #5 | #6 | #7 | #8 | #9 |
|---|---|---|---|---|---|---|---|---|---|
| μl (mM) | 60 | 6 | 70 | 150 | 185 | 20 | 80 | 365 | 60 |

* indicates when 10M NaOH was added to help dissolution of CMD solutions (pH was between 5.5 and 6). μl stands for solution final ionic strength.

Details of the fabrication procedures of these arrays of CMD graft layers are the same as in the previous example. Briefly, CMD arrays were made on borosilicate glass (Chemglass, Vineland, NJ) washed in 35% nitric acid overnight, rinsed with Milli-Q water, and dried with 0.2-μm filtered air. Borosilicate glass substrates were surface-modified by plasma polymerization of n-heptylamine (99.5% purity, Sigma-Aldrich, Oakville, Canada, cat.# 126802) in a custom-built plasma reactor. Conditions of plasma polymerization were an initial monomer pressure of 0.040 Torr, an ignition power of 80 W, an excitation frequency of 50 Hz, a deposition time of 45 sec, and a distance between the electrodes of 10 cm. The resulting n-heptylamine plasma polymer (HApp) layers expose functional amine groups that were used to graft CMD layers using carbodiimide chemistry.

NaCl was added to CMD solutions at varying ratios. In some samples, 10M NaOH was added to help dissolution (see Table 7 for conditions). CMD solutions were first filtered with 0.45-μm pore size membrane to limit CMD aggregates and then activated with (1-ethyl-3-(3-dimethylaminopropyl)carbodiimide (EDC, Sigma-Aldrich, cat. # E1769) and N-hydroxysuccinimide (NHS, Sigma-Aldrich, cat. # H7377) for 10 minutes. EDC/NHS ratio was fixed to 1 for all CMD immobilization conditions. It should be noted that upon addition of EDC/NHS to CMD solutions (even those in which NaOH was added), pH reaches values of 5.5-6.0. Solutions were then dispensed in duplicate with a non-contact robot designed to produce arrays (BioChip Arrayer, PerkinElmer) on HApp-coated surfaces. The piezo-tips were set to dispense 50 drops of solution per spot with 350 pL of sample per drop, resulting in ca. 600-μm diameter spots. The reaction was allowed to proceed overnight in a chamber saturated with humidity to maintain the substrates at dew point. CMD arrays were rinsed with 1 M NaCl solution (2x12 hours), then with Milli-Q water (2x12 hours). They were autoclaved and stored in sterile Milli-Q water at 4°C until needed.

As a comparison, a PEG solution at cloud-point conditions was also printed to produce PEG graft layers with excellent low-fouling properties. Cloud point conditions induce molecules packing, forming a dense layer. Boc-NH$_2$-poly (ethylene glycol)-NHS of 3400-Da MW (Nektar Therapeutics, San Carlos, CA, USA, cat. # 4M530F02) was attached on the HApp layers under cloud point conditions, using a 20% Na$_2$SO$_4$ solution at room temperature to yield a final PEG concentration of 1mg/ml.[4]

Testing arrays of CMD graft layers towards cell responses

Human foreskin fibroblasts were cultured in sterile Dulbecco's modified eagle's medium (DMEM, Gibco, Grand island, NY, cat. # 12100) with 10% fetal bovine serum (FBS, Sigma, cat. # F1051), 100 units/ml penicillin and 100 μg/ml streptomycin (Gibco, cat. # 15140). Cells were incubated at 37°C in a 5% CO$_2$ incubator. Fibroblasts were chosen to perform cell adhesion assays as these cells are known to easily adhere on most solid substrates.

Short-term cell adhesion assay

Cells were trypsinised (trypsine-EDTA, Gibco, cat. # 25200), suspended in complete culture medium and seeded at 15,000 cells/cm$^2$ on autoclaved CMD arrays. Autoclaving does not have a significant effect on the surface properties of CMD graft layers. Cell incubation was stopped after either 4 or 12 hours: for each incubation time, two arrays were fixed with 3.7% formaldehyde during 15 min for further labeling.

Long-term cell adhesion assay

Cells were seeded at 10,000 cells/cm$^2$ on autoclaved arrays made of CMD graft layers. Cells were observed every 24 hours and once 90% confluence was reached (72h, Day 3), experiments were allowed to proceed for another two days before cells were fixed with 3.7% formaldehyde. Images of each spot were taken using phase contrast microscopy (Nikon Eclipse TE2000-S) at 100x magnification during the three days of confluence (Days 3, 4 and 5). Spot area uncovered by cells was measured using SigmaScan Pro software (SPSS Inc., Chicago, IL). The area was outlined manually using the software.

Cell labeling

All antibodies used were purchased from Sigma. Fixed cells were rinsed with PBS and permeabilized with 0.5% Triton X-100 for 5 min and finally blocked either with 2% bovine serum albumin (BSA, cat. # A7906) for vinculin and actin labelling or with 5% goat serum (cat. # G9023) for fibronectin labeling. For actin filament labeling, samples were incubated with phalloidin-TRITC (1:400 dilution, cat. #P1951) for one hour at room temperature. For focal adhesion and secreted fibronectin staining, samples were incubated for one hour at room temperature with the primary antibody: mouse anti-vinculin (1:25, cat. # V4505) and mouse anti-human fibronectin (1:400, cat. # F0916), respectively, Then, samples were labeled with a FITC-goat-anti-mouse secondary antibody (1:250, cat. # F5262). Samples were rinsed and mounted on glass slides and observed with an epifluorescence microscope (Nikon Eclipse).

Results

The CMD arrays that were characterized in the previous example were exposed to cells: i) first, to study parameters influencing resistance of CMD layers towards cell attachment, with the aim to identify optimal immobilization conditions to produce CMD layers with excellent cell-resistance properties; ii) second, to examine cell adhesive behavior when exposed to CMD surfaces with various cell-resistance properties.

Initial cell interaction with CMD graft layers: short-term assays

Arrays of CMD graft layers were first seeded with cells for a short-time interval to investigate early responses of cells exposed to various CMD layers. *In vitro,* initial cell attachment on a substrate usually results in cell spreading, formation of focal adhesions and cytoskeletal reorganization. Focal adhesions are peripheral cellular structures where cells interact with the extracellular matrix (ECM) through integrin receptors. One of the most prominent structural component of focal adhesions is vinculin. Formation of focal adhesion sites and actin cytoskeletal organization into stress fibers are interdependent and regulate each other.

Cell morphology and secreted fibronectin matrix at 4 hours

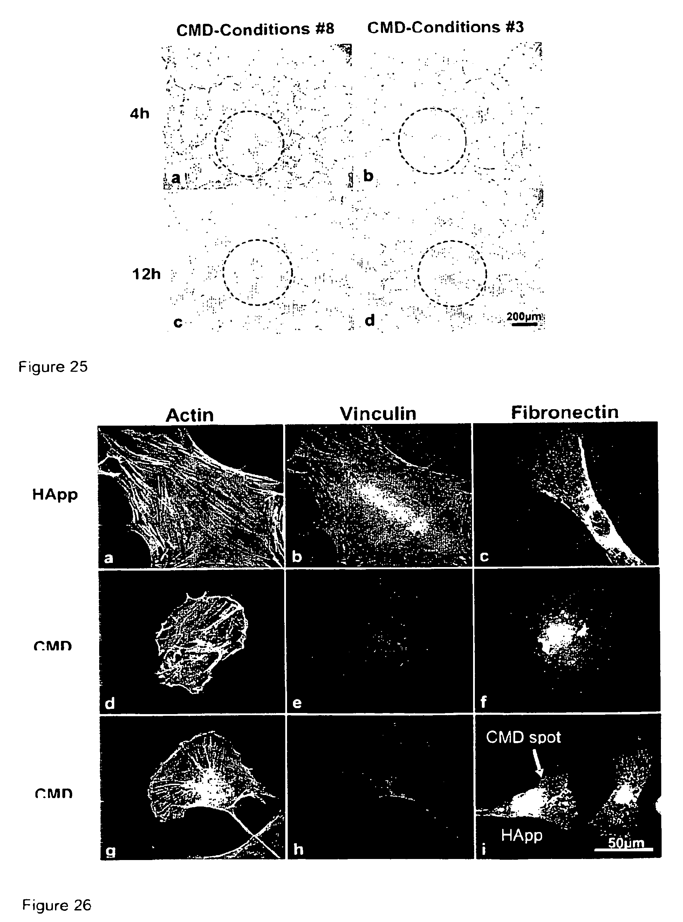

Arrays of CMD layers were seeded with a high density of fibroblasts (i.e. 15,000 cells/cm$^2$), and cells were fixed at 4 and 12 hours. Four hours following cell seeding, fibroblasts outside CMD spots, i.e. those attached on the HApp layer, appeared well spread with filopodia extensions. Few isolated ones looked small and rounded. The very few cells present on CMD surfaces were small and rounded (Figure 25b). Phalloidin actin-labeling revealed that, on HApp layers, cells had a quite organized actin cytoskeleton with the presence of stress fibers associated with focal adhesions that were characteristic of spread cells anchored to the surface (Figures 26a and 26b). Actin fibers of cells on the extreme periphery of CMD spots already tended to align along the spot curvature. However, the few small cells present on cell-resistant CMD graft layers appeared asymmetrical and showed no central stress fibers but most had bundles and a network of actin filaments at periphery (Figure 26d), usually associated with lamellipodia formation for cell spreading. Vinculin staining of focal adhesions was not visible on these cell-resistant CMD layers (Figure 26e).

As cytoskeletal architecture and ECM organization are dynamically linked through integrin signaling, human fibronectin deposition and reorganization were evaluated at this early stage of cell interaction with CMD surfaces. Fibronectin forms fibrils when activated by integrins present in fibrillar adhesions and when stress fibers exert tension on fibronectin matrix through focal adhesions. Fibronectin fibrils formation is essential as it affects the composition, organization and stability of the ECM and also the stability of cell-ECM fibrillar adhesion sites. On HApp layers, the presence of central fibronectin fibrils indicated that fibroblasts had the ability to deposit and remodel the fibronectin matrix (Figure 26c). For cells present on the CMD graft layers, no fibronectin structure, neither granular nor fibrillar, was distinguished (Figure 26f). Early cell interaction with CMD films induced limited spreading, rather cell rounding, associated with an absence of stress fibers and focal adhesions and CMD surfaces also altered cells ability to deposit fibronectin matrix.

Nonetheless, on some CMD graft layers and at the edge of some CMD spots, cells found in group exhibited thin actin stress fibers and weak focal adhesions (Figures 26g and 26h). They could remodel fibronectin matrix (Figure 26i). As cell-surface and cell-cell adhesion points both interact with the actin cytoskeleton, the formation of cell-cell adhesions also triggers reorganization of the actin cytoskeleton. Hence cell-cell adhesions modulate cell spreading and cell-surface inside-out signaling through integrins, favoring cell integrity and cell survival.

Cell morohology and fibronectin matrix deposition at 12 hours

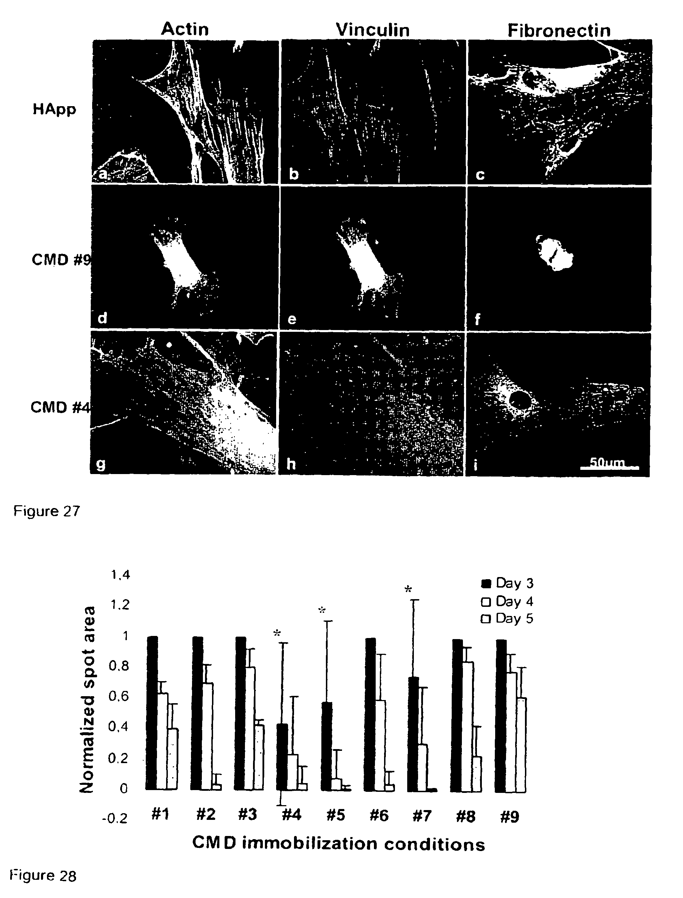

Figures 25c and 25d show that 12 hours following cell seeding, cells on HApp surfaces were more spread and emitted less filopodia than after 4 hours (Figures 25a and 25b). The presence of stress fibers associated with long focal adhesions indicates that cells strongly adhered to the HApp surfaces (Figures 27a and 27b). More fibronectin fibrils were observed at 12 hours than at 4 hours, suggesting advanced fibronectin matrix assembly. Contrary to cell responses observed on CMD arrays at 4 hours, three types of CMD spots could be distinguished at 12 hours. First, on CMD spots showing good resistance towards cell attachment, i.e. CMD layers made under conditions #1, #3, and #9, very few or no rounded cells were observed (Figure 25d). Second, on less cell-resistant CMD spots, i.e. those made under conditions #2, #6, and #8, some cells were present, rounded or spread (Figure 25c). These CMD graft layers can be referred to as semi-resistant. The few cells found on cell-resistant and semi-resistant CMD spots exhibited disrupted networks of actin cytoskeleton and vinculin, while fibronectin was not detected under the few cells (Figures 27d to 27f). Third, on CMD layers made using conditions #4, #5, and #7, cells appeared quite spread, with stress fibers associated with focal adhesions (Figures 27g and 27h). Fibronectin labeling revealed that isolated cells could deposit fibronectin in a granular structure, whereas grouped cells exposed some fibrils (Figure 27i). Twelve hours following cell seeding, fibroblasts were able to deposit proteins on those CMD layers and to establish cell-surface contacts.

CMD resistance following 3 days of cell confluence

CMD spot size evolution

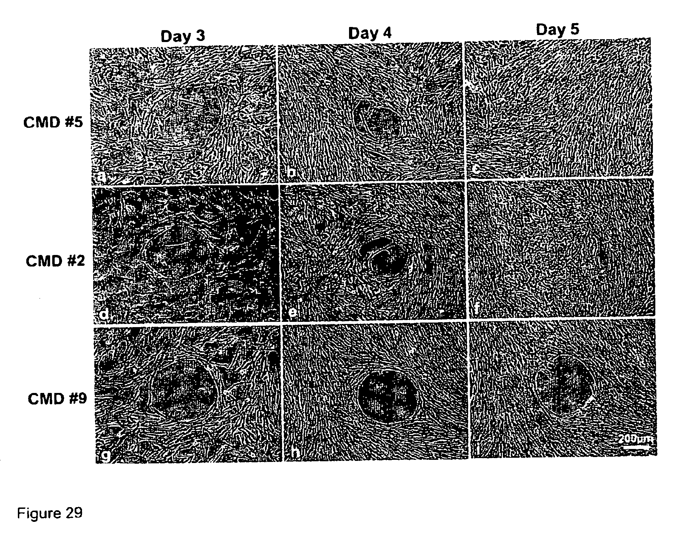

To further test CMD graft layers resistance towards cell attachment, arrays of CMD surfaces were exposed to confluent cells for three days. Preceding results showed that grouped cells having established cell-cell adhesions seemed less dependent on cell-surface signaling. Thus, exposing CMD graft layers to confluent cells constitute harder conditions to study cell resistance of thin films. CMD arrays were exposed to fibroblasts, at high seeding density (i.e. 10,000 cells/cm$^2$). Once 90% confluence was reached on Day 3, another 48 hours was elapsed before cells were fixed. The size of each spot (up to 8 spots per condition) was measured on Days 3, 4 and 5 and compared to the size measured on Day 3 to quantify surface coverage by cells (Figure 28). Figure 29 shows representative images of long-term cell adhesion assays. The 3 classes of CMD layers defined earlier regarding their cell-resistance are still observed. Some spots were partially covered by cells on Days 3 and 4 and disappeared on the third day of confluence (CMD surfaces made using conditions #4, #5, and #7; Figures 29a to 29c), while some showed good cell resistance on Days 3 and 4, but were invaded by cells on Day 5 (CMD surfaces made using conditions #2, #6, #8; Figures 29d to 29f). Finally, CMD spots made using conditions #1, #3, #9 resisted cell adhesion over the 3 days, but their size progressively decreased, with the expansion of the surrounding cell sheet (Figures 29g to 29i).

Cell morphology and fibronectin matrix on CMD array after 3 days of confluence

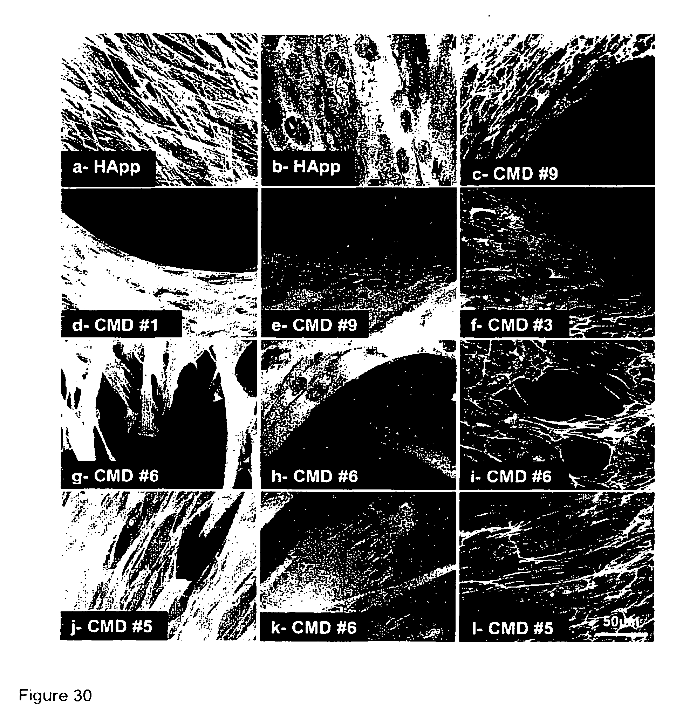

To shed more light on cell behavior observed on CMD surfaces presenting various resistance levels, cell labeling was carried out on Day 5.
Phalloidin actin-labeling of cells fixed on CMD arrays following 3 days of confluence revealed that outside CMD spots, i.e. on HApp layers, cells were arranged in a bilayer. They had well developed actin stress fibers and peripheral focal adhesions (Figures 30a and 30b). They were surrounded by a dense fibronectin fibrillar network. Cells found on covered CMD spots made using conditions #4, #5, and #7 presented stress fibers and were arranged in a bilayer as on HApp film (Figure 30j). On these CMD spots, fibronectin matrix was organized into a fibrillar network spanning several cells (Figure 30I). Except for cell density, almost no distinction could be noted between cells on HApp layers and cells on CMD spots made using conditions #4, #5 and #7 at Day 5.
On partially covered semi-resistant CMD spots (made using conditions #2, #6, #8), invading cell extensions presented a weak actin staining: they had few stress fibers when directly on CMD surfaces (Figure 30g) and few focal adhesions at extremities of isolated cells (Figure 30h), while focal adhesions were more numerous at the extremities of group of cells (Figure 30k). Some actin filaments appeared out of focus in Figure 30g: they correspond to cells forming a bridge over the spot. These cells leaned on surrounding cells and did not seem to interact with the CMD surface.
Finally, on the edge of cell-resistant CMD spots (made using conditions #1, #3 and #9), cells were piled up (i.e. stacked), showed actin stress fibers uniformly aligned on the spot curvature (Figure 30d) and a fibrillar fibronectin network. Some cells on the extreme edge showed no extracellular fibronectin (Figure 30c) probably owing to fibronectin inability to adsorb on these CMD surfaces. However, few cells "dived in" some cell-resistant CMD spots, grouped in a single layer. They showed some focal adhesions and granular fibronectin at their extremity inside the CMD spot (Figures 30e and 30f). Those invading cells seemed to need a support from neighboring cells settled on the HApp layer. Besides, as illustrated in Figures 29h and 29i, on Day 4, cells were already stacked along the spot edge and, some cells were able to get over the CMD barrier on Day 5, leaning on the cell sheet.

Validation of an optimal condition towards cell resistance

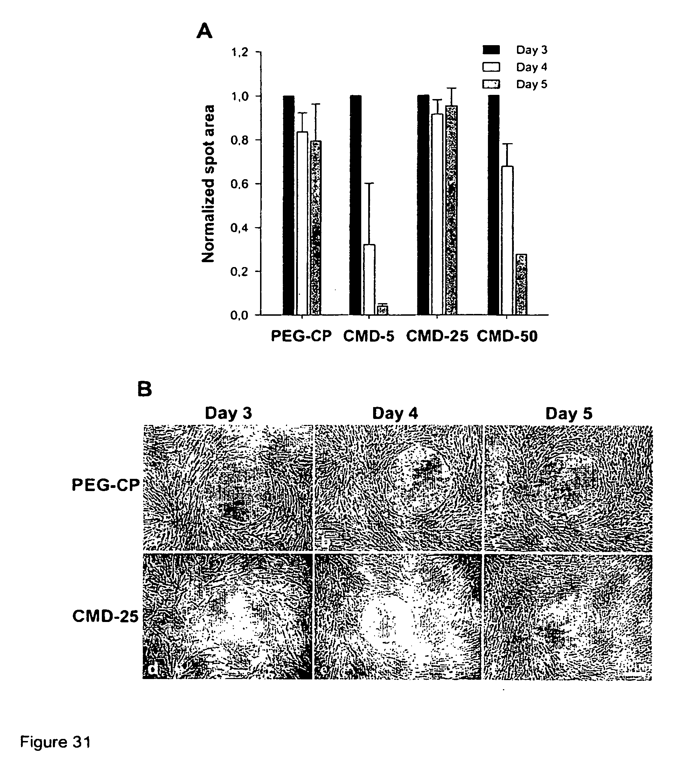

In order to determine an optimal cell-resistant CMD surface, CMD spots were made on HApp layers using a 2 mg/ml CMD solution concentration, 70-kDa CMDs, an EDC+NHS/COOH ratio of 5:1, with no added electrolyte, and with CMD molecules having 5%, 25% or 50% carboxylic residues. These conditions were selected based on results of the present example and on those of the previous example. These CMD surfaces, referred as to CMD-5, CMD-25 and CMD-50, were exposed to fibroblasts. Cell resistance of these CMD spots were compared to that of PEG layers made under cloud point conditions. These PEG layers were used as a reference, because it has been shown in a previous example that PEG layers produced under cloud point conditions had no detectable protein adsorption using quartz crystal microbalance. CMD-5 and CMD-50 layers spot size decreased progressively while CMD-25 was as resistant as PEG layers towards cell attachment and invasion (Figure 31). For some CMD-25 and PEG spots, spot size even increased between the second and third day, indicating that cells may have detached or retracted.

Polysaccharides and poly(ethylene glycol) (PEG) layers were studied for their capacity to resist protein and cell adhesion.

To find optimal low-fouling coatings, arrays of CMD layers immobilized under various conditions were developed, and their structure was analyzed in relation to their protein adsorption resistance in a first step. These results are reported in Table 8. CMD immobilization was assessed by X-ray electron spectroscopy (XPS) and the layers interactions with a silica sphere were analyzed by atomic force microscopy (AFM) force measurements. The results suggested that the ionic strength influenced polymer conformation upon initial adsorption; the presence of electrolytes induces the collapse of the CMD molecules. The coupling agent concentration (EDC+NHS) and the carboxylation degree rather regulated pinning density, controlling final surface coverage, layer density and thickness. Moreover, protein adsorption measured by surface plasmon resonance microscopy (SPR) showed that, better protein rejection was obtained for uniform, dense and thick CMD layers.

Table 8: Physico-chemical characterization of CMD layers and their resistance to protein adsorption and cell adhesion.

| Conditions | #1 | #2 | #3 | #4 | #5 | #6 | #7 | #8 | #9 |
|---|---|---|---|---|---|---|---|---|---|
| O/C ratio [a] | 0.19 | 0.20 | 0.19 | 0.13 | 0.13 | 0.24 | 0.13 | 0.18 | 0.20 |
| Separation distance 10 mM [b] | 63 | 48 | 44 | 19 | 50 | 65 | 20 | 10 | 60 |
| Separation distance 150 mM [b] | 33 | 44 | 22 | nd | 43 | 33 | nd | nd | 38 |
| Protein adsorption [c] | + | ++ | + | +++ | ++ | + | +++ | ++ | +/- |
| Cell coverage [d] | + | ++ | + | +++ | +++ | ++ | +++ | ++ | +/- |

[a] oxygen/carbon (O/C) atomic ratio determined by XPS analysis; [b] separation distance observed for AFM force curves obtained between a silica sphere and layers in 10 and 150 mM PBS solutions; [c] protein adsorption measured by SPR microscopy; [a, b, c] results are derived from ref 12;[d] CMD layers coverage by cells after 3 days of confluence.

In a second step, CMD arrays were exposed to cells to further screen CMD coatings cell resistance and to observe cell behavior towards layers with various structures.

Four hours after seeding CMD arrays with fibroblasts, few cells were present on CMD spots and those cells were rounded, with an undeveloped actin cytoskeleton, no focal adhesion, and they were not able to deposit matrix fibronectin. Like most polysaccharide coatings, all CMD layers offered some resistance to cell adhesion, impeding cell adhesion and/or spreading.

However, 12 hours after seeding, cells on CMD spots had either disappeared (possibly by detaching) or begun to spread. Observation of the various conditions allowed defining three classes of CMD layers depending on their resistance to cell adhesion; the same distinct categories were observed after exposing CMD arrays to confluent cells for 3 days. The difference in cell behavior towards the various CMD surfaces may be explained by a failure of the resistance barriers offered by CMD layers towards protein adsorption and cell adhesion.

Layers made of CMD conditions #4, #5 and #7 supported cell adhesion and spreading at 12 hours, associated with deposition of matrix fibronectin, formation of focal adhesions and organization of actin into stress fibers. Exposition of these CMD layers to confluent cells resulted in a rapid and complete coverage, with a cell organization similar to that on HApp layers. Protein adsorption assays revealed that these CMD surfaces presented the least resistance towards protein adsorption.[12] These CMD layers were immobilized with a low coupling agent concentration (resulting in a low pinning density) and with electrolyte. Physico-chemical analysis suggested that these CMD layers were not dense (low O/C ratio) and were irregular (random size distribution of loops and tails) and possibly presented pinholes. Polysaccharide layers immobilized with a low pinning density, either by polymer adsorption or end-on-grafting have been shown to be more subject to protein adsorption and cell adhesion than covalently immobilized polysaccharides in side-on configuration. They did not form a sufficiently dense protective barrier and the surface coverage may not completely hide the underlying surfaces, which are subject to non-specific protein adsorption and cell adhesion.

The second class of CMD layers is composed of conditions #2, #6 and #8, they are called semi-resistant. At 12 hours, some cells were still present on those CMD layers, although neither deposition of fibronectin, nor focal adhesion formation nor actin cytoskeleton was observed. After exposition to confluent cells, semi-resistant CMD spots were quite resistant to cell adhesion for 2 days, but they then were suddenly covered on the third day: few cells established contacts with the CMD layer, while some formed bridges above the surface, leaning on settled cells. Besides, observation of CMD spots covered at Day 5 revealed that while cells tended to be aligned in bundles on cell-covered CMD spots made using conditions #4, #5, and #7 (Figure 29c), similarly as on HApp surfaces, cells on covered semi-resistant CMD spots formed a lattice (Figure 29f), indicative of a different invasion mechanism. Layers made using conditions #2, #6 and #8 were immobilized either with a high EDC+NHS/COOH ratio (layers #6 and #8) or with almost no added electrolyte (layers #2), improving immobilization efficiency despite a low EDC+NHS/COOH ratio. XPS and AFM force measurements suggested that those layers have a higher surface density and that they were uniform, pinhole free, probably hiding the underlying HApp surface, which explains their better resistance to cell adhesion. Despite a uniform surface coverage, CMD layers made under conditions #2 and #8 were also subject to protein adsorption. Layers made under conditions #2 were extended, due to a very low electrolyte concentration, but they were diffuse, due to a low pinning density. These layers did not constitute a steric barrier as their open structure allows in-diffusion of proteins. Layers made under conditions #8 were dense but presented short extensions with limited mobility and compressibility, due to the immobilization in the presence of a high electrolyte concentration and a high pinning density, limiting steric-entropic repulsion of proteins.

Surprisingly, layers made using conditions #6 were as resistant to protein adsorption as layers made under conditions #1, #3 and #9, yet some cells were able to adhere and spread on CMD layers #6. CMD surfaces made under conditions #1, #3, #6 and #9 all resulted in dense, extended and compressible layers, hindering the access of proteins to the underlying plasma layer by forming a steric barrier. However, high MW dextrans (e.g., 500-kDa used in conditions #6) have an increased polydispersity index and few long branches. The degree of intra-molecular freedom is decreased and affects CMD molecules ability to rearrange on surfaces. At lower MW, CMD is linear with very few to no long branches, therefore having a greater conformational mobility and ability to reorient more effectively to optimize packing at the interface (70-kDa used in conditions #1, #3 and #9). AFM imaging of thiolated dextran and CMD of various molecular weights showed presence of bulges for 500-kDa dextran derivative layers while 70-kDa layers looked quite smooth.

Finally, graft layers made using CMD conditions #1, #3, #9 with a high pinning density, low electrolyte concentration and 70-kDa CMD, formed uniform, dense and extended hydrated layers providing a steric-entropic barrier to protein adsorption. Indeed, layers made using conditions #1, #3 and #9 showed some protein adsorption but to lower extent than other layers (except those made using condition #6). As they were made from 70-kDa CMD, they were supposed to present a homogeneous surface, possibly favoring the formation of a stable water layer, an additional protection towards protein adsorption and cell adhesion. Almost no additional cells were observed on these layers after 12 hours. When exposing CMD arrays to confluent cells, spots made using those conditions were progressively invaded by cells but were still apparent on the last day of the experiment. As mentioned earlier, few cells resting on fibroblasts settled at the spot edge could adhere on the CMD spots.

CMD layers made using conditions #1 and #3 were comparable and showed less resistance to protein adsorption and cell adhesion than those made under conditions #9. The latter performance could be attributed either to the use of a higher CMD concentration during the immobilization or to an intermediate carboxylation degree. Higher concentration would result in a denser layer, offering a better steric-entropic barrier, while carboxylation degree regulates pinning density, layer thickness via loops and tails size distribution, and charge density. Hence, three conditions with various carboxylation degrees and a concentration of 2 mg/ml were tested to determine the optimal immobilization conditions leading to a cell repulsive layer. They were immobilized with a high EDC+NHS/COOH ratio to increase surface coverage and density, with no added electrolyte to favor chains extension, and they were made of CMD 70-kDa, to allow the formation of a homogeneous surface. CMD-25 was resistant towards cell adhesion while CMD-5 and CMD-50 were progressively covered by cells, though they were immobilized with the same concentration. Martwiset et al. studied the effect of the modification degree of oxidized dextran layers on protein adsorption and they found better protein repulsion for dextran layers made with an intermediate degree of oxidation (25%). Modification degree first controls pinning density; dextran derivatives immobilized with lower modification degrees such as CMD-5 are more diffuse. However, although those layers were immobilized with a higher concentration than layers made under conditions #1, they were less repulsive. It should be noted than CMD-5 was grafted in total absence of added electrolytes therefore molecules can be immobilized on the HApp surface in a more extended conformation, presenting less carboxylic groups for covalent linkage to the amine surface, thus reducing pinning density. On the contrary, CMD-50 was immobilized with a high pinning density, resulting in short loops and tails with a probably reduced mobility; it must have been immobilized with a higher density due to a higher concentration. It was also noted that dextran derivatives with higher modification degrees had reduced dissolution. Reactive groups added confer a different molecular structure to the polymer chains, which may alter the interactions with the surrounding water molecules and the structure of the interfacial water layer. Finally, in the case of CMD, increasing carboxylation degree and surface density lead to an increased charge density, generally associated with protein adsorption via electrostatic interactions. Hence, without being bound to a particular theory, layers ability to reject protein adsorption and cell adhesion results from a combination of different types of interactions such as i) electrostatic interactions, which should be minimized by decreasing surface charge density, ii) steric-entropic effects, maximum for dense and flexible layers, and iii) molecular interactions between water molecules and polymer moieties at the interphase, which may provide a protective "shell".

CMD layers were compared to PEG surfaces. PEG was immobilized close to cloud-point (CP) conditions, as this immobilization mode yields the highest chain density and the best low-fouling property. CMD-25 and PEG-CP layers both resisted cell adhesion while exposed to confluent cells for three days; they can be considered as non-fouling layers. Then, CMD layers immobilized with optimal conditions can provide a good alternative to PEG layers, being as cell-resistant, less expensive, and derived from a natural source. CMD has also the advantage of being multivalent: carboxylic groups present in its structure can be easily modified yielding an interface useful for the development of bioactive surfaces on which biosignals can be covalently incorporated.

Conclusions

Arrays of carboxy-methyl-dextran (CMD) graft layers were exposed to fibroblasts for short-term and long-term cell adhesion assays. Behavior of cells exposed to the different CMD layers was studied and CMD layers cell resistance was screened in relationship with the immobilization conditions used.

Cell-CMD layer interactions were time-dependent. Non-resistant CMD layers, characterized by a non-uniform surface coverage, allowed matrix protein adsorption and the subsequent formation of focal contacts, cell adhesion and cell spreading. On semi-resistant CMD layers, cells encountered some resistance and most of them relied on interactions with attached neighboring cells for spot invasion. Only the dense and thick CMD graft layers made using 70-kDa CMD could partially resist cell adhesion after exposition to confluent cells. By minimizing protein adsorption, resistant CMD layers impaired the formation of cell-surface adhesion sites, preventing cell adhesion and cell survival.

Finally, screening of CMD layer cell resistance led to the synthesis of an optimized CMD layer, which was dense, thick, and homogeneous. This optimized CMD layer was as cell-resistant as PEG layers produced under cloud point conditions and represent a good alternative.

**EXAMPLE 7**

**CMD-based Bioactive Arrays**

The present example relates to the use of an array of the invention to study the specific effect of biomolecules on endothelial cells (EC) behavior in order to develop bioactive surfaces favoring EC adhesion, growth and formation of tubular structures. Formation of functional blood vessels is important for medical applications such as tissue engineering, regenerative medicine and wound healing. The adhesive peptide GRGDS (RGD) was co-immobilized on a cell-resistant CMD layer with peptides believed to specifically enhanced EC adhesion (GREDVDY (Re) and GDSVVYGLR (SV)) and/or with vascular endothelial growth factor (VEGF), a growth factor specific for ECs. Arrays were tested with endothelial cells and fibroblasts seeking specificity of adhesion.

Substrates were covered by a low-fouling CMD layer (70MW, 25% COOH, 2mg/mL CMD solution concentration, EDC:COOH 5:1) prior to biomolecules immobilization. Proteins and peptides were dissolved in a sterile buffer (PBS, pH 8.5) at varied concentrations (RGD (R): 25 $\mu$g/ml, Re and SV: 1 $\mu$g/ml, VEGF (V): 2 $\mu$g/ml). The CMD substrate was activated with EDC/NHS for 10 minutes and dried. Then, the biomolecule arrays were made on the CMD surface with the Biochip Arrayer, each spot was made of 50 drops of solution. The arrays were immediately placed in a small humidity chamber (dew point) overnight, and were then rinsed with a saline solution. Prior to exposition to cells, biomolecules arrays were incubated in an antibiotic solution overnight at 4°C and rinsed with sterile PBS.

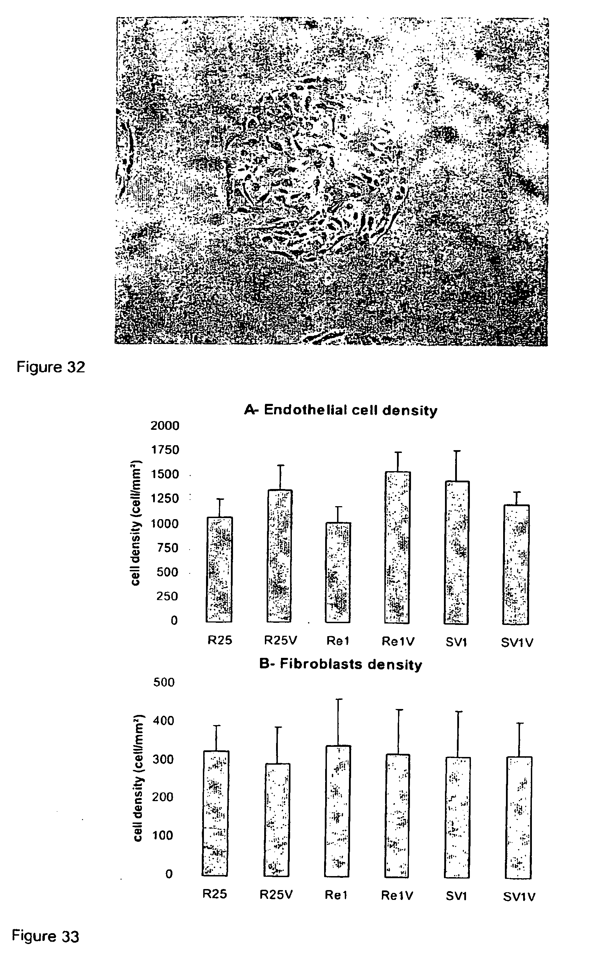

Pictures of cells on RGD spots are presented in Figure 32. RGD is a fragment of fibronectin, known to promote cell adhesion and spreading. We observed no cell adhesion outside of every biomolecule spot, underlying i) the ability of CMD layers to resist cell adhesion, and ii) the possibility to covalently immobilize functional peptides, restricting cell adhesion to designed zones.

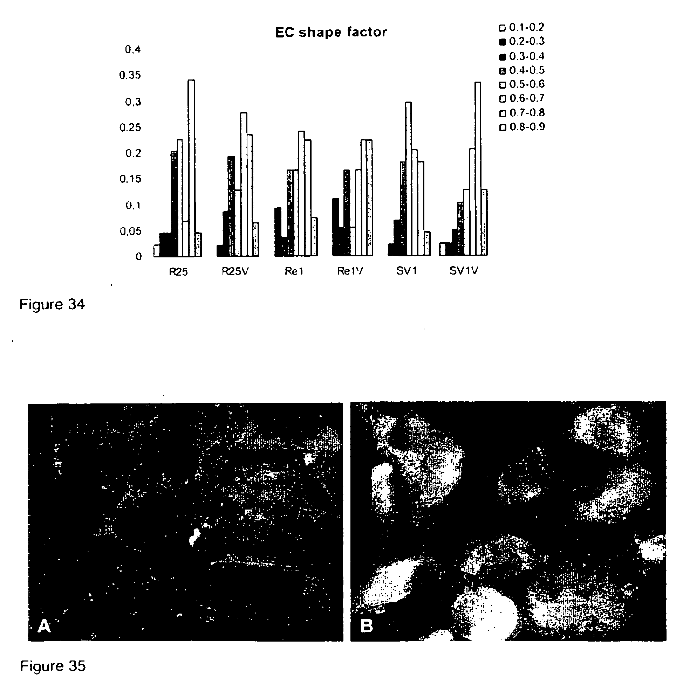

Figure 33 shows average cell density on various biomolecules spots 6 hours after seeding arrays with EC (Figure 33A) or fibroblasts (Figure 33B). Co-immobilization of RGD with Re, SV or VEGF did not yield any effect on fibroblasts density or spreading. However, co-immobilization of RGD or RGD+Re with VEGF induced an increased in EC density. Moreover, co-immobilization of RGD with a low-concentration of SV also induced a significant increase in EC density. Finally, addition of Re and/or VEGF seems to induced an increase in cell shape factor, that is cell rounding associated with spreading (Figure 34). Hence, the use of bioactive arrays made on a CMD cell-resistant layer allowed to analyze molecules effects specific for EC, as fibroblasts did not seem able to distinguish the various signals. Immobilization of molecules on a cell-resistant layer such as CMD allows assessment of direct interaction between the immobilized molecule and cell receptors.

Finally, after long term culture, ECs on spots still expressed markers specific for their integrity (VE-cadherin expression, Figure 35A) and function (von Willebrand factor synthesis, Figure 35B). Bioactive molecules immobilized on a CMD cell-resistant layer allow cells to preserve their differentiated state.

## EXAMPLE 8

### Bioactive Arrays Made of Poly(ethylene glycol) (PEG) / Poly(ethylene oxide) (PEO) Underlying Hard Layers or PEG Arrays

NHS-PEG-NH$_2$-t-Boc was reacted with amine plasma layers or poly(ethylenimine) (PEI) grafted onto aldehyde plasma polymer layers. Other interlayers and/or PEG materials may be used, including the use of different PEG end-group functionalities and protection molecules. An aspect of this reaction is the possibility to modulate grafting density and, thus, the low cell binding and mechanical properties of the polymer layer by using different polymer concentrations or by reacting the polymer in its cloud point condition. Another aspect is the chemical availability of reactive end-groups on the immobilized polymers.

To create arrays made of active bio-molecules, the polymer layer is deprotected and chemically activated prior to the robot-assisted deposition of bioactive molecules which are allowed to bind to the surfaces. NHS-PEG-NH$_2$-t-Boc is Boc-deprotected using trifluoroacetic acid prior to the deposition of bioactive molecules solutions using the Biochip Arrayer from PerkinElmer onto exposed reactive amine groups. The bioactive molecules are deposited on the desired locations on the surfaces and allowed to covalently bind in a water saturated environment to prevent droplet evaporation before reaction. Each spot is made of 1 to 5000 drops of solution (time of activation is then about 10 minutes.). Substrates are then put in small chambers overnight (substrate at dew point). Arrays are rinsed with Milli-Q water (3x2h agitation) and can be autoclaved. Arrays can be stored at 4°C in Milli-Q water or they may be air-dried and stored at room temperature for extended periods of time. The rinsed surfaces are available to be used; for example, to promote the localized adhesion of c-kit positive cells (such as hematopoietic stem cells) onto surfaces upon which the stem cell factor was immobilized. The final product can be sterile or non-sterile depending on end-use.

## EXAMPLE 9

### Bioactive Arrays Made of Carboxy Methyl Dextran (CMD) Underlying Hard Layers or CMD Arrays

Substrates are cleaned and can be, for example, glass slides, mica sheets, FEP strips or SPR substrates and microscope cover slips.

To create bioactive arrays made of CMD underlying layers, CMD is first immobilized from a CMD solution that has been sonicated for 10 minutes (to remove air bubbles) and filtered (to remove aggregates). EDC and NHS are added (activation time of 10 min), then substrates are put in the CMD solution for 2 hours with agitation. Substrates are rinsed with NaCl 1 M solution (3x2h, agitation) and then milliQ water (3x2h, agitation) and stored at 4°C in MilliQ water. CMD surfaces may be made to be uniform or not (CMD of various MW or COOH degrees are mixed to promote protein patterns: instead of a uniform protein distribution, we can expect formation of clusters of proteins).

Proteins are dissolved in a sterile buffer (e.g., PBS, pH 7.4) at varied concentrations. The protein solution may contain glycerol to diminish/prevent drop evaporation. The protein array is made on the CMD surface with the Biochip Arrayer, and each spot may be made of 1 to 5000 drops of solution. The CMD substrate is previously activated with EDC/NHS for 10 minutes and then dried. Once the array is made, the substrate is placed in a small humidity chamber (dew point) overnight, and rinsed with a saline solution and MilliQ water. It is possible to use various coupling agent to link proteins (e.g., carbodiimide chemistry, conjugation chemistry directed toward specific amino-acids /chemical groups like -SH, tag recognition in case of tagged proteins, avidin-biotin conjugation system for biotinylated proteins, etc.). It is also possible to add spacers. The array is dropped in PBS, exposed to UV for 15 minutes and rinsed with sterile PBS.

**EXAMPLE 10**

**Bioactive Arrays Made of Poly(acrylic)acid (PAAC) Underlying Hard Layers or PAAC Arrays**

The concentration of the PAAC solution, the molecular weight of the polymer and the coupling agents/polymer ratio are parameters that can be modulated to create PAAC-based polymer chips with different properties.

To fabricate the support of the invention with a PAAC underlayer, in an embodiment, a specific polymer solution is sonicated to eliminate the air that can be trapped inside of it. Then, the coupling agents (EDC, NHS) (Sigma Aldrich) are added to the polymer solution and left to react for 10 minutes. The chosen substrate is placed in contact with the activated PAAC solution and left to react for 8 hours. The bioactive molecules can then be immobilized in the same ways described for the PEG and CMD protocols (Examples 7 and 8).

**EXAMPLE 11**

**Bioactive Arrays Design**

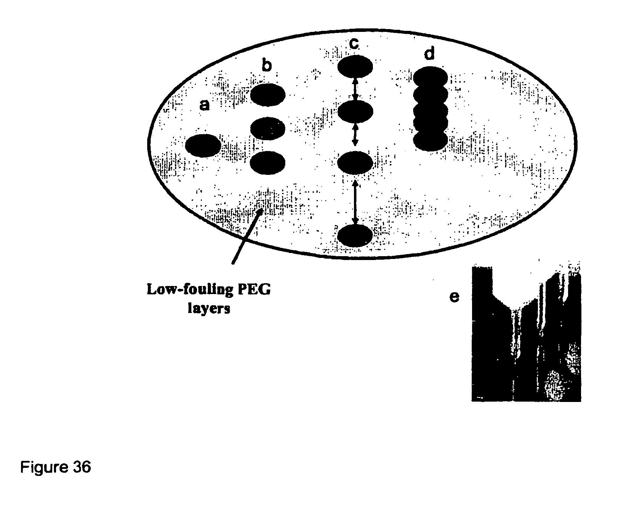

Microarrays illustrated in Figure 36 are made with low-cell binding layers immobilized on amine-bearing plasma covered surface. Then, bio-signals are deposited using the BioChip Arrayer illustrated in Figure 36e. Bioactive niches composed of a mixture of bio-signals (Figure 36a), different bioactive niches composed of individual bio-signals (Figure 36b), and/or tracks of individual bio-signals with concentration gradient along the tracks are created by covalent immobilization of the bio-signals on activated low cell binding layers. The separation distance between different bioactive niches can be accurately modulated (Figure 36c). Cells are deposited on these microarrays bearing a large number of bioactive niches allowing rapid (high-throughput) cell testing to recreate bioactive niches in laboratory. The BioChip Arrayer allows deposition of ca. 290 pL. Larger volumes can also be dispensed allowing modulation of the spot size from few micro-meters to millimeters.

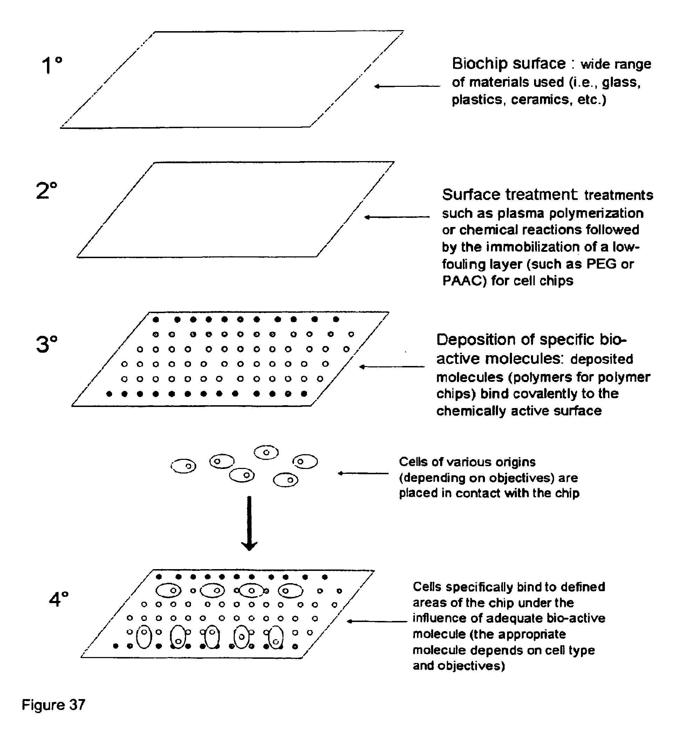

Figure 37 shows a schematic of the possible fabrication steps of the above-described microarray.

Although the present invention has been described hereinabove by way of specific embodiments thereof, it can be modified.

**Claims**

1.   An assay support for assaying selective binding or response of a cell, said support comprising:

    (a) a substrate having a surface, preferably a substrate selected from the group consisting of tissue culture plates, membranes, microscope slides, glass, mica, silicon wafers, plastic materials, polytetrafluoroethylene, FEP materials, polyurethanes, polypropylene, polycarbonate, polyethylene, metallic substrate, gold-based substrate, silver-based substrate, stainless steel-based substrate, titanium-based substrate, palladium-based substrate and copper-based substrate;
    (b) one or more first layers covalently attached to one or more areas of said surface, said first layers exhibiting low-cell binding properties; and
    (c) bioactive molecules which are covalently attached to said one or more first layer and which are selected from the group consisting of natural proteins, recombinant proteins, growth factors, antibodies, enzymes, peptides, drugs, amino acid sequences, lipids, polysaccharides, proteoglycans, vitamins and mixtures thereof, comprised in one or more discrete regions of said first layer, said bioactive molecules being capable of cell binding,

    wherein said support is obtainable by covalently attaching said one or more first layers to said one or more areas of said surface, and disposing said bioactive molecules onto one or more discrete regions of the first layer.

2.   The support of claim 1 wherein said low-cell binding properties lead to a statistically significant lower number of cells binding to said first layer per area unit relative to the number of cells binding to said discrete region, wherein said statistically significant lower number of cell has a p-value of 0.05 or less.

3.   The support of claim 1 wherein said first layer comprises a poly(ethylene glycol) (PEG)-based polymer, preferably linear PEG or star PEG, more preferably linear PEG.

4. The support of claim 3 wherein said linear PEG has been attached to said substrate from a PEG solution in cloud point conditions.

5. The support of claim 1 wherein said first layer comprises a polysaccharide, preferably carboxy methyl dextran (CMD).

6. The support of claim 1 wherein said first layer comprises a polyelectrolyte, preferably poly(acrylic acid).

7. The support of claim 1, which is preferably a microarray, said support comprising two or more discrete regions, preferably discrete regions geometrically arranged on said substrate.

8. A method of manufacturing a support for assaying selective binding or response of a cell, said method comprising:

(a) covalently attaching one or more first layers to one or more areas of a surface of a substrate, said one or more first layers exhibiting low-cell binding properties; and
(b) disposing bioactive molecules as defined in claim 1 onto one or more discrete regions of said first layer, said bioactive molecules being covalently attached to said one or more first layer.

9. The method of claim 8 wherein said support is the support of any one of claims 1 to 7.

10. A support produced by the method of claim 8 or 9.

11. A method of determining cell activity comprising contacting the surface of the support of claim 1 with said cell.

12. A method of studying the activity of a compound of interest, preferably a drug, said method comprising:

(a) contacting the surface of the support of claim 1 with a sample comprising cells; and
(b) contacting said cells with said compound.

13. A method of selectively enriching a first cell, preferably a hematopoietic cell, from a population of cells, comprising contacting the support of claim 1 with said population, wherein said bioactive molecules are capable of selectively binding said first cell.

14. A method of immobilizing cells, preferably hematopoietic cells, myoblasts, endothelial cells, fibroblasts, platelets or activated platelets comprising contacting the support of claim 1 with said cells.


**Patentansprüche**

1. Assayträger zur Untersuchung der selektiven Bindung oder Antwort einer Zelle, wobei der genannte Träger umfasst:

(a) ein Substrat, das eine Oberfläche besitzt, vorzugsweise ein Substrat ausgewählt aus der Gruppe, bestehend aus Gewebekulturplatten, Membranen, Mikroskopobjektträgern, Glas, Glimmer, Siliziumwafern, Plastikmaterialien, Polytetrafluorethylen, FEP-Materialien, Polyurethanen, Polypropylen, Polycarbonat, Polyethylen, metallischem Substrat, Gold-basiertem Substrat, Silberbasiertem Substrat, Edelstahl-basiertem Substrat, Titanbasiertem Substrat, Palladium-basiertem Substrat und Kupferbasiertem Substrat;
(b) eine oder mehr erste Schichten, die kovalent an eine oder mehr Bereiche der genannten Oberfläche gebunden sind, wobei die genannten ersten Schichten geringe Zellbindungseigenschaften aufweisen; und
(c) bioaktive Moleküle, die kovalent an eine oder mehr erste Schicht(en) gebunden sind, und die ausgewählt sind aus der Gruppe, bestehend aus natürlichen Proteinen, rekombinanten Proteinen, Wachstumsfaktoren, Antikörpern, Enzymen, Peptiden, Wirkstoffen, Aminosäuresequenzen, Lipiden, Polysacchariden, Proteoglykanen, Vitaminen und Gemischen davon, die in einer oder mehr diskreten Regionen der genannten ersten Schicht enthalten sind, wobei die bioaktiven Moleküle zur Zellbindung fähig sind,

wobei der genannte Träger erhalten werden kann durch kovalente Anbindung einer oder mehr ersten Schicht(en) an eine oder mehr der genannten Bereiche der genannten Oberfläche, und durch Verteilen der genannten bioaktiven Moleküle auf eine oder mehr diskrete Regionen der ersten Schicht.

2. Träger gemäß Anspruch 1, worin die genannten geringen Zellbindungseigenschaften zu einer statistisch signifikant geringeren Anzahl an Zellen, die an die genannte erste Schicht binden, pro Flächeneinheit relativ zur Anzahl an Zellen, die an die genannte diskrete Region binden, führen, wobei die genannte statistisch signifikant geringere Anzahl an Zellen einen p-Wert von 0,05 oder kleiner hat.

3. Träger gemäß Anspruch 1, wobei die erste Schicht ein Poly(ethylenglykol)(PEG)-basiertes Polymer, vorzugsweise lineares PEG oder sternförmiges PEG, stärker bevorzugt lineares PEG, umfasst.

4. Träger gemäß Anspruch 3, wobei das genannte lineare PEG unter Trübungspunktbedingungen aus einer PEG-Lösung an das genannte Substrat gebunden wurde.

5. Träger gemäß Anspruch 1, wobei die erste Schicht ein Polysaccharid, vorzugsweise Carboxymethyldextran (CMD), umfasst.

6. Träger gemäß Anspruch 1, wobei die erste Schicht einen Polyelektrolyten, vorzugsweise Poly(acrylsäure), umfasst.

7. Träger gemäß Anspruch 1, der vorzugsweise ein Mikroarray ist, wobei der genannte Träger zwei oder mehr diskrete Regionen, vorzugsweise diskrete Regionen, die geometrisch auf dem genannten Substrat angeordnet sind, umfasst.

8. Verfahren zur Herstellung eines Trägers zur Untersuchung der selektiven Bindung oder Antwort einer Zelle, wobei das Verfahren umfasst:

(a) kovalentes Anbinden einer oder mehrerer erster Schichten an einen oder mehrere Bereiche einer Oberfläche eines Substrats, wobei die eine oder mehr erste Schichten geringe Zellbindungseigenschaften aufweisen; und
(b) Verteilen bioaktiver Moleküle, wie in Anspruch 1 definiert, auf eine oder mehr diskrete Region(en) der genannten ersten Schicht, wobei genannte bioaktive Moleküle kovalent an eine oder mehr genannte erste Schicht(en) gebunden sind.

9. Verfahren gemäß Anspruch 8, wobei der genannte Träger der Träger gemäß einem der Ansprüche 1 bis 7 ist.

10. Träger, hergestellt durch das Verfahren gemäß Anspruch 8 oder 9.

11. Verfahren zur Bestimmung einer Zellaktivität, welches das Kontaktieren der Oberfläche des Trägers, gemäß Anspruch 1, mit genannter Zelle umfasst.

12. Verfahren, um die Aktivität einer Verbindung von Interesse, vorzugsweise eines Wirkstoffs, zu untersuchen, wobei das genannte Verfahren umfasst:

(a) Inkontaktbringen der Oberfläche des Trägers gemäß Anspruch 1 mit einer Probe, die Zellen umfasst; und
(b) Inkontaktbringen der genannten Zellen mit der genannten Verbindung.

13. Verfahren, um selektiv eine erste Zelle, vorzugsweise eine blutbildende Zelle, aus einer Zellpopulation anzureichern, das das Inkontaktbringen des Trägers gemäß Anspruch 1 mit genannter Population umfasst, wobei die genannten bioaktiven Moleküle zur selektiven Bindung der genannten ersten Zelle fähig sind.

14. Verfahren zur Immobilisierung von Zellen, vorzugsweise blutbildenden Zellen, Myoblasten, Endothelzellen, Fibroblasten, Plättchen oder aktivierten Plättchen, das das Inkontaktbringen des Trägers gemäß Anspruch 1 mit den genannten Zellen umfasst.

## Revendications

1. Support d'essai permettant de tester une liaison ou réponse sélective d'une cellule, lequel support comporte :

a) un substrat doté d'une surface, qui est de préférence un substrat choisi dans l'ensemble constitué par les plaques de culture de tissu, membranes, lames de microscope, verre, mica, plaquettes de silicium, matières plastiques, polytétrafluoroéthylène, polymères fluorés de type FEP, polyuréthanes, polypropylènes, polycarbonates, polyéthylènes, substrats métalliques, substrats à base d'or, substrats à base d'argent, substrats à base d'acier inoxydable, substrats à base de titane, substrats à base de palladium, et substrats à base de cuivre ;

b) une ou plusieurs première(s) couche(s), attachée(s) par covalence à une ou plusieurs zone(s) de ladite surface, lesquelles premières couches présentent des caractéristiques de bas niveau de liaison aux cellules ;
c) et des molécules bioactives qui sont attachées par covalence à ladite ou auxdites première(s) couche(s) et qui sont choisies dans l'ensemble constitué par les protéines naturelles, protéines recombinantes, facteurs de croissance, anticorps, enzymes, peptides, médicaments, séquences d'acides aminés, lipides, polysaccharides, protéoglycanes et vitamines, ainsi que leurs mélanges, présentes dans une ou plusieurs régions(s) discrète(s) de ladite première couche, lesquelles molécules bioactives sont capables de se lier à des cellules ;

étant entendu que l'on peut obtenir ledit support en attachant par covalence ladite ou lesdites première(s) couche(s) à ladite ou auxdites zone(s) de ladite surface, et en disposant lesdites molécules bioactives sur une ou plusieurs région(s) discrète(s) de la première couche.

2. Support conforme à la revendication 1, dans lequel lesdites caractéristiques de bas niveau de liaison aux cellules conduisent à ce que le nombre de cellules se liant à ladite première couche par unité d'aire est plus petit, de manière statistiquement significative, que le nombre de cellules se liant à ladite région discrète, étant entendu que ledit nombre de cellules plus petit de manière statistiquement significative présente un paramètre p valant au plus 0,05.

3. Support conforme à la revendication 1, dans lequel ladite première couche comprend un polymère à base de polyéthylène-glycol ou PEG, lequel est de préférence un PEG linéaire ou en étoile et mieux encore un PEG linéaire.

4. Support conforme à la revendication 3, dans lequel ledit PEG linéaire a été attaché audit substrat à partir d'une solution de PEG, dans des conditions correspondant à un point de trouble de celle-ci.

5. Support conforme à la revendication 1, dans lequel ladite première couche comprend un polysaccharide, qui est de préférence un carboxy-méthyl-dextrane ou CMD.

6. Support conforme à la revendication 1, dans lequel ladite première couche comprend un polyélectrolyte, qui est de préférence un poly(acide acrylique).

7. Support conforme à la revendication 1, qui est de préférence un microréseau, lequel support comprend deux régions discrètes ou plus, de préférence des régions discrètes disposées sur ledit substrat selon une certaine géométrie.

8. Procédé de fabrication d'un support permettant de tester une liaison ou réponse sélective d'une cellule, lequel procédé comporte les étapes suivantes :

a) attacher par covalence une ou plusieurs première(s) couche(s) à une ou plusieurs zone(s) d'une surface d'un substrat, laquelle ou lesquelles première(s) couche(s) présentent des caractéristiques de bas niveau de liaison aux cellules ;
b) et disposer des molécules bioactives, telles que définies dans la revendication 1, sur une ou plusieurs région(s) discrète(s) de ladite première couche, lesquelles molécules bioactives sont attachées par covalence à ladite ou auxdites première(s) couche(s).

9. Procédé conforme à la revendication 8, dans lequel ledit support est un support conforme à l'une des revendications 1 à 7.

10. Support produit selon un procédé conforme à la revendication 8 ou 9.

11. Procédé permettant de déterminer l'activité d'une cellule, lequel procédé comporte le fait de mettre ladite cellule en contact avec la surface d'un support conforme à la revendication 1.

12. Procédé permettant d'étudier l'activité d'un composé intéressant, qui est de préférence un médicament, lequel procédé comporte les étapes suivantes :

a) mettre un échantillon comprenant des cellules en contact avec la surface d'un support conforme à la revendication 1;
b) et mettre lesdites cellules en contact avec ledit composé.

13. Procédé permettant d'accumuler sélectivement des cellules d'un premier type, de préférence des cellules hématopoïétiques, à partir d'une population de cellules, lequel procédé comporte le fait de mettre ladite population en contact avec un support conforme à la revendication 1, dont lesdites molécules bioactives sont capables de se lier sélectivement auxdites cellules de premier type.

14. Procédé permettant d'immobiliser des cellules, de préférence des cellules hématopoïétiques, des myoblastes, des cellules endothéliales, des fibroblastes, des plaquettes ou des plaquettes activées, lequel procédé comporte le fait de mettre lesdites cellules en contact avec un support conforme à la revendication 1.

Figure 1

Figure 2

Figure 3

Figure 4

Figure 5

Figure 6

Figure 7

Figure 8

Figure 9

Figure 10

Diagnostic
slide

Silicone
gasket

Figure 11

Shear
rate (s⁻¹)

Slit width: 200 μm

Shear
rate (s⁻¹)

Slit width: 1000 μm

Figure 12

Figure 13

[YM I]

Figure 14

Figure 15

Figure 16

Figure 17

Figure 18

Figure 19

Figure 20

Figure 21

Figure 22

Figure 23

Figure 24

**CMD-Conditions #8**     **CMD-Conditions #3**

Figure 25

Figure 26

|  | Actin | Vinculin | Fibronectin |
|---|---|---|---|
| HApp | a | b | c |
| CMD #9 | d | e | f |
| CMD #4 | g | h | i |

Figure 27

Figure 28

Figure 29

Figure 30

Figure 31

Figure 32

Figure 33

Figure 34

Figure 35

Figure 36

**1°** — Biochip surface : wide range of materials used (i.e., glass, plastics, ceramics, etc.)

**2°** — Surface treatment : treatments such as plasma polymerization or chemical reactions followed by the immobilization of a low-fouling layer (such as PEG or PAAC) for cell chips

**3°** — Deposition of specific bio-active molecules: deposited molecules (polymers for polymer chips) bind covalently to the chemically active surface

Cells of various origins (depending on objectives) are placed in contact with the chip

**4°** — Cells specifically bind to defined areas of the chip under the influence of adequate bio-active molecule (the appropriate molecule depends on cell type and objectives)

Figure 37

## REFERENCES CITED IN THE DESCRIPTION

### Patent documents cited in the description

- US 80402106 P
- US 20040147045 A, Nelson
- US 20040253607 A, Nock
- WO 02052045 A
- WO 2005047529 A
- US 6844028 B
- US 6806050 B
- US 5436161 A
- US 200504455 B
- US 6878523 B
- US 6951761 B
- US 7033761 B

### Non-patent literature cited in the description

- **SEONG et al.** *Proteomics,* 2003, 3
- **SNYDER et al.** *Curr Opn in Chem Biol,* 2003, 7
- **KATAOKA et al.** *ChemBioChem,* 2004, 5
- **ZHU et al.** *Biomaterials,* 2005, 25
- **VERMETTE, P. ; MEAGHER, L.** *Colloids and Surfaces B-Biointerfaces,* 2003, vol. 28, 153-198
- **GRIESSER et al.** *Journal of Colloid and Interface Science,* 2003, vol. 259, 13-26
- **CLEVELAND et al.** *Review of Scientific Instruments,* 1993, vol. 64, 403-405
- **PANG, P. ; ENGLEZOS, P.** *Colloids and Surfaces A-Physicochemical and Engineering Aspects,* 2002, vol. 204, 23-30
- **PIEHLER et al.** *Biosens. Bioelectron.,* 1997, vol. 12, 531-538
- **MCARTHUR et al.** *Colloids and Surfaces B-Biointerfaces,* 2000, vol. 17, 37-48